# EUROPEAN PATENT SPECIFICATION

(11) **EP 3 775 171 B1**
(45) Date of publication and mention of the grant of the patent: **03.01.2024**
(21) Application number: 19774552.4
(22) Date of filing: 26.03.2019
(51) Int. Cl.: C12N 5/095, A61K 39/00, A61K 31/506, A61K 31/517, A61K 38/18, A61K 45/06, A61K 31/519, A61P 35/04

(54) **METHODS OF TREATING MINIMAL RESIDUAL CANCER**
VERFAHREN ZUR BEHANDLUNG VON MINIMALEM RESTKREBS
PROCÉDÉS DE TRAITEMENT D'UN CANCER RÉSIDUEL MINIMAL

(30) Priority: 26.03.2018 US 201862648166 P
(43) Date of publication of application: 17.02.2021
(73) Proprietor: Icahn School of Medicine at Mount Sinai, New York, NY 10029 (US)
(72) Inventor: AGUIRRE-GHISO, Julio, A., New York, NY 10029 (US)
(74) Representative: Potter Clarkson
(86) International application number: PCT/US2019/024097
(87) International publication number: WO 2019/191115

(56) References cited:
- WO-A1-2018/194885
- WO-A2-2005/030933
- US-A1- 2010 167 945
- KOBAYASHI AYA ET AL: "Bone morphogenetic protein 7 in dormancy and metastasis of prostate cancer stem-like cells in bone", JOURNAL OF EXPERIMENTAL MEDICINE, vol. 208, no. 13, 19 December 2011 (2011-12-19), pages 2641-2655, XP055871651, US ISSN: 0022-1007, DOI: 10.1084/jem.20110840
- JEROEN T. BUIJS ET AL: "BMP7, a Putative Regulator of Epithelial Homeostasis in the Human Prostate, Is a Potent Inhibitor of Prostate Cancer Bone Metastasis in Vivo", THE AMERICAN JOURNAL OF PATHOLOGY, vol. 171, no. 3, 1 September 2007 (2007-09-01), pages 1047-1057, XP055707780, US ISSN: 0002-9440, DOI: 10.2353/ajpath.2007.070168
- JEROEN T BUIJS ET AL: "TGF-[beta] and BMP7 interactions in tumour progression and bone metastasis", CLINICAL & EXPERIMENTAL METASTASIS, KLUWER ACADEMIC PUBLISHERS, DO, vol. 24, no. 8, 16 November 2007 (2007-11-16), pages 609-617, XP019550031, ISSN: 1573-7276, DOI: 10.1007/S10585-007-9118-2
- MARITTE R BOON ET AL: "Bone morphogenetic protein 7: A broad-spectrum growth factor with multiple target therapeutic potency", CYTOKINE & GROWTH FACTOR REVIEWS, vol. 22, no. 4, 15 September 2011 (2011-09-15), pages 221-229, XP028112256, ISSN: 1359-6101, DOI: 10.1016/J.CYTOGFR.2011.08.001 [retrieved on 2011-08-24]
- BUIJS J T ET AL: "The BMP2/7 heterodimer inhibits the human breast cancer stem cell subpopulation and bone metastases formation", ONCOGENE, vol. 31, no. 17, 1 April 2012 (2012-04-01) , pages 2164-2174, XP055873314, London ISSN: 0950-9232, DOI: 10.1038/onc.2011.400 Retrieved from the Internet: URL:https://www.nature.com/articles/onc201 1400.pdf>
- AGUIRRE-GHISO et al.: "Targeting dormant cancer", Nature Medicine, vol. 19, no. 3, 6 March 2013 (2013-03-06), pages 276-277, XP055639439,
- TATE et al.: "A BMP7 variant inhibits the tumorigenic potential of glioblastoma stem-like cells", Cell Death Differentiation, vol. 19, no. 10, 27 April 2012 (2012-04-27), pages 1644-1654, XP055639442,
- SANDOVAL et al.: "PERK :Inhibition as a possible therapy for hypoxia- induced solidary dormant tumor cells", ABSTRACT A45Proceedings of the AACR Special Conference on Tumor Metastasis, vol. 76, no. 7 30 November 2015 (2015-11-30), XP055639444, Austin, TX. Philadelphia (PA Retrieved from the Internet: URL:https://cancerres.aacrjournals.org/con tent/76/7_Supplement/A45
- SOSA et al.: "Mechanisms of disseminated cancer cell dormancy: an awakening field", Nature Reviews. Cancer, vol. 14, no. 9, 14 August 2014 (2014-08-14), - September 2014 (2014-09), pages 611-622, XP055639486,

## Description

This application claims the priority benefit of U.S. Provisional Patent Application Serial No. 62/648,166, filed March 26, 2018.

This invention was made with government support under R01 CA109182, U54 CA16131, and P30 CA196521 awarded by the National Institute of Health/National Cancer Institute and BC 132674 awarded by the Department of Defense-Congressionally Directed Medical Research Programs. The government has certain rights in the invention.

### FIELD

The present disclosure relates to compounds for use in methods of treating minimal residual cancer in a subject.

### BACKGROUND

The unfolding of proteins in the endoplasmic reticulum ("ER") lumen activates three main pathways, PERK, IRE1α, and ATF6, also known as the unfolded protein response ("UPR"), which allows cells to correct and survive this stress (Walter et al., "The Unfolded Protein Response: From Stress Pathway to Homeostatic Regulation," Science 334:1081-1086 (2011) and Ron et al., "Signal Integration In the Endoplasmic Reticulum Unfolded Protein Response," Nat. Rev. Mol. Cell Biol. 8:519-529 (2007)). Recent evidence suggests that in various types of cancer the UPR is a mechanism that allows tumor cells to respond to demands on the ER and oxidative conditions imposed by an enhanced translational load caused by oncogenes and hypoxia, among other signals (Blais et al., "Activating Transcription Factor 4 is Translationally Regulated by Hypoxic Stress," Mol. Cell. Biol. 24:7469-7482 (2004); Chevet et al., "Endoplasmic Reticulum Stress-Activated Cell Reprogramming in Oncogenesis," Cancer Discov. 5:586-597 (2015); Tameire et al., "Cell Intrinsic and Extrinsic Activators of the Unfolded Protein Response in Cancer: Mechanisms and Targets for Therapy," Semin. Cancer Biol. 33:3-15 (2015); Hart et al., "ER Stress-Mediated Autophagy Promotes Myc-Dependent Transformation and Tumor Growth," J. Clin. Invest. 122:4621-4634 (2012); Martin-Perez et al., "Activated ERBB2/HER2 Licenses Sensitivity to Apoptosis Upon Endoplasmic Reticulum Stress Through a PERK-Dependent Pathway," Cancer Res. 74:1766-1777 (2014); Rajasekhar et al., "Postgenomic Global Analysis of Translational Control Induced by Oncogenic Signaling," Oncogene 23:3248-3264 (2004); Rajasekhar et al., "Oncogenic Ras and Akt Signaling Contribute to Glioblastoma Formation by Differential Recruitment of Existing mRNAs to Polysomes," Mol. Cell 12:889-901 (2003); Rojo et al., "4E-Binding Protein 1, A Cell Signaling Hallmark in Breast Cancer that Correlates With Pathologic Grade and Prognosis," Clin. Cancer Res. 13:81-89 (2007); and Sequeira et al., "Inhibition of eIF2alpha Dephosphorylation Inhibits ErbB2-Induced Deregulation of Mammary Acinar Morphogenesis," BMC Cell Biol. 10:64 (2009)). Oncogene-activated pathways increase ER client protein load by activating mTOR signaling and translation initiation (Hart et al., "ER Stress-Mediated Autophagy Promotes Myc-Dependent Transformation and Tumor Growth," J. Clin. Invest. 122:4621-4634 (2012); Ozcan et al., "Loss of the Tuberous Sclerosis Complex Tumor Suppressors Triggers the Unfolded Protein Response to Regulate Insulin Signaling and Apoptosis," Mol. Cell 29:541-551 (2008); and Tameire et al., "Cell Intrinsic and Extrinsic Activators of the Unfolded Protein Response in Cancer: Mechanisms and Targets for Therapy," Semin. Cancer Biol. 33:3-15 (2015)). PERK and the IRE1α-XBP-1 pathways have been further shown to contribute to adaptation to hypoxia and microenvironmental stress (Bi et al., "ER Stress-Regulated Translation Increases Tolerance to Extreme Hypoxia and Promotes Tumor Growth," EMBO J. 24:3470-3481 (2005); Blais et al., "Activating Transcription Factor 4 is Translationally Regulated by Hypoxic Stress," Mol. Cell. Biol. 24:7469-7482 (2004); Chen et al., "XBP1 Promotes Triple-Negative Breast Cancer by Controlling the HIF1alpha Pathway," Nature 508:103-107 (2014); Romero-Ramirez et al., "X box-Binding Protein 1 Regulates Angiogenesis in Human Pancreatic Adenocarcinomas," Transl. Oncol. 2:31-38 (2009); Rouschop et al., "The Unfolded Protein Response Protects Human Tumor Cells During Hypoxia Through Regulation of the Autophagy Genes MAP1LC3B and ATG5," J. Clin. Invest. 120:127-141 (2010); Schewe et al., "ATF6alpha-Rheb-mTOR Signaling Promotes Survival of Dormant Tumor Cells In Vivo," Proc. Nat'l. Acad. Sci. U.S.A. 105:10519-10524(2008); and Ye et al., "The GCN2-ATF4 Pathway is Critical for Tumour Cell Survival and Proliferation in Response to Nutrient Deprivation," EMBO J. 29:2082-2096 (2010)), suggesting that the UPR can allow for adaptation to changing milieu.

PERK activation coordinates an antioxidant and autophagy response to protect mammary epithelial cells during loss of adhesion to the basement membrane (Avivar-Valderas et al., "PERK Integrates Autophagy and Oxidative Stress Responses to Promote Survival During Extracellular Matrix Detachment," Mol. Cell. Biol. 31:3616-3629 (2011)). This survival response involves an ATF4 and CHOP transcriptional program (Avivar-Valderas et al., "PERK Integrates Autophagy and Oxidative Stress Responses to Promote Survival During Extracellular Matrix Detachment," Mol. Cell. Biol. 31:3616-3629 (2011)) coupled to a rapid activation of the LKB1-AMPK-TSC2 pathway that inhibits mTOR (Avivar-Valderas et al., "Regulation of Autophagy during ECM Detachment is Linked to a Selective Inhibition of mTORC1 by PERK," Oncogene 32(41):4932-40 (2013)). Human DCIS lesions displayed enhanced PERK phosphorylation and autophagy (Avivar-Valderas et al., "PERK Integrates Autophagy and Oxidative Stress Responses to Promote Survival During Extracellular Matrix Detachment," Mol. Cell. Biol. 31:3616-3629 (2011) and Espina et al., "Malignant Precursor Cells Pre-Exist in Human Breast DCIS and Require Autophagy for Survival," PloS One 5:e10240 (2010)) and conditional ablation of PERK in the mammary epithelium delayed mammary carcinogenesis promoted by the HER2 oncogene (Bobrovnikova-Marjon et al., "PERK Promotes Cancer Cell Proliferation and Tumor Growth by Limiting Oxidative DNA Damage," Oncogene 29:3881-3895 (2004) and Bobrovnikova-Marjon et al., "PERK-Dependent Regulation of Lipogenesis During Mouse Mammary Gland Development and Adipocyte Differentiation," Proc. Nat'l. Acad. Sci. U.S.A. 105:16314-16319 (2008)). Further, HER2 increases the levels of proteotoxicity in tumor cells activating JNK and IRE signaling and allowing HER2⁺ cancer cells to cope with this stress (Singh et al., "HER2-mTOR Signaling-Driven Breast Cancer Cells Require ER-Associated Degradation to Survive," Sci. Signal. 8:ra52 (2015)). Accordingly, the cBIO database (Cerami et al., "The cBio Cancer Genomics Portal: An Open Platform for Exploring Multidimensional Cancer Genomics Data," Cancer Discov. 2:401-404 (2012)) showed that -14% of HER2 amplified human breast tumors display upregulation of PERK mRNA, further supporting the notion that HER2⁺ tumors may be dependent on PERK and/or other UPR pathways for survival.

Dormant (quiescent) tumor cells have also been shown to be dependent on PERK and ATF6 signaling for survival (Ranganathan et al., "Dual Function of Pancreatic Endoplasmic Reticulum Kinase in Tumor Cell Growth Arrest and Survival," Cancer Res. 68:3260-3268 (2008); Ranganathan et al., "Functional Coupling of p38-Induced Up-Regulation of BiP and Activation of RNA-Dependent Protein Kinase-Like Endoplasmic Reticulum Kinase to Drug Resistance of Dormant Carcinoma Cells," Cancer Res. 66:1702-1711 (2006); and Schewe et al., "ATF6alpha-Rheb-mTOR Signaling Promotes Survival of Dormant Tumor Cells In Vivo," Proc. Nat'l. Acad. Sci. U.S.A. 105:10519-10524(2008)). Quiescent pancreatic disseminated cancer cells ("DCCs") in livers also displayed a PERK-dependent UPR that was linked to loss of E-cadherin expression and downregulation of MHC-I, favoring immune evasion during dormancy (Pommier et al., "Unresolved Endoplasmic Reticulum Stress Engenders Immune-Resistant, Latent Pancreatic Cancer Metastases," Science 360(6394):eaao4908 (2018)). In the MMTV-HER2 model, quiescent DCCs in bone marrow and lungs were also found to be E-cadherin negative (Harper et al., "Mechanism of Early Dissemination and Metastasis in Her2+ Mammary Cancer," Nature 540:588-592 (2016)) but the link to the UPR was not tested. Together these data suggest that the UPR may serve as a stress and immune microenvironmental adaptive survival mechanism for DCCs.

The present disclosure is directed to overcoming deficiencies in the art. Kobayashi et al (2011) J Exp Med 208(13):2641-2655 relates to Bone morphogenetic protein 7 in dormancy and metastasis of prostate cancer stem-like cells in bone.
Buijs et al (2007) Am J Pathol 171(3):1047-1057 discusses that BMP7, a putative regulator of epithelial homeostasis in the human prostate, is a potent inhibitor of prostate cancer bone metastasis *in vivo.*
WO 2005/030933 A2 relates to methods for diagnosing and treating cancer based on the property of bone morphogenetic protein-7 (BMP-7) to inhibit or reverse progression of a cancer cell to a more malignant state.
Buijs et al (2007) Clin Exp Metastasis 24(8):609-617 relates to TGF-beta and BMP7 interactions in tumour progression and bone metastasis.
Boon et al (2011) Cytokine Growth Factor Rev 22(4):221-229 relates to Bone morphogenetic protein 7: a broad-spectrum growth factor with multiple target therapeutic potency.
Buijs et al (2012) Oncogene 31(17):2164-2174 discusses that the BMP2/7 heterodimer inhibits the human breast cancer stem cell subpopulation and bone metastases formation.
Aguirre-Ghiso et al (2013) Nat Med 19(3):276-277 relates to targeting dormant cancer.
Tate et al (2012) Cell Death Differ 19(10):1644-1654 discusses that a BMP7 variant inhibits the tumorigenic potential of glioblastoma stem-like cells.
Sandoval et al discusses PERK-Inhibition as a possible therapy for hypoxia-induced solitary dormant tumor cells (In: Proceedings of the AACR Special Conference on Tumor Metastasis; 2015 Nov 30-Dec 3; Austin, TX. Philadelphia (PA): AACR; Cancer Res 2016;76(7 Suppl):Abstract nr A45).
Sosa et al (2014) Nat Rev Cancer 14(9):611-622 relates to mechanisms of disseminated cancer cell dormancy.
US 2010/0167945 A1 relates to compositions and methods for detecting the activation states of components of signal transduction pathways in tumor cells.
WO 2018/194885 A1 relates to phenyl-2-hydroxy-acetylamino-2-methyl-phenyl compounds, to pharmaceutical compositions comprising the compounds, and to methods of using the compounds to treat physiological disorders such as cancer.

### SUMMARY

The invention is as set out in the appended set of claims. The references in this description to methods of treatment by therapy are to be interpreted as references to compounds of the present invention for use in those methods. Otherwise disclosed herein is a method of treating minimal residual cancer in a subject, which method involves contacting disseminated cancer cells (DCCs) in a subject with a bone morphogenic protein 7 ("BMP7") derivative protein, where said contacting induces or maintains dormancy in the contacted DCCs of the subject to treat minimal residual cancer in the subject. Methods of this aspect may be utilized to prevent minimal residual cancer from progressing to aggressive growth in the subject.

The claims relate to a protein kinase RNA-like endoplasmic reticulum kinase (PERK) inhibitor for use in a method of treating minimal residual cancer in a subject, which method involves contacting disseminated cancer cells (DCCs) in a subject with a protein kinase RNA-like endoplasmic reticulum kinase ("PERK") inhibitor selected from LY2, LY3, and LY4, where said contacting eradicates DCCs in the subject to treat minimal residual cancer in the subj ect.

Otherwise disclosed herein is another aspect that relates to a method of treating late stage cancer in a subject. This method involves contacting disseminated cancer cells (DCCs) in a subject with a protein kinase RNA-like endoplasmic reticulum kinase (PERK) inhibitor selected from LY2, LY3, and LY4, where said contacting eradicates DCCs in the subject to treat late stage cancer in the subject.

Described *infra* is, among other things, the demonstration that LY4, a selective and potent inhibitor of PERK, can block HER2-driven metastasis as a result of its ability to specifically cause the eradication of dormant DCCs. As of this disclosure, PERK inhibitors represent a new strategy to target solitary dormant cells during minimal residual disease stages, either alone or in combination with anti-proliferative therapies to help prevent lethal metastases. Also described *infra* is the demonstration that bone morphogenic derivative proteins can induce dormancy in disseminated tumor cells.

### BRIEF DESCRIPTION OF THE DRAWINGS

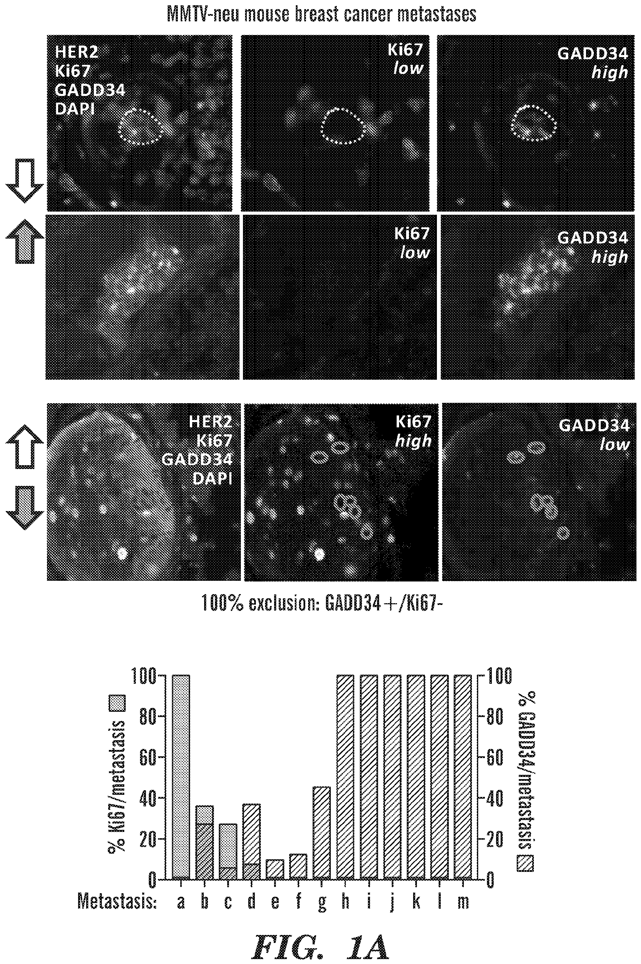

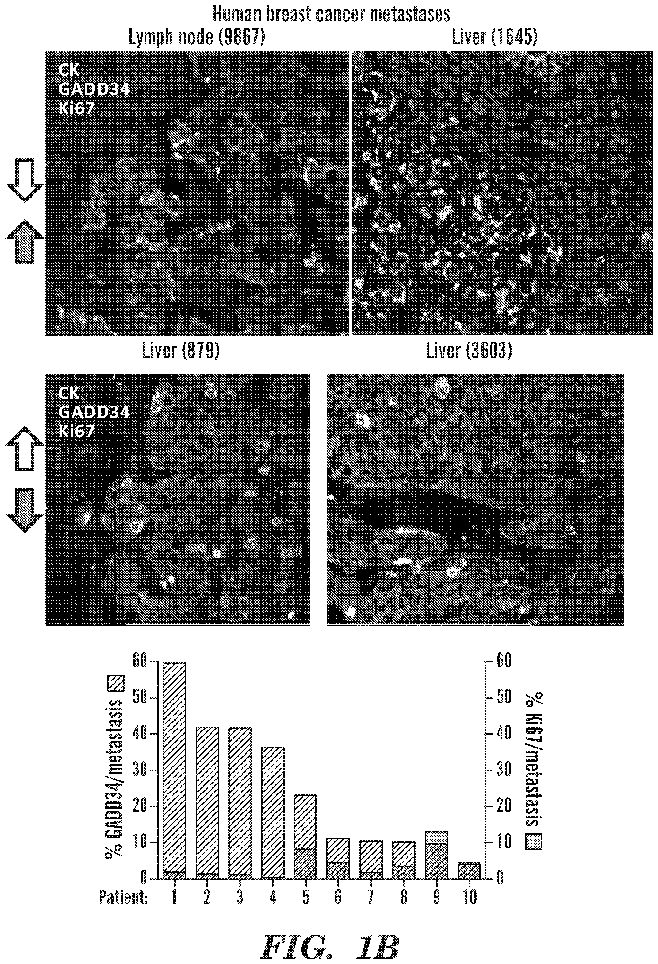

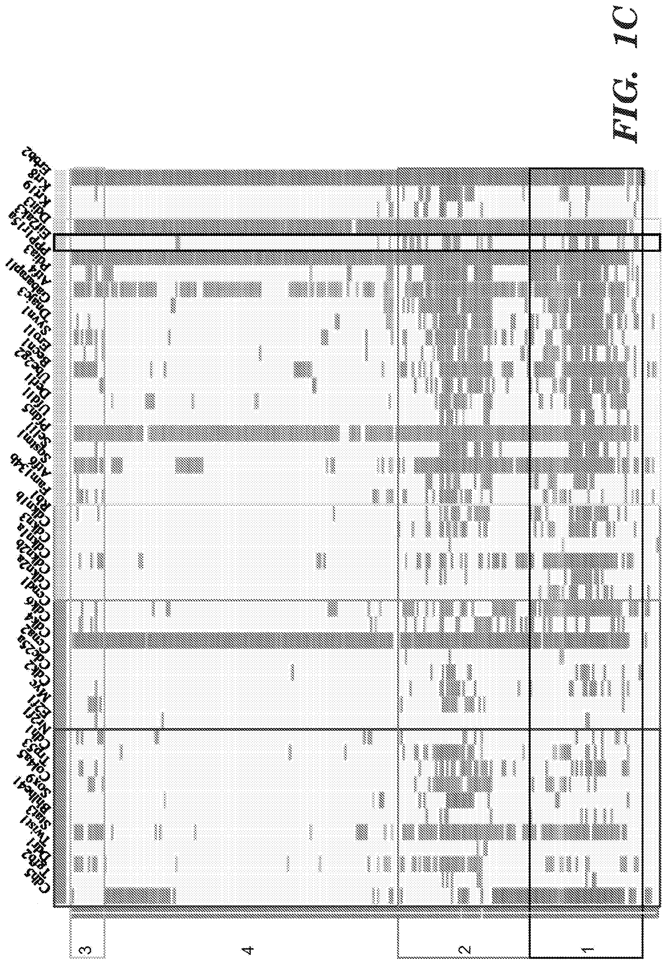

FIGs. 1A-1C show that quiescent disseminated HER2⁺ cells display high levels of ER stress pathway activation. FIG. 1A shows images of lung sections of MMTV-HER2 animals stained for HER2, Ki67 (proliferation), and GADD34 (ER stress). The graph in FIG. 1A shows the quantification of cells/metastasis positive for both markers as a percentage of total cells. FIG. 1B shows images of human breast cancer metastasis from different locations (lymph node, liver, lung) stained for cytokeratins, Ki67 (proliferation), and GADD34 (ER stress). The graph in FIG. 1B shows the quantification of cells/metastasis positive for both markers as a percentage of total cells. FIG. 1C shows hierarchical clustering of the high-throughput targeted gene expression (columns) profile of single cells (lung disseminated tumor cells ("DTCs")) (rows).

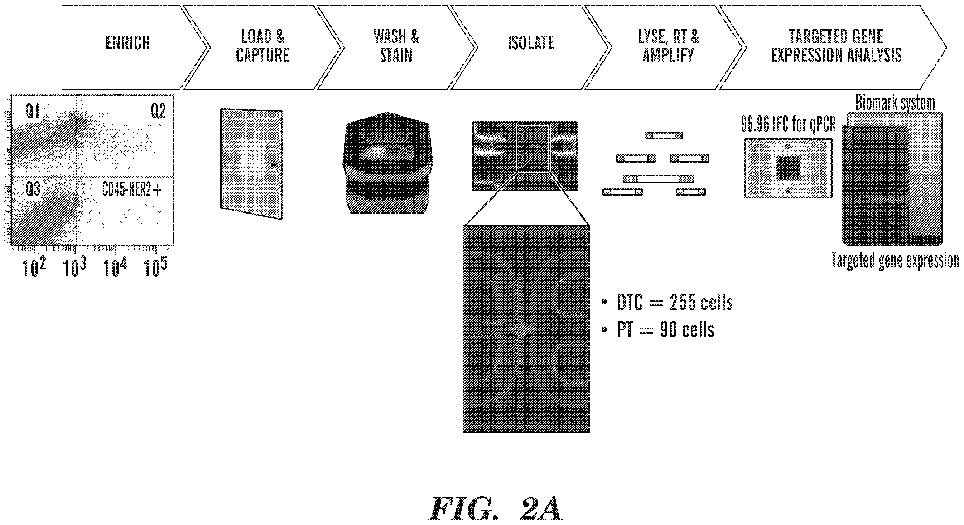

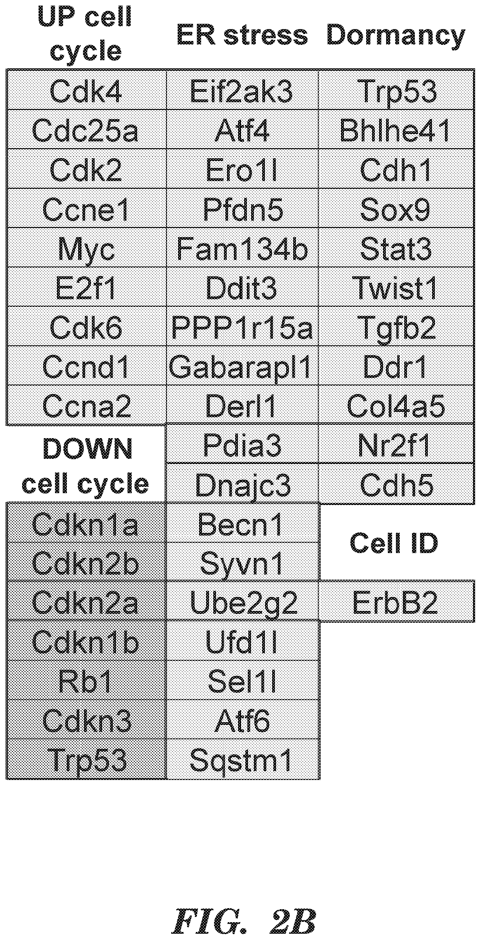

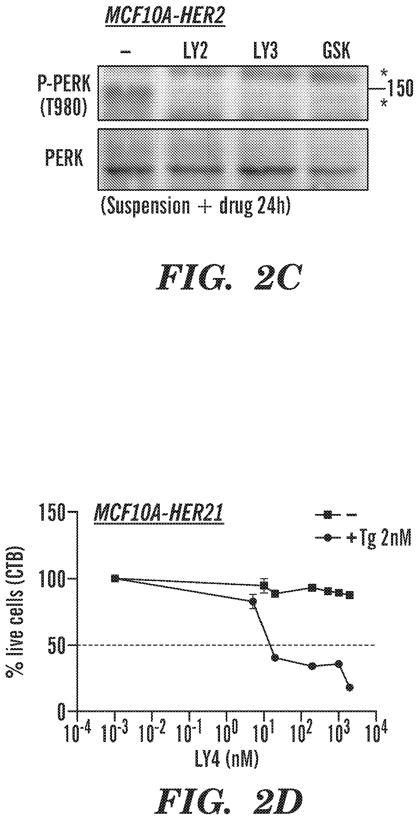

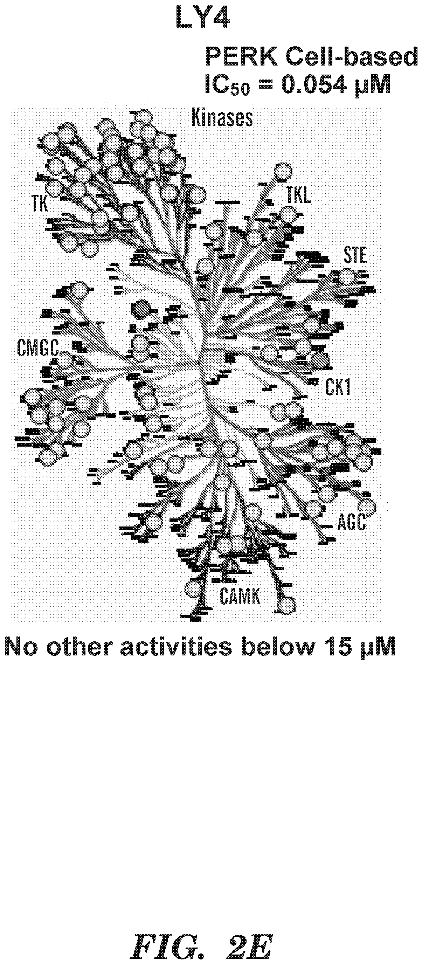

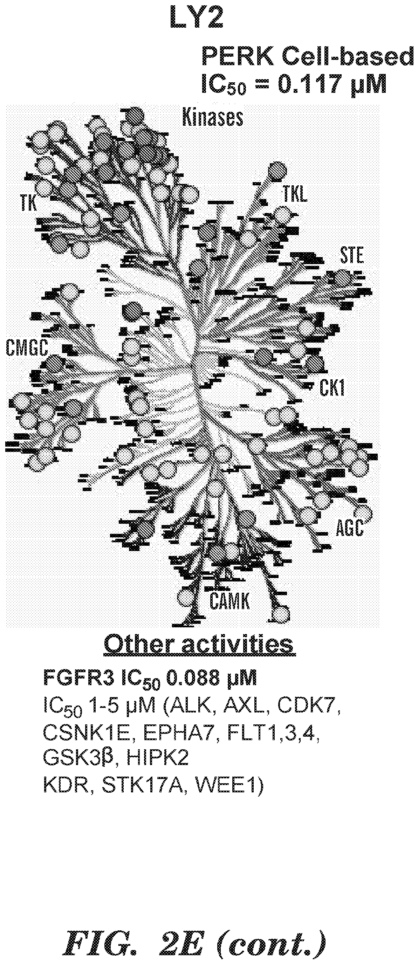

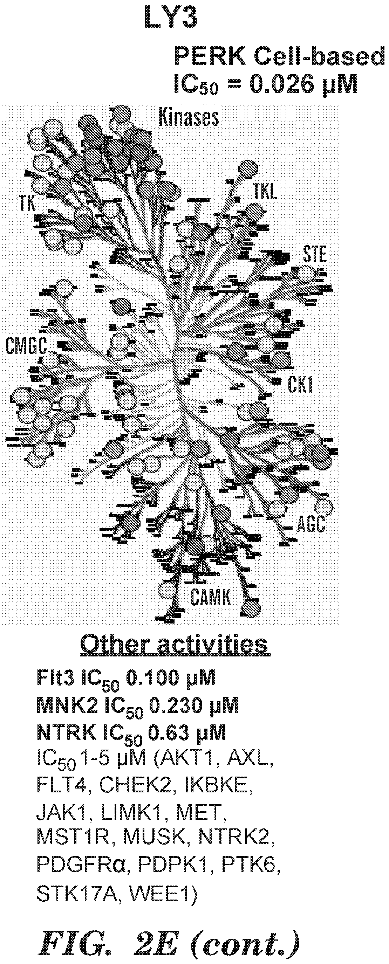

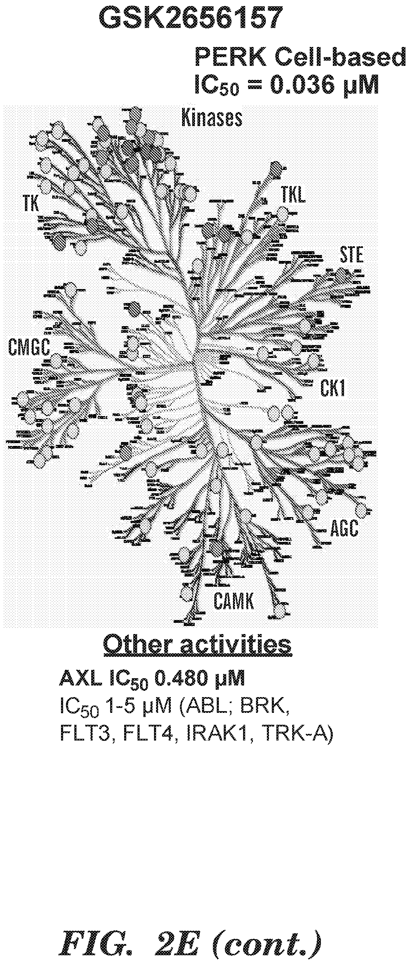

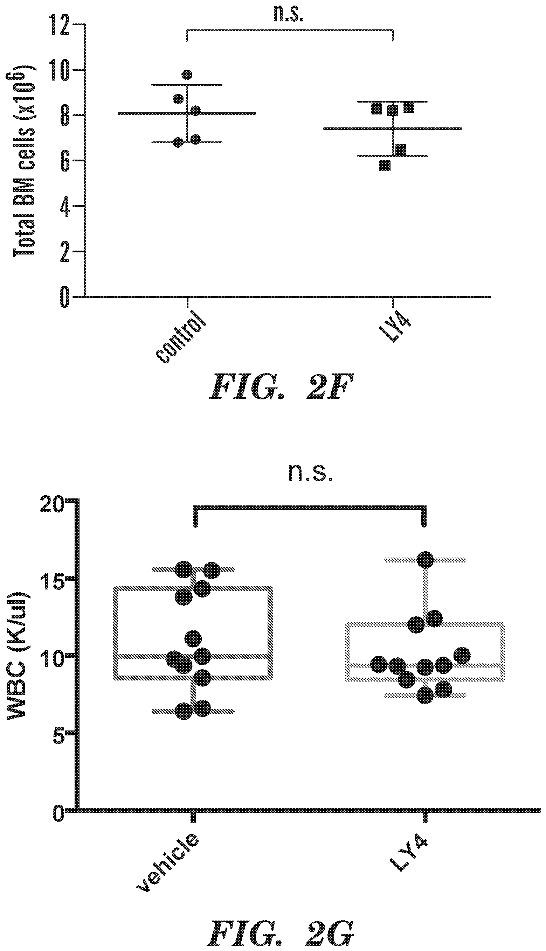

FIGs. 2A-2G demonstrate that PERK inhibition is upregulated in a HER2⁺ cancer cell patient. FIG. 2A is a flow diagram of the steps followed for single cell gene expression analysis with C1 and Biomark HD Fluidigm. A total of 255 DCCs and 90 primary tumor ("PT") cells were analyzed. FIG. 2B shows a list of the genes analyzed by high-throughput qPCR. FIG. 2C is an immunoblot showing the inhibition of PERK phosphorylation by LY series inhibitors (LY2, LY3, and LY4) and GSK2656157 (2 µM) in MCF10A-HER2 cells stressed by placing them in suspension for 24 hours. indicates nonspecific bands. FIG. 2D shows a LY4 dose-response cell viability curve (Cell Titer Blue, CTB) in MCF10A-HER2 cells, in the absence (-) or in the presence of stress (low dose thapsigargin, Tg 2 nM) after 48 hours. The dashed line indicates IC₅₀ (≈ 9 nM). FIG. 2E shows the kinase selectivity of PERK inhibitors LY4, LY2, LY3, and GSK2656157 as evaluated by enzymatic biochemical assay. FIG. 2F shows the effect of LY4 on total bone marrow cells (in two lower limbs) in MMTV-HER2 females treated for 2 weeks. FIG. 2G shows the effect of LY4 on total white blood cells in MMTV-HER2 females treated for 2 weeks.

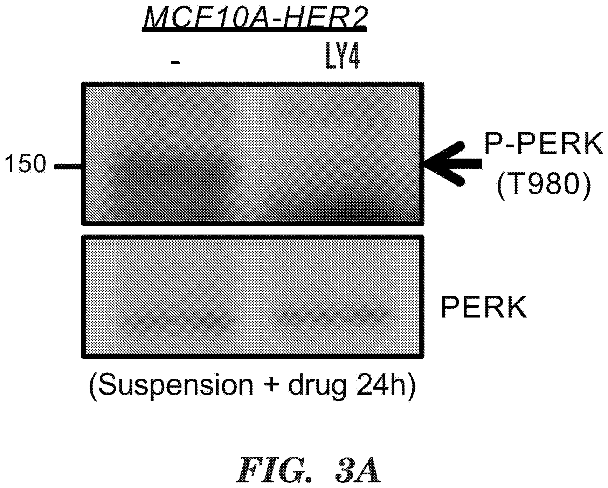

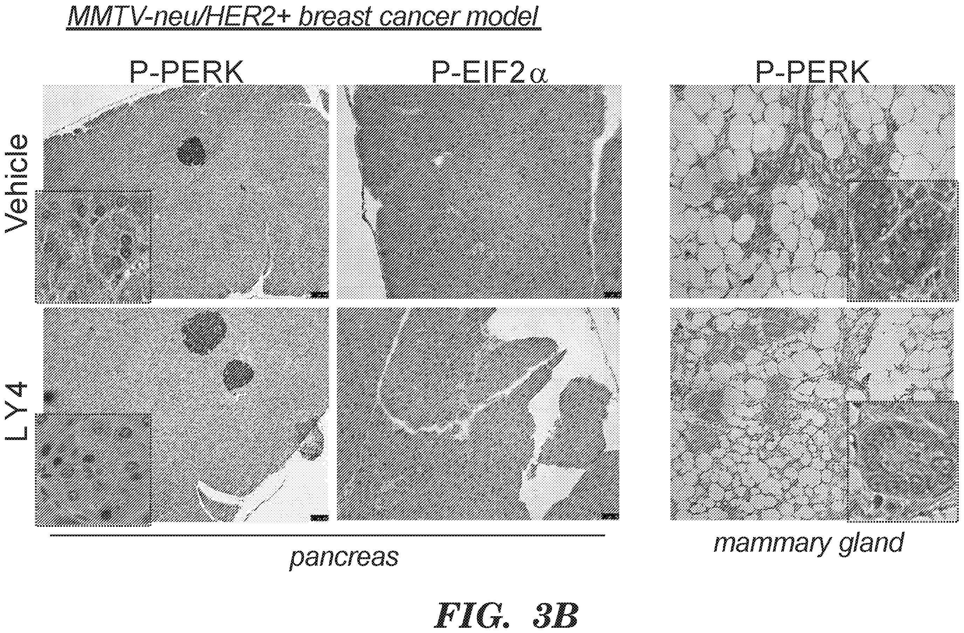

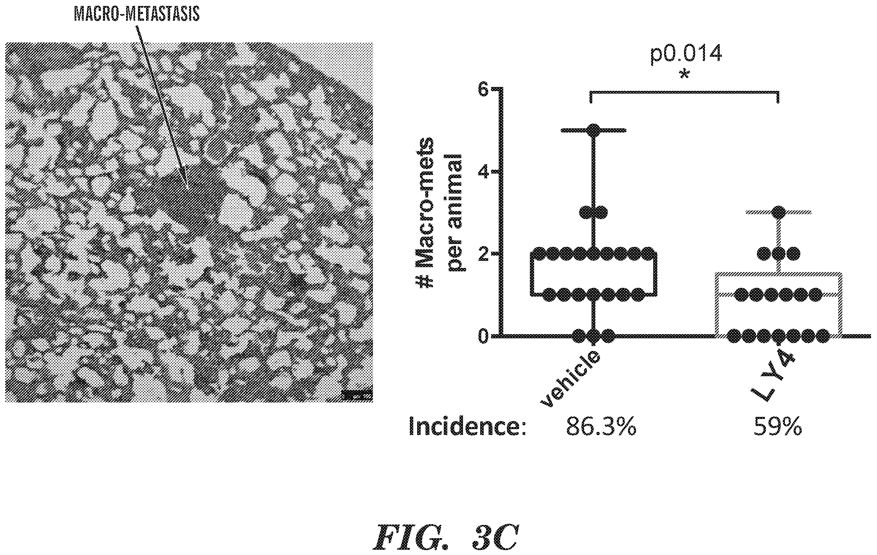

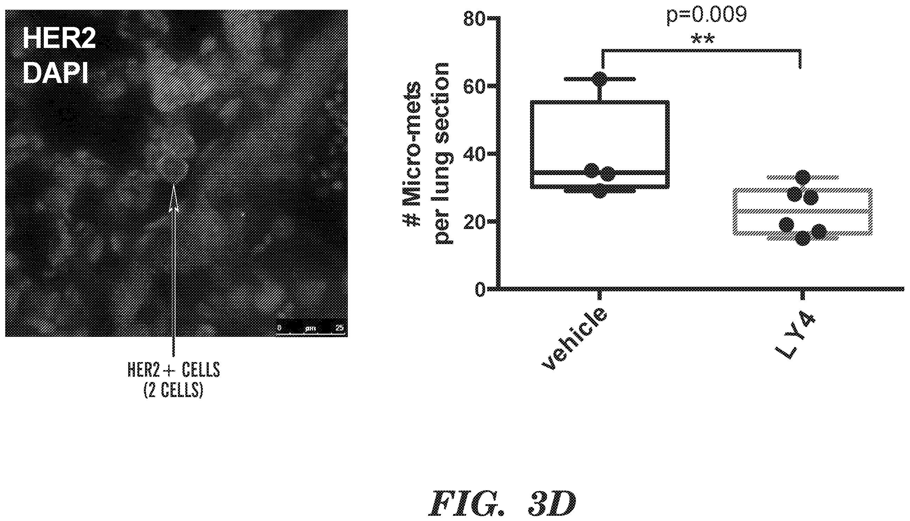

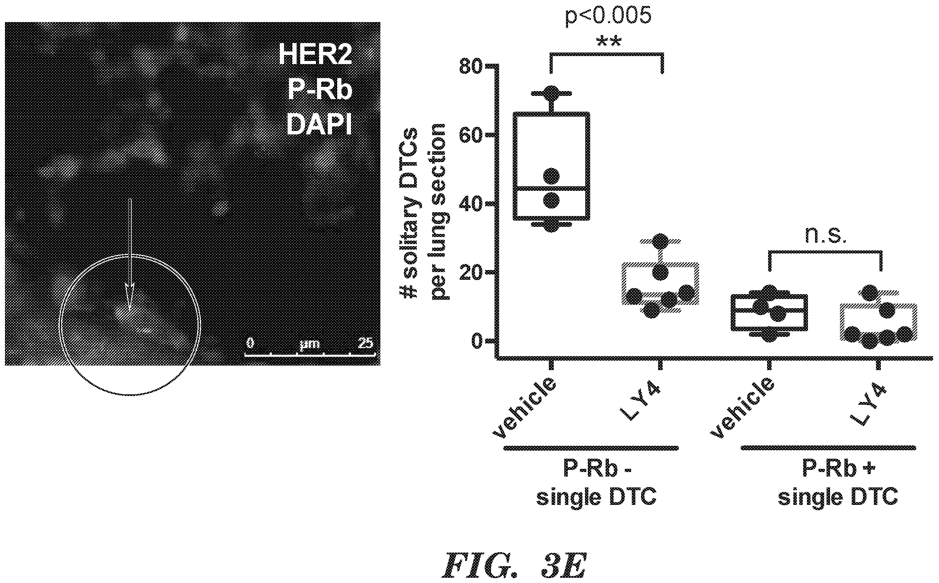

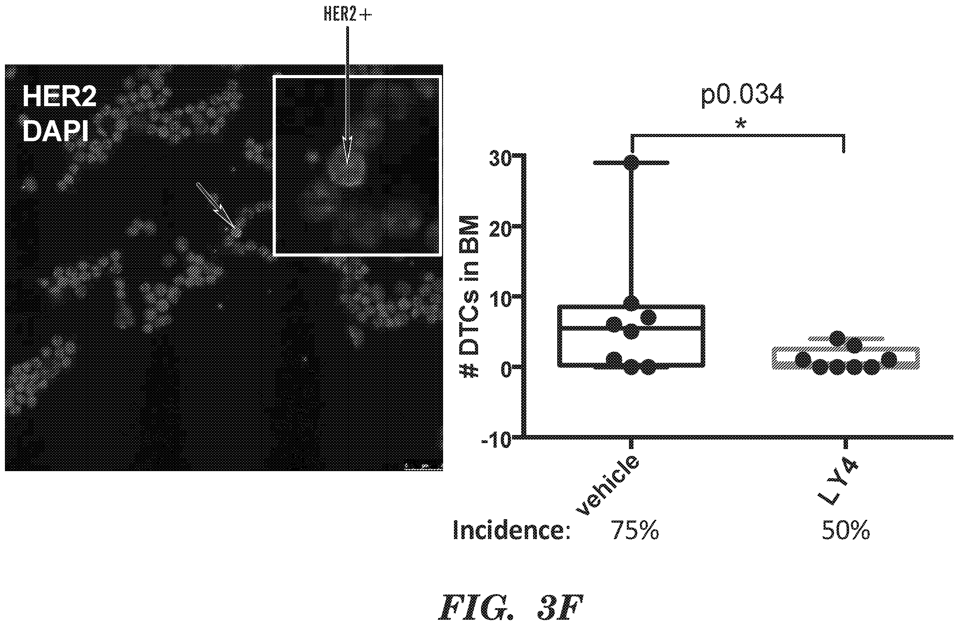

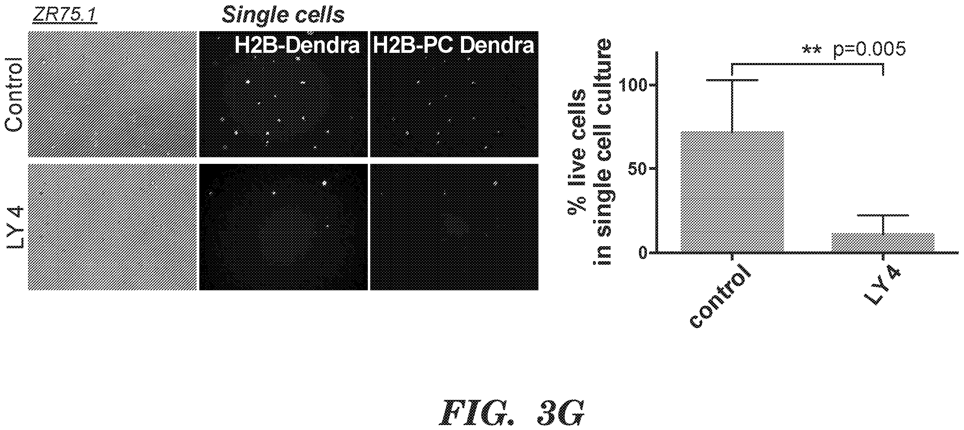

FIGS. 3A-3G show that LY4 PERK inhibition decreases metastatic disease in lungs and bone marrow at the single disseminated tumor cell level. FIG. 3A is an immunoblot showing the inhibition of PERK phosphorylation (T980) by the PERK inhibitor LY4 (2 µM) in MCF10A-HER2 cells serum-starved overnight and treated with EGF (100 ng/ml) for 15 min. In FIG. 3B, MMTV-HER2⁺ females (24-week old) were injected daily with vehicle or LY4 (50 mpk) for 2 weeks. Immunohistochemistry ("IHC") of pancreas and mammary gland sections with antibodies to P-PERK and P-EIF2α are shown. Inserts show higher magnifications. Scale bars, 100 µm. FIG. 3C shows an image and quantification (right graph) of macro-metastases (>100 cells) detected by H&E staining and quantified in 5 lung sections/animal (n=16). Scale bar, 100 µm. p by Mann-Whitney test. FIG. 3D shows an image and quantification (right graph) of micro-metastases (2-100 cells) detected by IHC staining using an anti-HER2 antibody, and quantified per lung section/animal ± s.d. (n=6). Scale bar, 25 µm. p by Mann-Whitney test. FIG. 3E shows an image and quantification (right graph) of solitary disseminated tumor cells (DTCs) detected by IHC staining for HER2, classified as P-Rb⁺ or P-Rb⁻ and quantified per lung section ± s.d. (n=6). Scale bar, 25 µm. p by Mann-Whitney test. Arrow and circle in the image indicate a solitary DTC. FIG. 3F shows an image and quantification (right graph) of disseminated tumor cells in bone marrow detected by IF staining for CK8/18 and HER2 in cytospins from mature hematopoietic cell-depleted bone marrow tissue (n=8). Scale bar, 25 µm. p by Mann-Whitney test. Arrows indicate Her2⁺ cells. FIG. 3G shows representative images of ZR75.1 HER2⁺ cells engineered to express Dendra-tagged H2B proteins seeded on Matrigel at low density (single cells). At day 0, the Dendra tag (green fluorescence) was photoconverted with a single UV-light pulse to red fluorescence, and used as an indication of quiescence. Wells were treated from day 2 to day 8 with vehicle (DMSO) or LY4 (2 µM). The graphs show the percentage of live cells on day 8 measured ± s.d. (n=4). p by Student's t test.

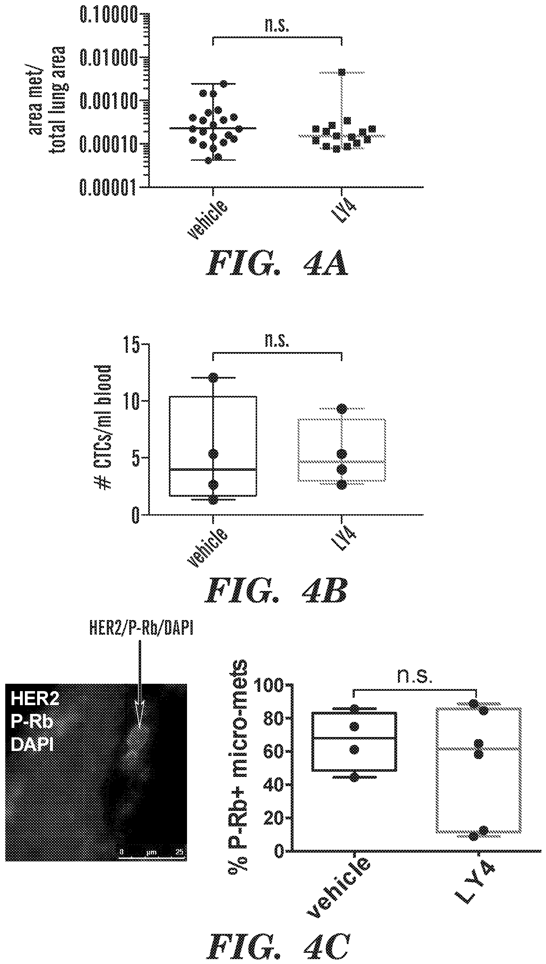

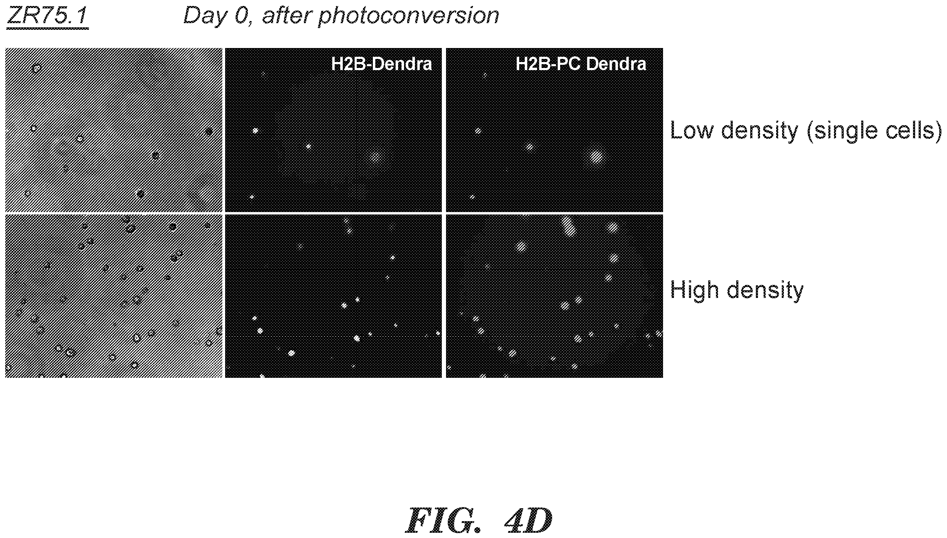

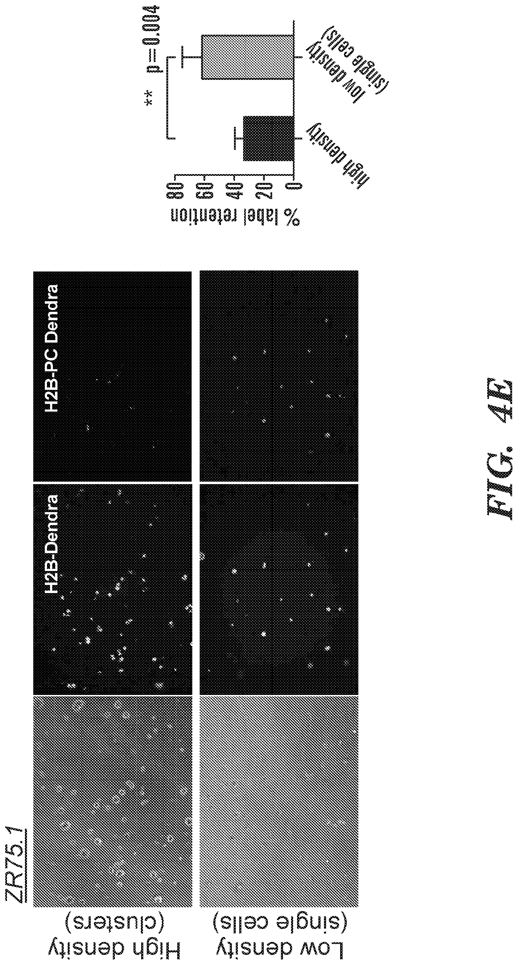

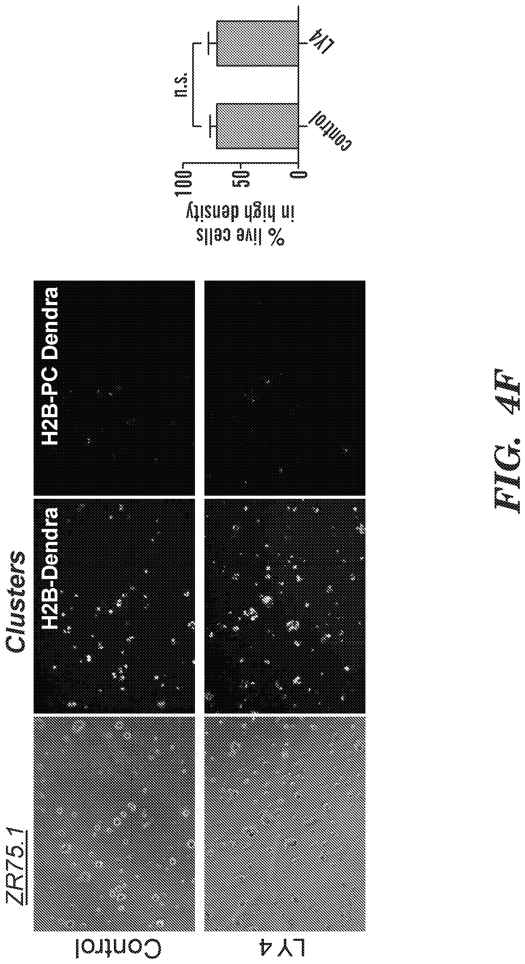

FIGs. 4A-4F show the effect of LY4 treatment on metastasis and circulating tumor cells ("CTCs"). FIG. 4A shows the normalized area of single macro-metastases in vehicle and LY4-treated animals (n=21 and 15). p by Mann-Whitney test. FIG. 4B is a graph showing the quantification of circulating tumor cells/ml blood by HER2 staining of cytospins. FIG. 4C shows an image and graph showing the percentage of P-Rb⁺ micro-metastasis per lung section/animal (n=4 and 6). FIG. 4D is an image showing 100% photoconversion in ZR75.1-H2B-Dendra from green fluorescence to red fluorescence at day 0, after seeding in 3D Matrigel. In FIG. 4E, ZR75.1-H2B-Dendra photoconverted cells were seeded at low (single cells) or high density. The graph shows the percentage of red label retention in cells seeded as single cells or at high density ± s.d. (n=4). p by Student's t test. FIG. 4F is as in FIG. 4D, but cells seeded at high density were treated from day 2 to day 8 with vehicle (DMSO) or LY4 (2 µM). The graph shows the percentage of live colonies was measured ± s.d. on day 8 (n=4). p by Student's t test.

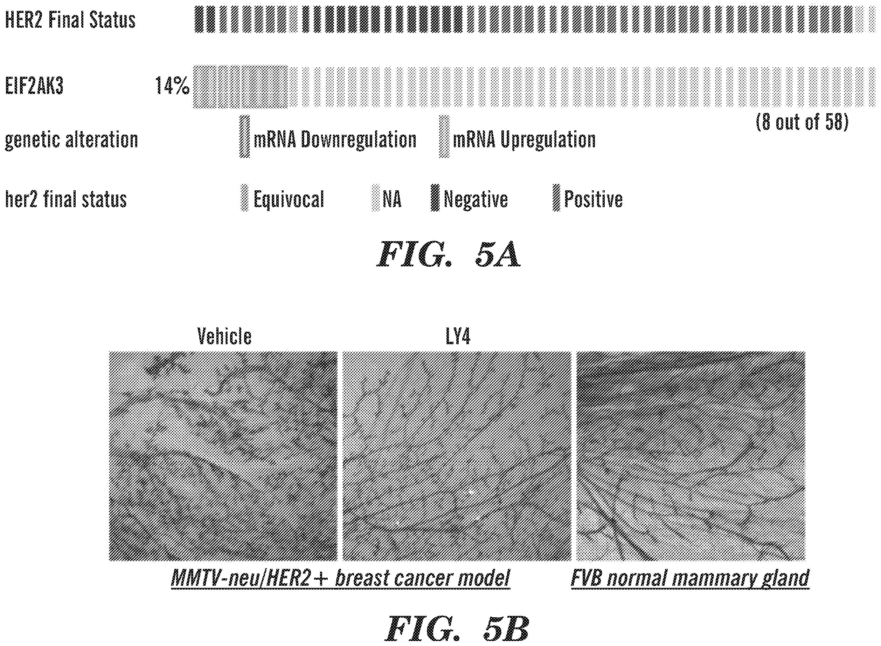

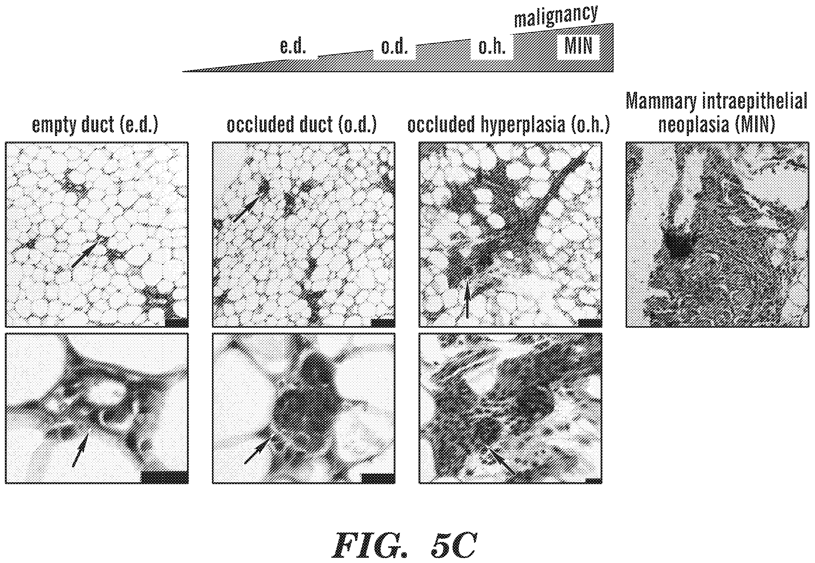

FIGs. 5A-5C demonstrate that PERK inhibition is upregulated in Her2⁺ cells. FIG. 5A shows that PERK (EIF2AK3) is upregulated in a sub-population of HER2⁺ breast cancer patients. Analysis of TCGA breast cancer data HER2⁺ cases (58 tumors) using cBioPortal. FIG. 5B shows representative images of carmine staining of a whole mount FVB normal mammary gland compared to vehicle- and LY4-treated MMTV-neu mammary gland whole mount. FIG. 5C shows the quantification of histological structures (from normal empty duct to DCIS-like mammary intraepithelial neoplasia), top images and higher magnifications in lower row) present in H&E stained mammary gland sections.

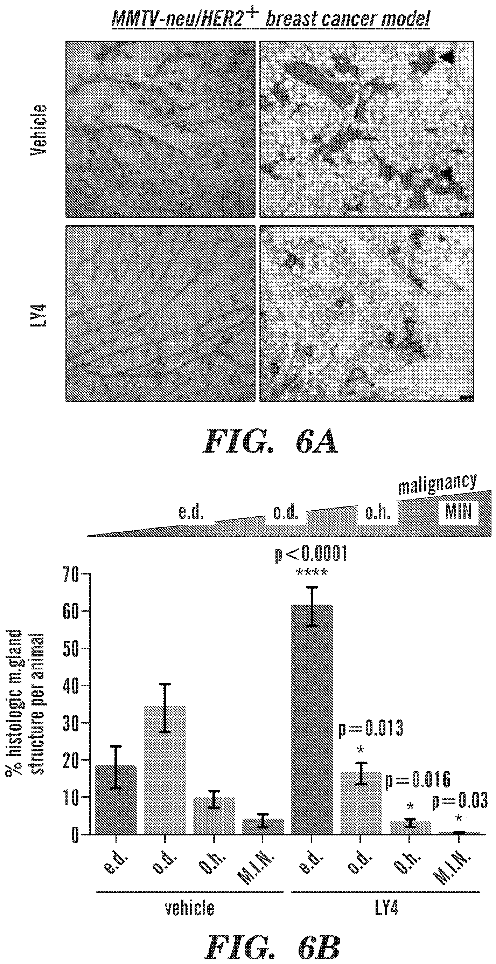

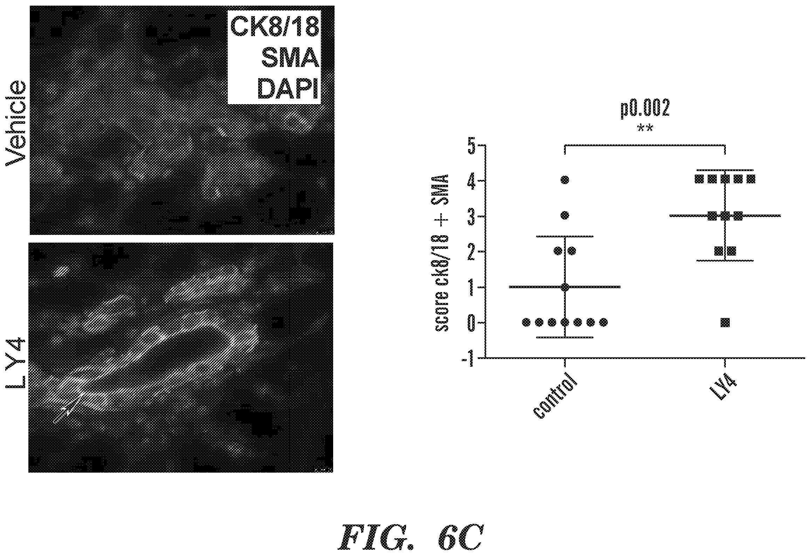

FIGs. 6A-6C show that the PERK inhibitor LY4 causes mammary gland "normalization" in the MMTV-HER2⁺ breast cancer model. FIG. 6A shows representative images of carmine staining of whole mount mammary glands and H&E-stained mammary gland sections from vehicle- and LY4-treated animals. Scale bar, 100 µm. FIG. 6B shows the quantification of histological structures (empty duct e.d., occluded duct o.d., occluded hyperplasia o.h., and DCIS-like mammary intraepithelial neoplasia M.I.N) present in H&E-stained mammary gland sections (n=50/animal, animals n=13) found in vehicle- and LY4-treated animals ± s.e.m. Statistical significance (p) calculated by Mann-Whitney test. FIG. 6C shows IHC for epithelial luminal marker cytokeratin 8/18 (CK8/18) and myoepithelial marker Smooth Muscle actin ("SMA") in mammary gland sections. The graph shows the score for CK8/18⁺ and SMA⁺ structures per animal, n=12. p by Mann-Whitney test. Scale bar, 75 µm.

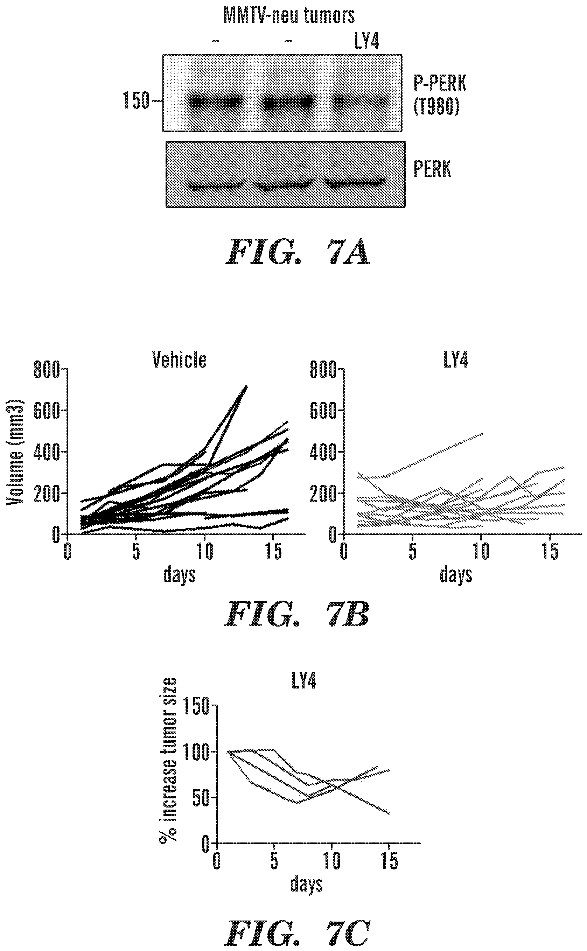

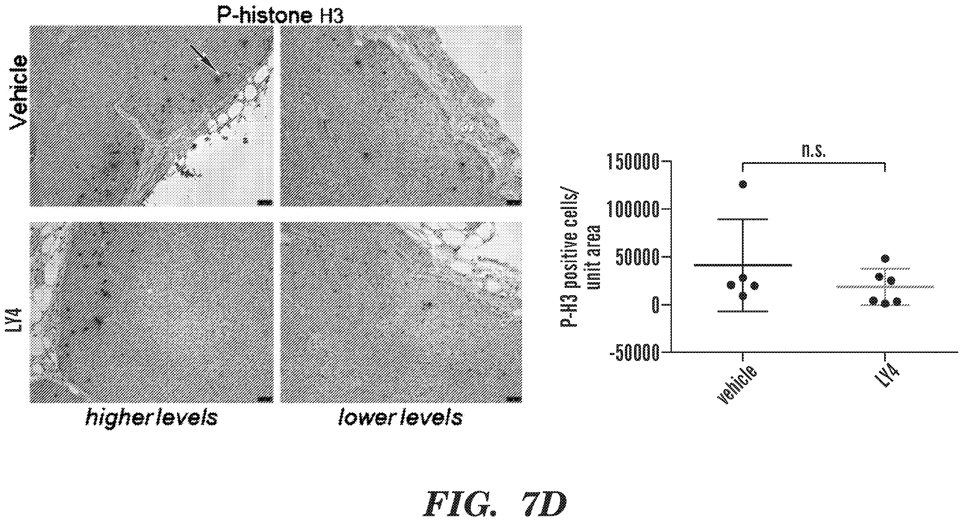

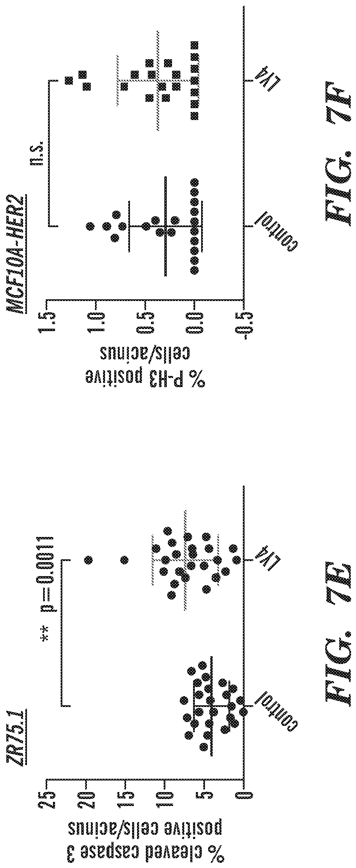

FIGs. 7A-7F show the effect of LY4 treatment on P-PERK levels, P-histone H3 levels, and tumor size. FIG. 7A is a Western blot for P-PERK levels in MMTV-neu tumor lysates from vehicle- and LY4-treated animals. FIG. 7B shows tumor volumes from vehicle-(upper) and LY4-treated (lower) females (mm³). Each line represents a tumor. FIG. 7C shows the percentage decrease in tumor size in LY4-treated females that showed tumor shrinkage. Each line represents a tumor and animal. FIG. 7D shows IHC for P-histone H3 in mammary gland tumor sections, representative images and quantification (right graph). p by Mann-Whitney test. In FIG. 7E, HER2-overexpressing ZR75.1 cells were seeded on matrigel and after acinus establishment (day 10) wells were treated with vehicle (control) or LY4 (2 µM) for 10 days. The graph shows the percentage of cleaved caspase-3 positive cells per acini (n=20) ± s.d. p by Student's t test. In FIG. 7F, MCF10A-HER2 cells were seeded on Matrigel and after acinus establishment (day 4) wells were treated with vehicle (control) or LY4 (2 µM) for 10 days. The graph shows the percentage of P-histone H3-positive cells per acini (n=20) ± s.d. p by Student's t test.

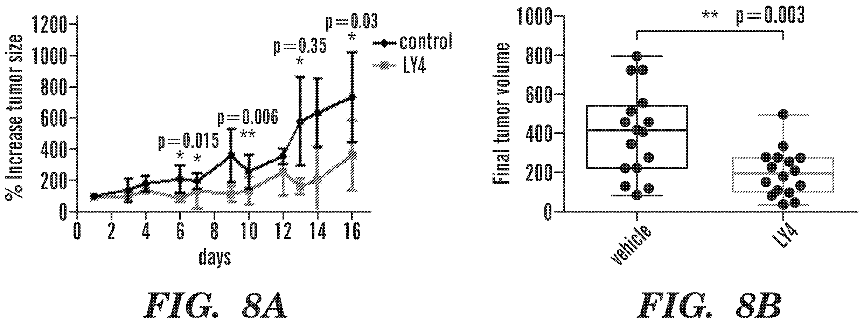

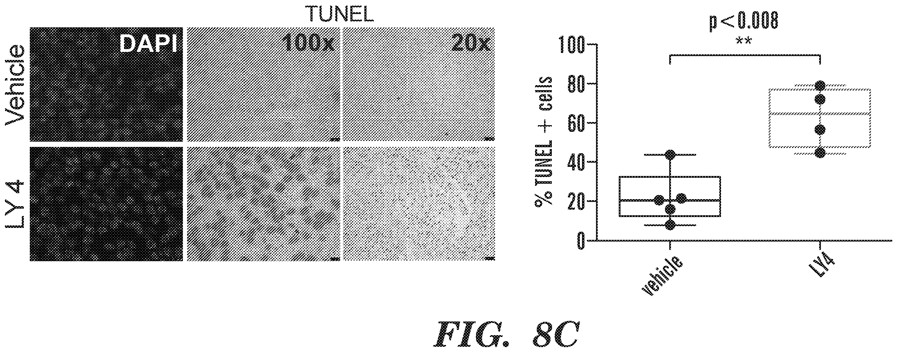

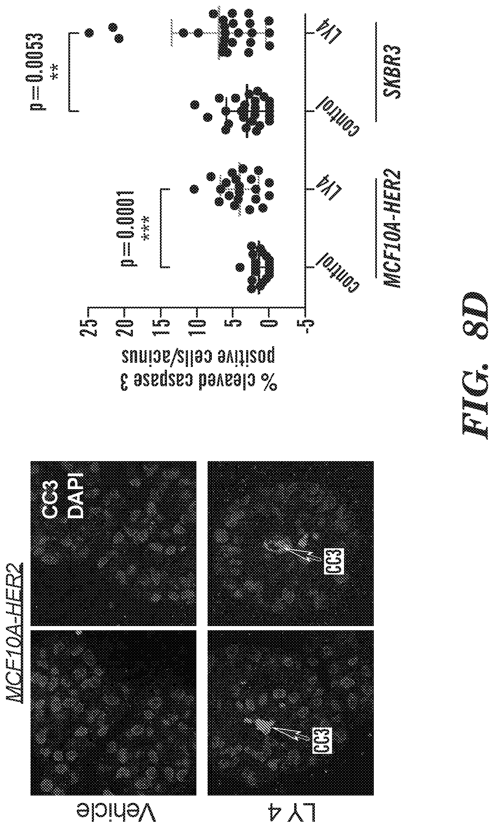

FIGs. 8A-8D show that PERK inhibition impairs tumor growth in MMTV-HER2⁺ females. In FIG. 8A, MMTV-neu females (24- to 32-week old) presenting overt tumors were injected daily with vehicle or LY4 (50 mpk) for 2 weeks. The graph shows the percentage variation of tumor size in vehicle- and LY4-treated animals ± s.d. (n=16). p by Mann-Whitney test. FIG. 8B is a graph showing final tumor volume (mm³). The whiskers represent the min and max of the data (n=16). p by Mann-Whitney test. FIG. 8C shows representative IHC of TUNEL staining to measure apoptosis levels in tumor sections. Scale bars, 10 and 50 µm. Graph, percentage TUNEL positive cells in vehicle- and LY4-treated tumor sections (n=5). p by Mann-Whitney test. In FIG. 8D, HER2⁺ MCF10A-HER2 or SKBR3 cells were seeded on Matrigel and after acinus establishment (day 4) wells were treated with vehicle (control) or LY4 (2 µM) for 10 days. The graph shows the percentage of cleaved caspase-3 positive cells per acini (n=20) ± s.d. p by Student's t test. Representative confocal images of MCF10A-HER2 acini stained for cleaved caspase-3.

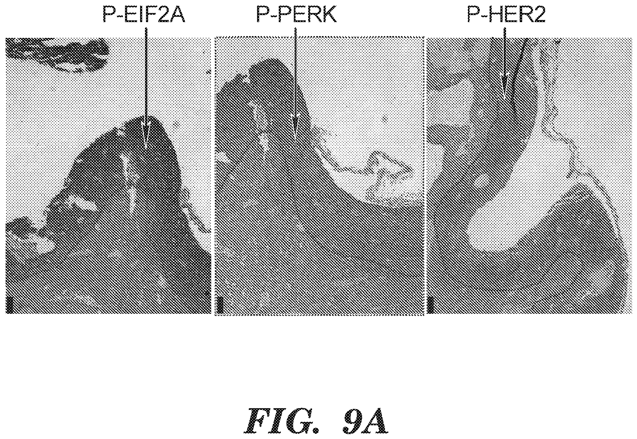

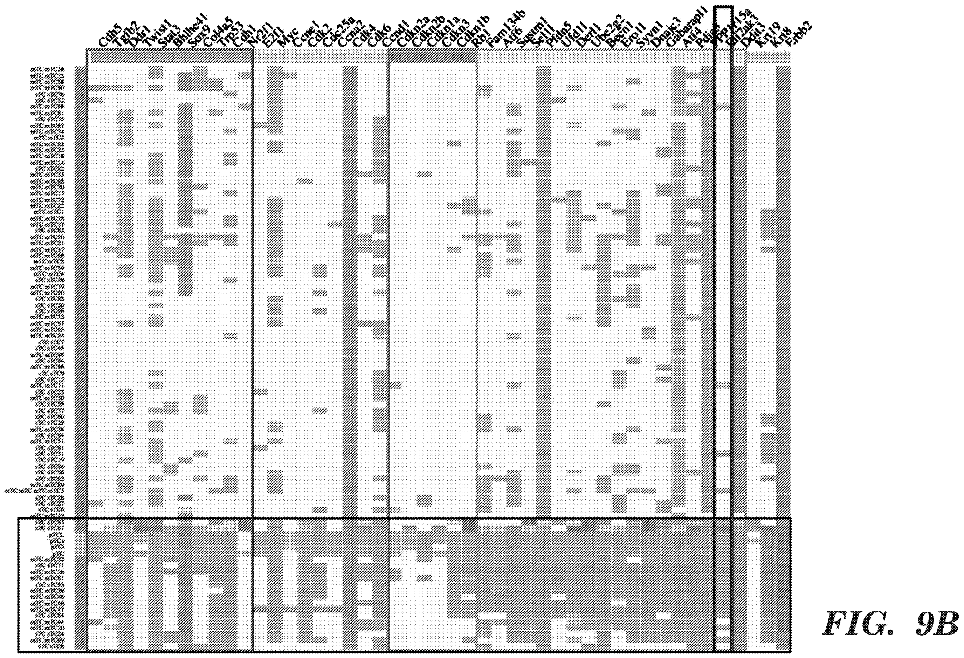

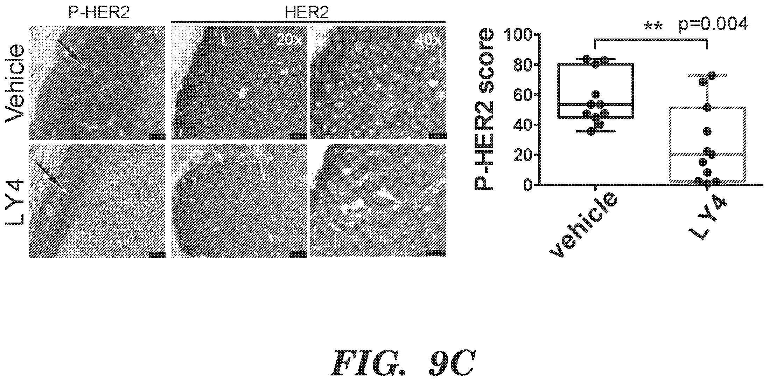

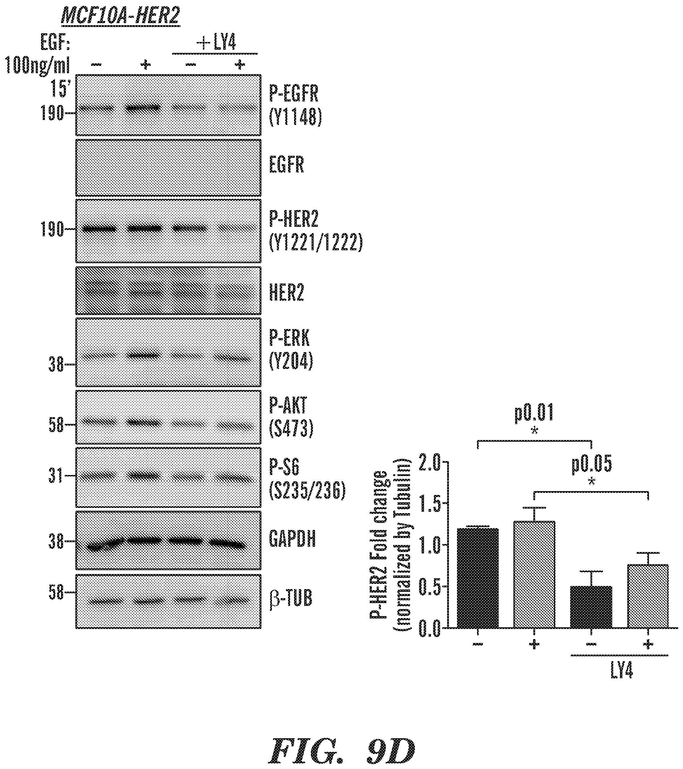

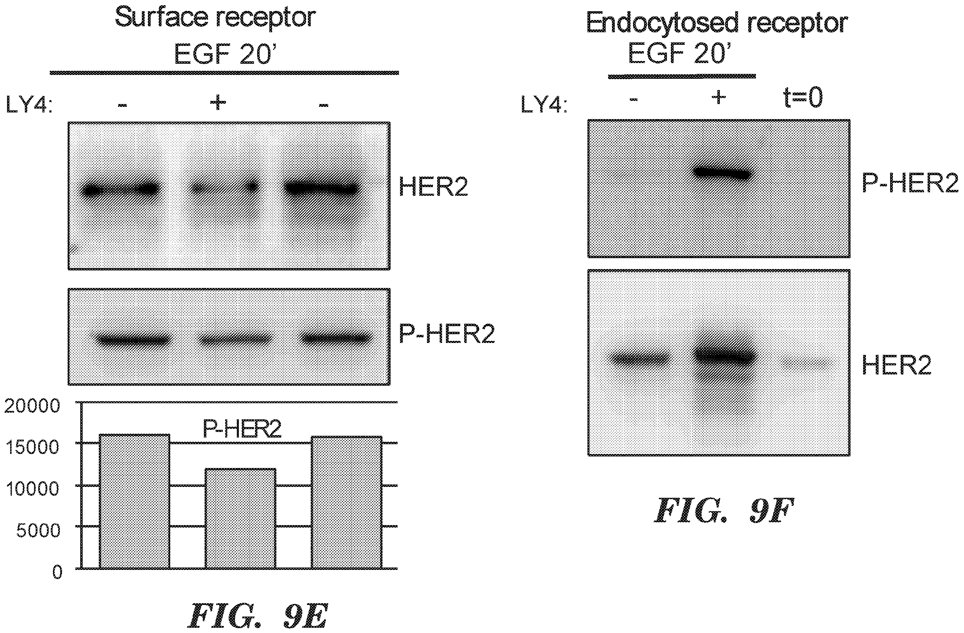

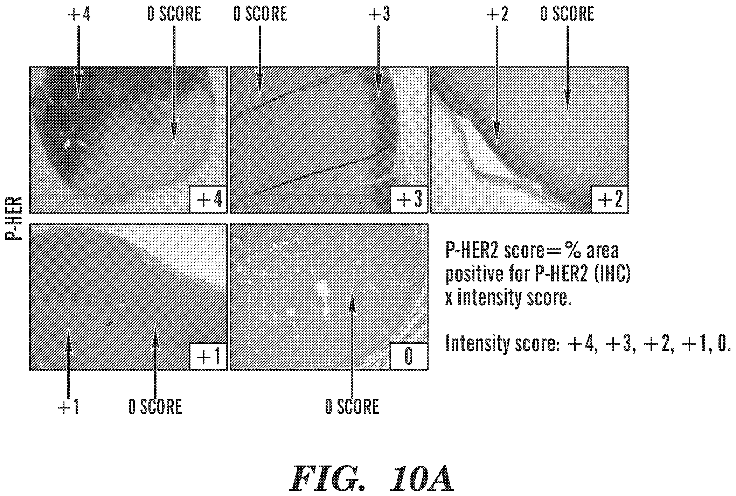

FIGs. 9A-9F show that LY4 treatment decreases the levels of phospho-HER2 and downstream signaling pathways. FIG. 9A shows representative images of IHC for P-HER2, P-PERK, and P-EIF2α in a MMTV-HER2 breast tumor section. Note that the rim positive for P-HER2 overlaps with P-PERK and P-EIF2α stainings. Scale bar, 100 µm. FIG. 9B shows hierarchical clustering of the high-throughput targeted gene expression (columns) profile of single cells (primary breast tumor) (rows) from MMTV-HER2 females. FIG. 9C shows representative P-HER2 and total HER2 IHC staining in vehicle- and LY4-treated breast tumors. The graph shows the P-HER2 score in vehicle and LY-treated tumors. Quantification of P-HER2 levels in tumor sections, by IHC intensity and area scoring (n=11) (*see* FIG. 10A). Scale bar, 50 µm. p by Mann-Whitney test. In FIG. 9D, MCF10A-HER2 cells were starved overnight and treated +/-LY4 (2 µM), after which +/-EGF (100 ng/ml) was added for 15 minutes before collection. The levels of P-HER2, P-EGFR, P-AKT, P-S6, and P-ERK, as well as total HER2 and EGFR were assessed by Western blot. GAPDH and β-TUB were used as loading controls. Representative blot of three is shown. Densitometry analysis for P-HER2 (n=3) ± s.d. p by Student's t test. In FIG. 9E, MCF10A-HER2 cells were treated as in FIG. 9D and surface receptor biotinylation assay was performed. Surface levels of total HER2 and P-HER2 were assessed. Densitometry for P-HER2 is shown. In FIG. 9F, MCF10A-HER2 cells were treated as in FIG. 9D and reversible surface receptor biotinylation assay was performed. Endocytosed levels of total HER2 and P-HER2 were assessed. One of two experiments shown.

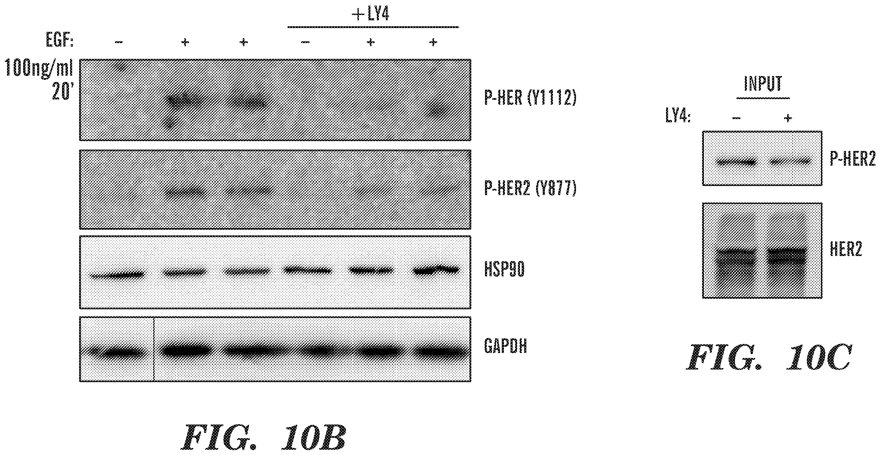

FIGs. 10A-10C show the quantification of P-HER2 levels in MCF10A-HER2 cells. FIG. 10A shows the scoring system used for the quantification of P-HER2 levels in mammary gland tumor sections. The IHC P-HER2 positive area was multiplied by its intensity score according to established score shown in these representative images. Scale bar, 100 µm. In FIG. 10B, MCF10A-HER2 cells were starved overnight and treated +/-LY4 (2 µM), after which +/-EGF (100 ng/ml) was added for 20 minutes before collection. The levels of P-HER2/Y1112 and P-HER2/Y877 were assessed by Western blot. GAPDH and HSP90 were used as loading controls. FIG. 10C shows the input for the extracts used in the surface biotinylation assay.

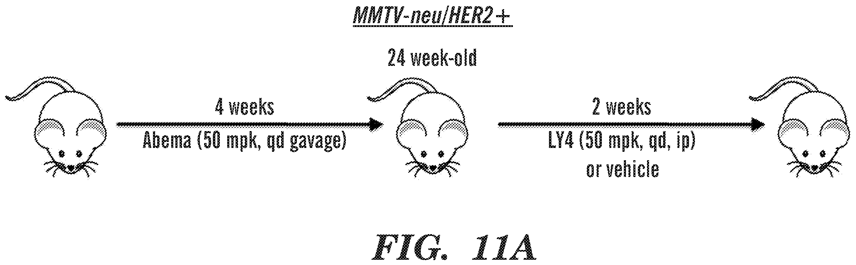

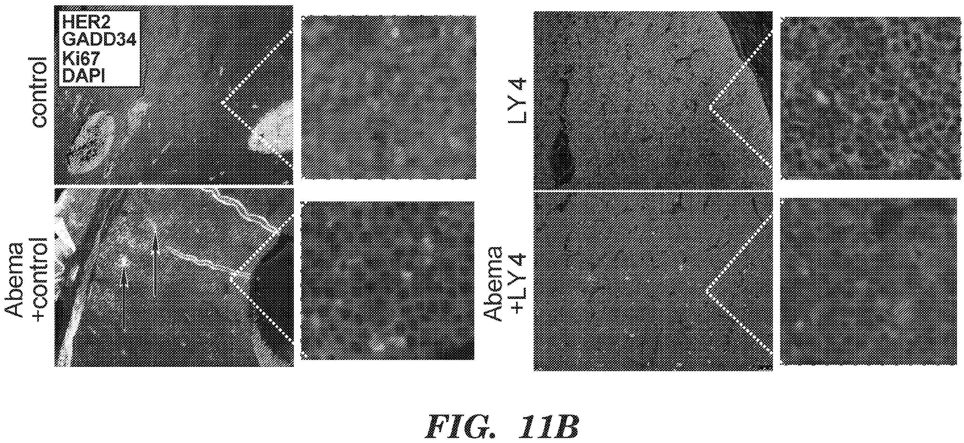

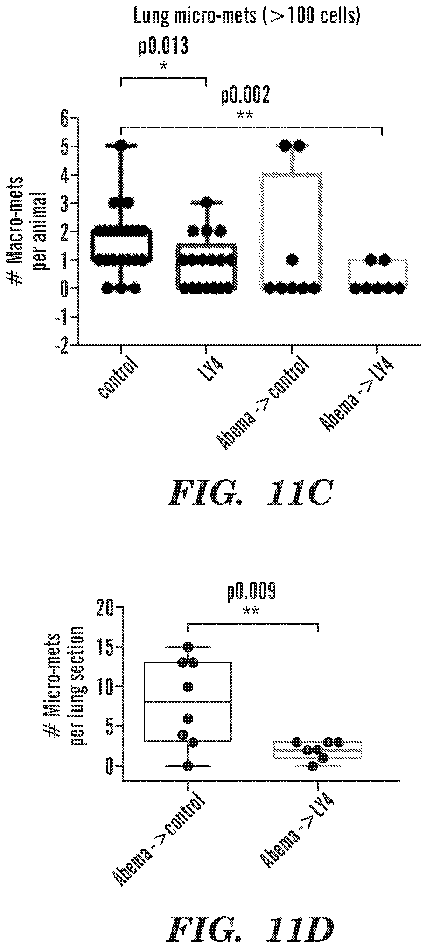

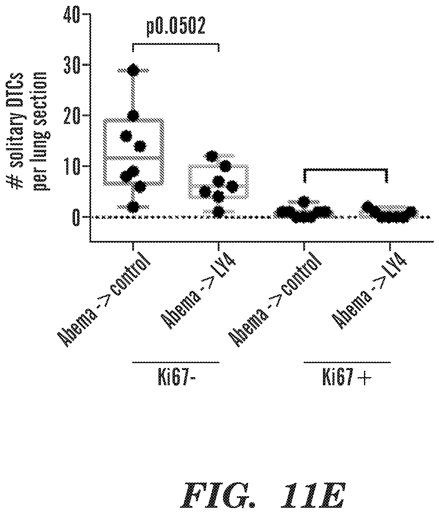

FIGs. 11A-11E show that sequential CDK4/6 inhibition followed by PERK inhibition enhances the anti-metastatic effect of LY4. FIG. 11A is a schematic illustration of an *in vivo* experiment designed to evaluate the sequential treatment of Abemaciclib and LY4 in an MMTV-neu/HER2⁺ mouse model. MMTV-neu/HER2⁺ female mice (24-weeks old) were treated daily with the CDK4/6 inhibitor Abemaciclib (50 mpk) for 4 weeks, followed by +/- LY4 (50 mpk). FIG. 11B is a series of fluorescence IHC of tumor sections for HER2, Ki67 (proliferation), and GADD34 (ER stress). Scale bars, 100 µm. Arrows indicate high fluorescence. FIG. 11C is a graph showing the number of macro-metastasis (>100 cells) detected by H&E staining and quantified in 5 lung sections/animal (n=8). p by Mann-Whitney test. FIG. 11D is a graph showing the number of micro-metastasis (2-100 cells) detected by IHC staining using an anti-HER2 antibody and quantified per lung section/animal ± s.d. (n=8). p by Matt-Whitney test. FIG. 11E is a graph showing the number of solitary disseminated tumor cells detected by IHC staining for HER2, classified as Ki67⁺ or Ki67⁻ and quantified per lung section ± s.d. (n=8). p by Matt-Whitney test.

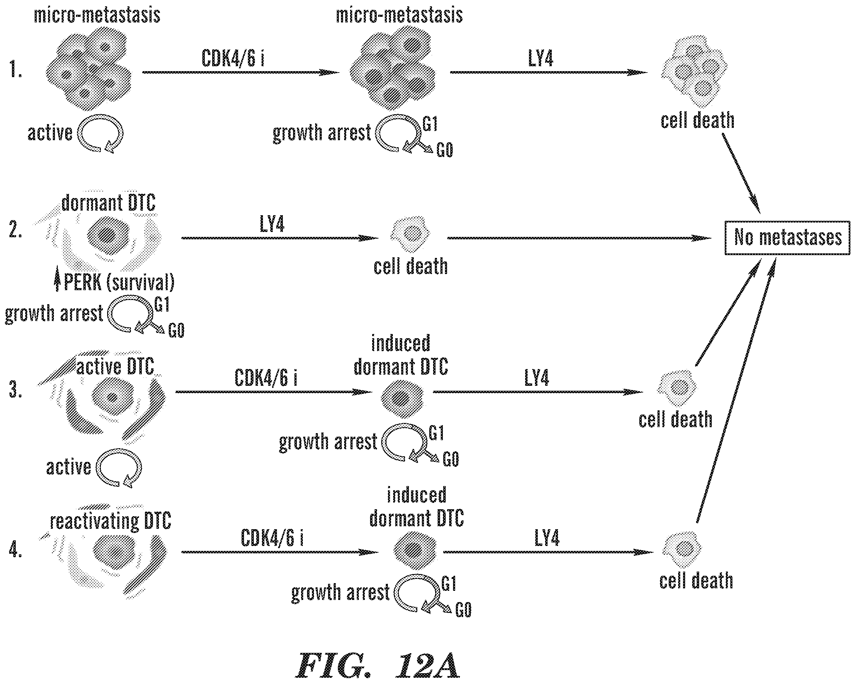

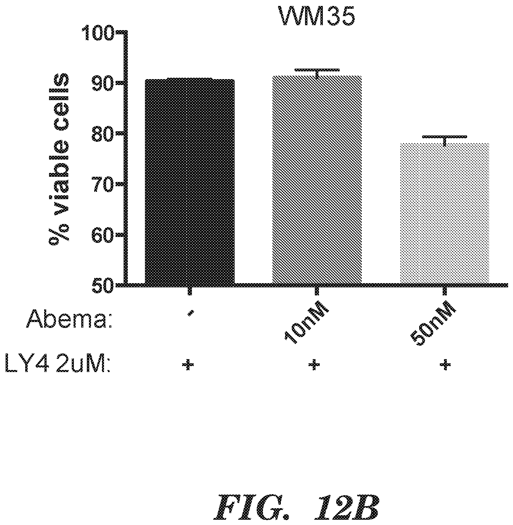

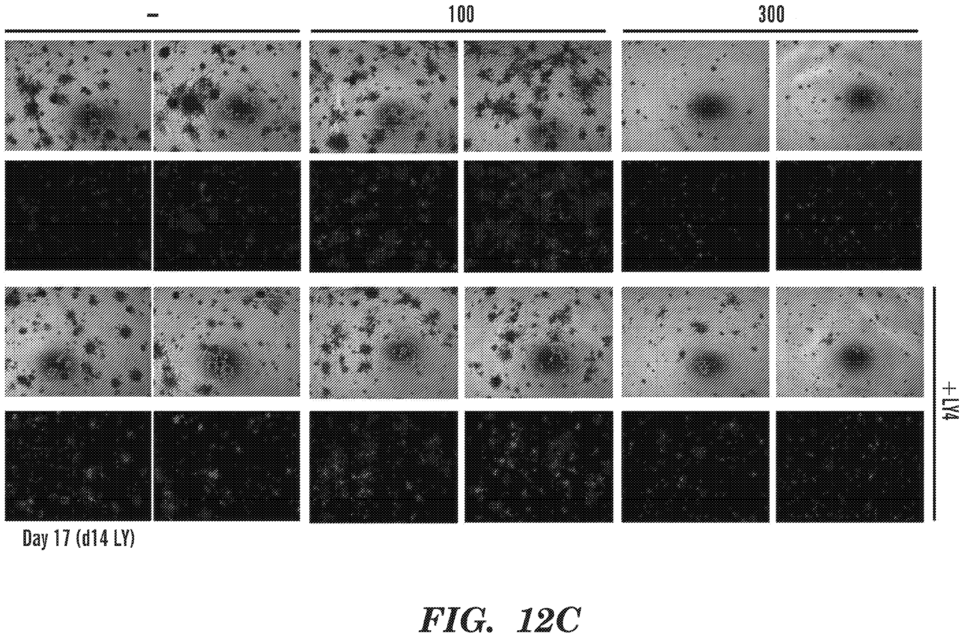

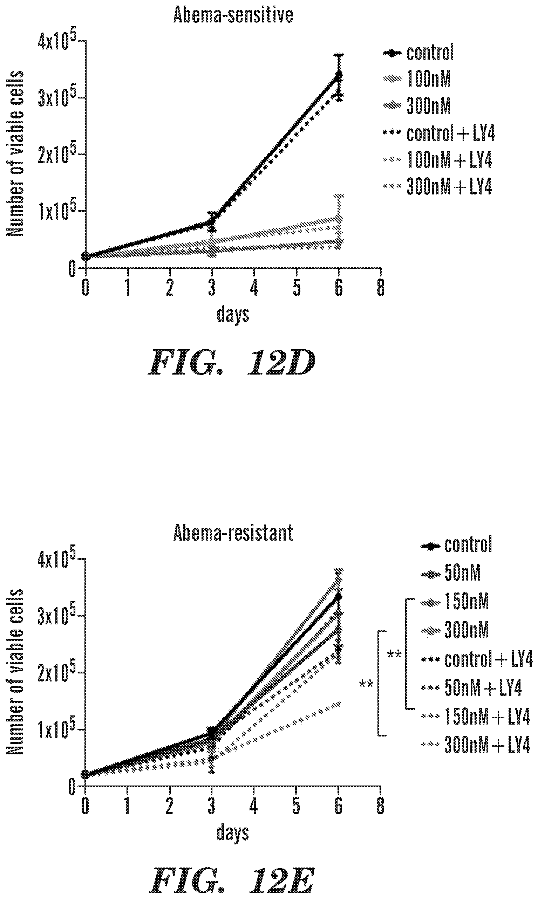

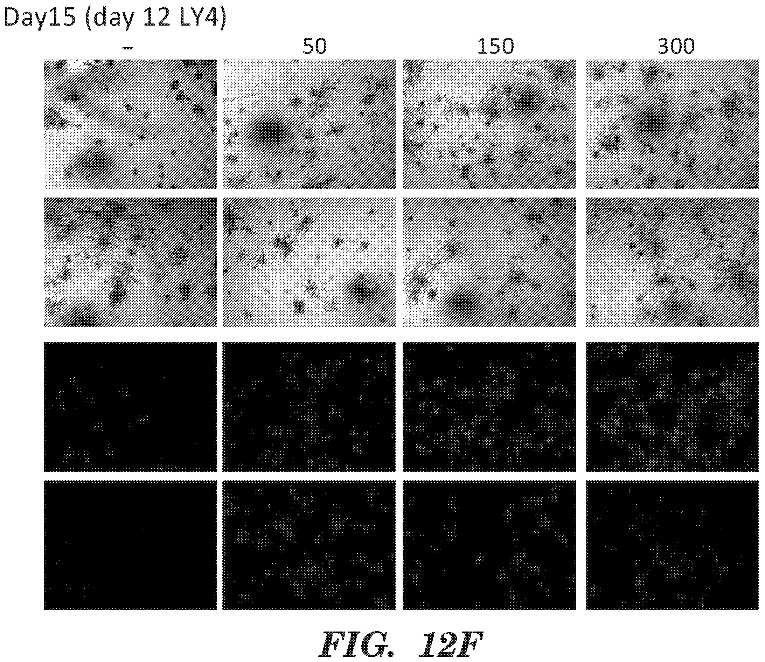

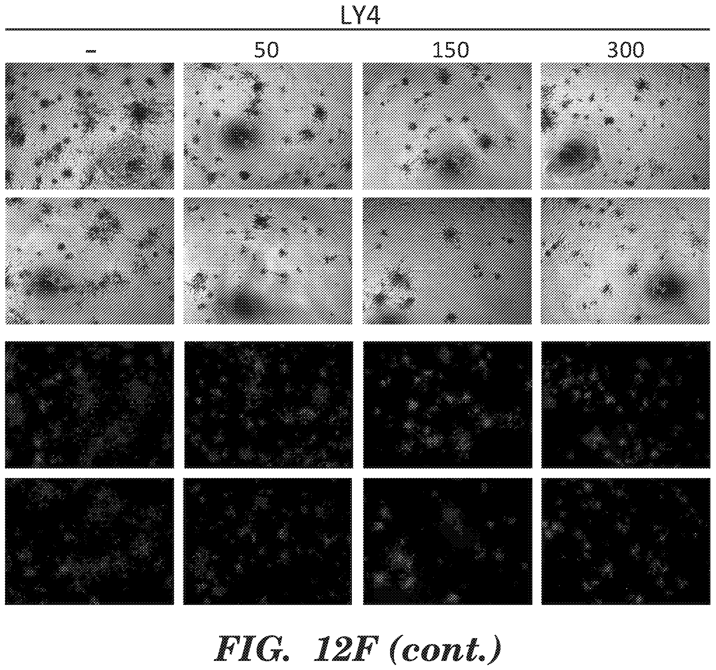

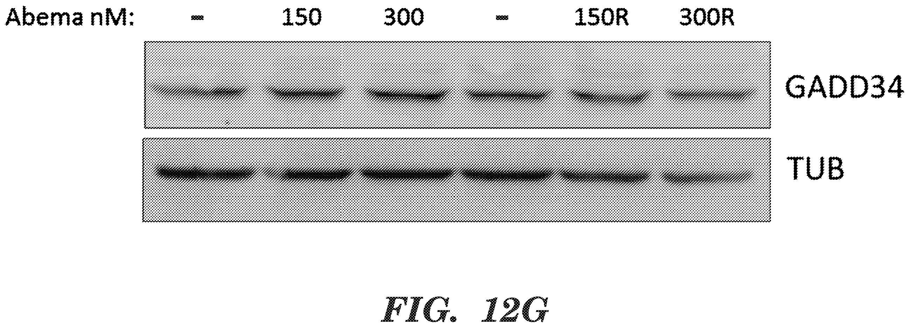

FIGs. 12A-12G show proposed mono or combination therapies that include the use of LY4 and experiments showing that the treatment of melanoma cells with the CDK4/6 inhibitor Abemaciclib in combination with LY4 differentially affects *in vitro* cell viability in 2D and 3D culture. FIG. 12A is a schematic illustration of the rationale for the combination of Abemaciclib and LY4. FIG. 12B is a bar graph showing the results of an *in vitro* treatment of Braf-mutant melanoma cells (WM35) with 0 nM, 10 nM, or 50 nM Abemaciclib for 1 week followed by 48 hour treatment with 2 µM LY4. FIG. 12C includes images of cells stained with DAPI following pre-treatment with Abemaciclib for 1 week, followed by treatment with 2 µM LY4. 5,000 cells were seeded on matrigel. In FIGs. 12D-12E, WM35 melanoma cells were pre-treated with Abemaciclib for 5 weeks, followed by treatment with LY4 in complete media and Abemaciclib. Cells were stained with Trypan blue to identify viable cells. FIG. 12D is a graph showing Abemaciclib sensitive cells. FIG. 12E is a graph showing Abemaciclib-resistant cells. FIG. 12F show images of cells stained with DAPI following pre-treatment with Abemaciclib for 5 weeks and co-treatment with 2 µM LY4 and Abemaciclib. 1,000 cells were seeded on matrigel. FIG. 12G suggests that when growth arrest is induced with Abemaciclib, the cells upregulate a PERK target (GADD34), which may explain why cells are sensitive to LY4. WM35 melanoma cells naive or resistant (R) to Abemaciclib were treated in culture with vehicle (-) or 150 and 300 nM Abemaciclib for 24 hours. Cells were then lysed and probed via western blot for GADD34 expression. Tubulin expression was used as a loading control. Note that in the Abemaciclib naive cells GADD34 is upregulated, suggesting PERK activation. Resistant cells appear to show higher levels of GADD34 that do not change or decrease after additional Abemaciclib treatment.

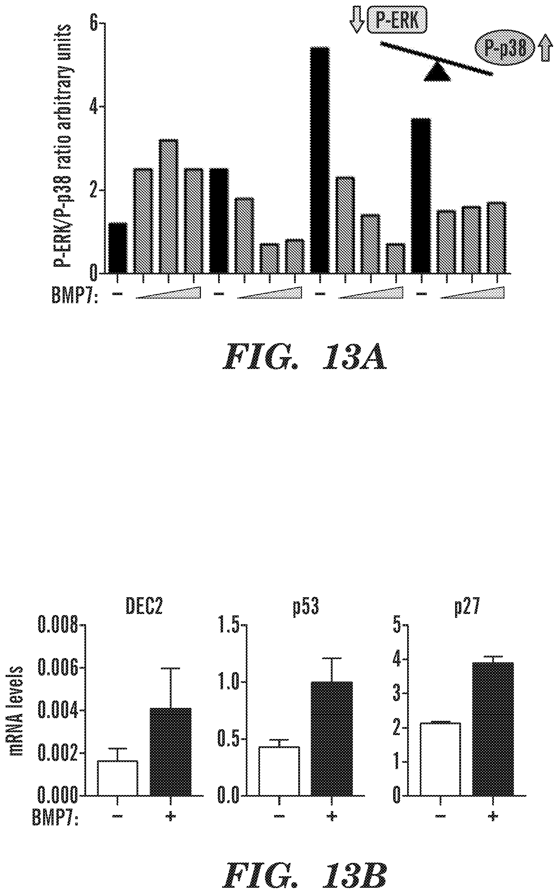

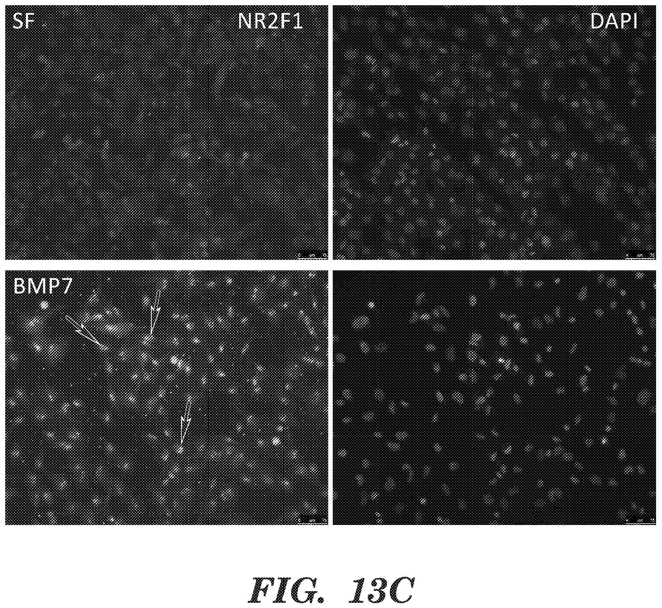

FIGs. 13A-13C demonstrate the effect of BMP7-F9 on the ERK/p38 activity ratio and various mRNAs associated with dormancy signature genes. FIG. 13A shows that BMP7-F9 treatment at 2 ng/ml, 5 ng/ml, and 10 ng/ml (second, third, and fourth gray bars, respectively: control is first black bar) reduces the ERK/p38 activity ratio over control, as determined by Western blot in HEp3 HNSCC cells. The effect on the ERK/p38 activity ratio is observed after 2-6 and 24 hours (second through fourth group of columns). In the first 30 minutes, ERK activity is stimulated by BMP7 (first column set). FIG. 13B shows that BMP7-F9 treatment induces DEC2, p53, and p27 mRNAs (10ng/ml BMP7-F9, 24 hours), which encode dormancy signature genes. FIG. 13C shows that BMP7-F9 treatment of the same cells induces nuclear accumulation of NR2F1, a potent dormancy inducing transcription factor, as determined by immunofluorescence (10 ng/ml, 24 hours). Arrows indicate NR2F1 flourescence. Differences in FIG. 13A and FIG. 13B, p<0.05 as calculated using Student's t test. These data support the hypothesis that BMP7-F9 is a strong inducer of dormancy genes found upregulated in spontaneously dormant DCCs or those induced via reprogramming or TGFβ2 signaling in the bone marrow.

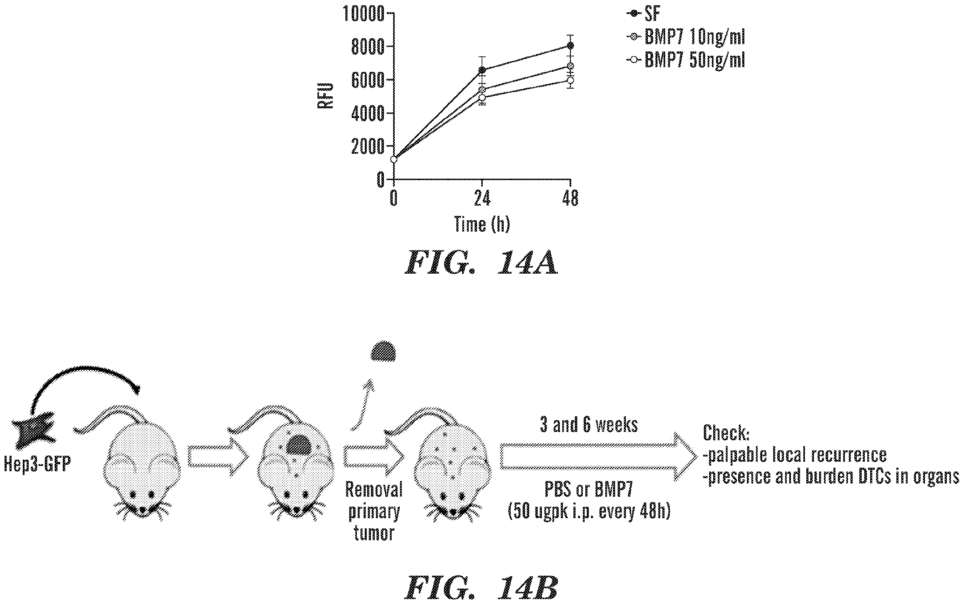

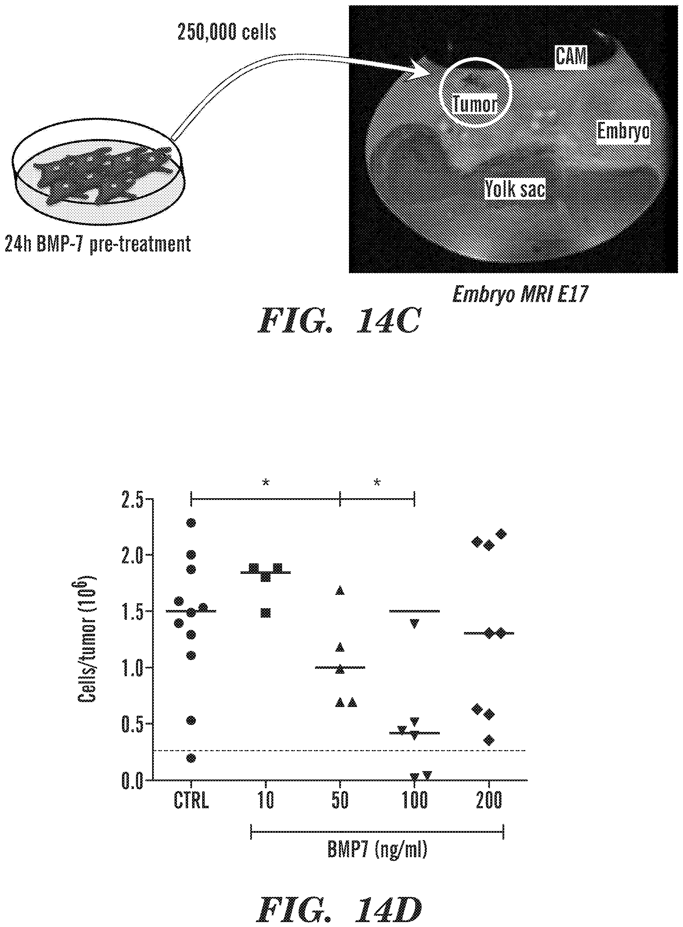

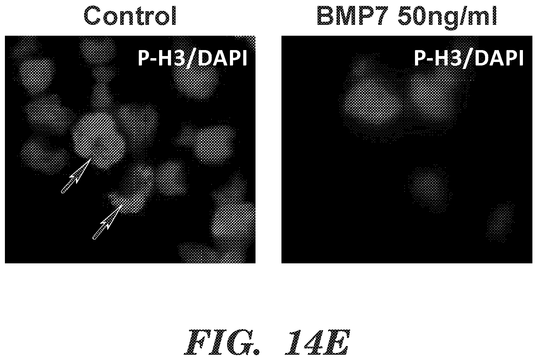

FIGs. 14A-14E show how *in vitro* and *in vivo* BMP7-F9 induces growth arrest of T-HEp3 cells. FIG. 14A shows that BMP7-F9 treatment of T-HEp3 cells inhibits their proliferation *in vitro* for 48 hours, as determined by cell titer blue assay (RFU, relative fluorescence units). FIG. 14B is a schematic illustration of the *in vivo* experimental procedure used in FIGs. 14C-14D. T-HEp3 cells were pre-treated for 24 hours with BMP7-F9 *in vitro* and then inoculated on chicken embryo chorioallantoic membranes ("CAM"s) (FIG. 14C), where they were treated daily *in vivo* with vehicle or BMP7-F9 (50 ng/ml) prior to collection of the tumors and quantification of number of HEp3 HNSCC cells (FIG. 14D) and levels of P-H3 (FIG. 14E). Arrows in FIG. 14E indicate overlapping P-H3 and DAPI fluorescence. These data support the hypothesis that the dormancy markers identified in FIG. 13B correlate with growth suppression *in vitro* and *in vivo* in a short-term experiment in the CAM system.

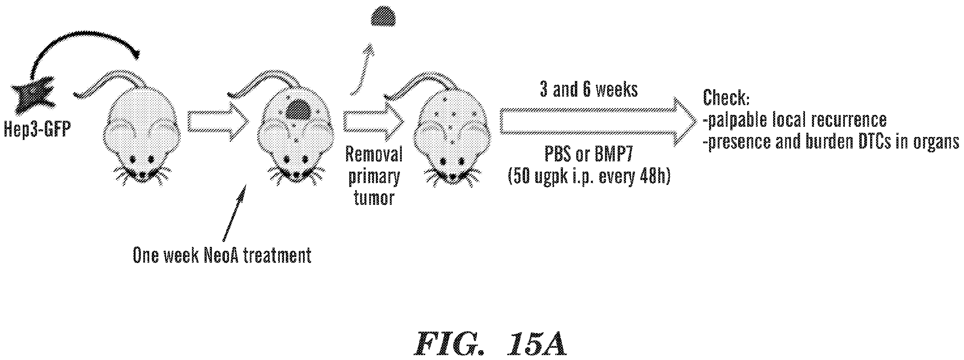

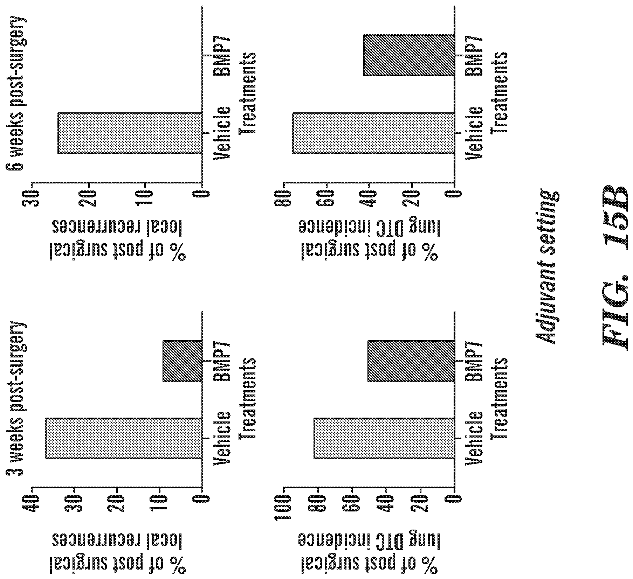

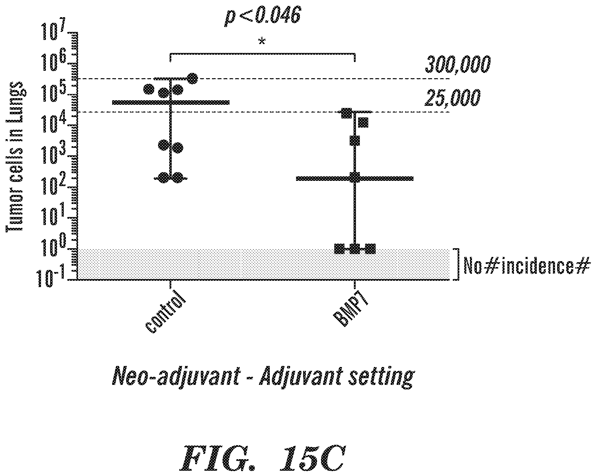

FIGs. 15A-15C show the evaluation of BMP7-F9 treatment in a mouse model of disease. FIG. 15A is a schematic illustration of an *in vivo* experimental procedure used to evaluate the effect of BMP7-F9 on metastasis initiation. HEp3-GFP HNSCC tumors were grown until they were approximately 300 mm³ and then treated in the neo-adjuvant setting with 50 µg/kg BMP7-F9 until tumors were approximately 600 mm³. Tumors were then removed via surgery. 1-2 days after surgery, the adjuvant treatment with BMP7-F9 was continued for another 3, 4, or 6 weeks. Animals were then euthanized and the DCC burden in lung was scored using fluorescence microscopy. FIG. 15B shows that BMP7 limits development of local and distant recurrences post-tumor surgery. NSG mice were treated following the protocol in FIG. 15A for 3 and 6 weeks. At those time points, the percentage of local recurrence and DCC incidence was scored. In FIG. 15C, mice were treated as in FIG. 15A, except that the adjuvant treatment was for 4 weeks. The number of GFP positive cells in dissociated lungs was scored following treatment. This is a measure of DCC burden in lungs which is significantly decreased by BMP7-F9 treatment. Note that the median of DCC burden drops one log and that BMP-7 apparently cures from DCCs 3 of 7 animals.

### DETAILED DESCRIPTION

Disclosed herein are methods of treating minimal residual cancer in a subject. One aspect of the disclosure relates to a method of treating minimal residual cancer in a subject. This method involves contacting disseminated cancer cells (DCCs) in a subject with a bone morphogenic protein 7 (BMP7) derivative protein. Contacting disseminated cancer cells (DCCs) in a subject with a bone morphogenic protein 7 (BMP7) derivative protein induces or maintains dormancy in the contacted DCCs of the subject to treat minimal residual cancer in the subject.

As used herein, the phrase "minimal residual cancer" includes a situation or condition where, by standard radiographic and histologic criteria, there lacks evidence of cancer in a subject, but where the subject in fact has residual cancer cells (*i.e*., DCCs) in the blood (as CTCs) or bone marrow or lymph nodes (as DTCs). Minimal residual cancer may occur after cancer treatment by chemotherapy, surgery, and/or radiation therapy. Standard radiographic and histologic detection methods may include, for example, imaging tests (X-rays, ultrasound, MRI); blood or immunochemical tests for known tumor markers or circulating tumor markers such as PSA; testing biopsies or cytology specimens for known tumor markers to assess, for example, the number of tumor cells present or the relative rarity of such cells.

It is well known in the art that tumor cells may disseminate early from primary tumors as CTCs and DTCs. Indeed, DTCs have been identified in subjects with no evidence of disease post tumor surgery. In rare cases where history of cancer has not ruled out transplant donation, recipients have developed donor-derived metastasis even if a donor was disease-free for up to 30 years (MacKie et al., "Fatal Melanoma Transferred in a Donated Kidney 16 Years after Melanoma Surgery," N. Engl. J. Med. 348:567-568 (2003)).

Tumor phylogenetics and whole genome sequencing of metastasis within individual patients has suggested primary tumor-to-metastasis and metastasis-to-metastasis transmission, which provides evidence that a continual/linear growth model does not account for late (10+ year) relapses in individual patients (Gundem et al., "The Evolutionary History of Lethal Metastatic Prostate Cancer," Nature 520:353-357 (2015) and Naxerova et al., "Using Tumour Phylogenetics to Identify the Roots of Metastasis in Humans," Nature Reviews Clinical Oncology 12:258-272 (2015)).

Single cell CTC analysis has also shown a genetic lineage link between CTCs and primary tumors (Ni et al., "Reproducible Copy Number Variation Patterns Among Single Circulating Tumor Cells of Lung Cancer Patients," PNAS 110(52):21083-88; Heitzer et al., "Complex Tumor Genomes Inferred from Single Circulating Tumor Cells by Array-CGH and Next-Generation Sequencing," Cancer. Res. 73:2965-75 (2013); and Lohr et al., "Whole-Exome Sequencing of Circulating Tumor Cells Provides a Window into Metastatic Prostate Cancer," Nature Biotech. 32:479-484 (2014)).

Further, in humans, CTCs/DTCs do not correlate with stage or size of primary cancer (Krishnamurthy et al., "Detection of Minimal Residual Disease in Blood and Bone Marrow in Early Stage Breast Cancer," Cancer 116(14):3330-3337 (2010)). Instead, CTCs and DTCs are thought to retain the capability to form metastasis/recurrent disease. In particular, the detection of CTCs and DTCs has been shown to be predictive of metastases and relapse in breast and prostate cancers (Braun et al., "A Pooled Analysis of Bone Marrow Micrometastasis in Breast Cancer," NEJM 353:793-802 (2005); Hayes et al., "Circulating Tumor Cells at Each Follow-up Time Point During Therapy of Metastatic Breast Cancer Patients Predict Progression-Free and Overall Survival," Clin. Cancer Res. 12(14):4218-4224 (2006); and de Bono et al., "Circulating Tumor Cells Predict Survival Benefit from Treatment in Metastatic Castration-Resistant Prostate Cancer," Clin. Cancer Res. 14:6302-6309 (2008)).

Metastases are thought to arise from proliferative but also dormant DCCs that have undergone reactivation. Given that patients can develop metastasis years after tumor resection, it is thought that dormant DCCs may account for the major entity responsible for late relapse in cancers. As used herein, the term "dormancy" refers to a temporary mitotic and growth arrest, defined as cellular dormancy, where intrinsic and/or extrinsic mechanisms drive solitary or small groups of DCCs to enter quiescence (a reversible growth arrest). A second category of dormant lesions is defined by angiogenic dormancy, where the tumor mass is kept constant by a balance between dividing cells and cells that die due to poor vascularization. A third category is immune-mediated dormancy, where the immune system keeps a proliferating tumor mass constant via a persistent cytotoxic activity that persistently trims the population of growing cancer cells (*see, e.g*., Sosa et al., "Mechanisms of Disseminated Cancer Cell Dormancy: An Awakening Field," Nat. Rev. Cancer 14(9):611-622 (2014)). Dormant cells may arise from established primary tumors, secondary tumors, and/or pre-invasive lesions.

In one embodiment of the methods disclosed herein, contacted DCCs are dormant cancer cells, meaning the cancer cells are experiencing temporary mitotic/growth arrest or a senescent-like behavior.

DCCs can be detected in bone marrow aspirates by performing a negative selection eliminating hematopoietic lineage cells and then positively staining for EpCAM or CK8/18. In combination, the cells can be stained for dormancy markers to determine whether these are in a proliferative or dormant state. The latter can be done post-fixation. To perform whole genome or whole transcriptome analysis, EpCAM positive DCCs from bone marrow are isolated live and processed for the whole genome or transcriptome analysis (Gužvić et al., "Combined Genome and Transcriptome Analysis of Single Disseminated Cancer Cells from Bone Marrow of Prostate Cancer Patients Reveals Unexpected Transcriptomes," *Cancer Res.* 74(24):7383-94 (2014)).

Methods described herein may further involve detecting the presence of DCCs in the subject prior to said contacting. As shown in Table 1 below, dormant DCCs can be identified, because they are phenotypically distinguishable from other cell types (Sosa et al., "Mechanisms of Disseminated Cancer Cell Dormancy: An Awakening Field," Nat. Rev. Cancer 14(9):611-622 (2014)).

**Table 1. DCC Markers**

| **Cancer** | **Dormant DCC Phenotype** | **Active DCC Phenotype** |
|---|---|---|
| Breast | TGFβR3 ↑ | P-FAKf |
| | BMPR2 ↑ | EDG2 ↑ |
| | P-ERK ↓ | P-Src ↑ |
| | P-p38 ↑ | P-ERK ↑ |
| | GRP78 ↑ | P-p38 ↓ |
| | POSTN ↓ | NR2F1 ↓ |
| | TSP receptors ↑ | |
| | NR2F1 ↑ | |
| Head and Neck Squamous Cell Carcinoma (HNSCC) | TGFβR3 ↑ | P-FAK ↑ |
| | BMPR2 ↑ | P-Src ↑ |
| | P-ERK ↓ | P-ERK ↑ |
| | P-p38 ↑ | P-p38 ↓ |
| | GRP78 T | NR2F1 ↓ |
| | NR2F1 ↑ | |
| Prostate | TGFβR3 ↑ | P-FAK ↑ |
| | BMPR2 ↑ | P-Src ↑ |
| | P-ERK ↓ | P-ERK ↑ |
| | P-p38 ↑ | P-p38 ↓ |
| | GRP78 ↑ | NR2F1 ↓ |
| | NR2F1 ↑ | |
| Glioblastoma, osteosarcoma and liposarcoma | POSTN ↓ | Not Determined |
| | TSP receptors ↑ | |
| Ovarian | ARHI ↑ | Not Determined |
| | ATG genes ↑ | |
| Pancreatic | IFNR ↑ | Not Determined |
| | TNFR ↑ | |

DTCs have been identified in the bone marrow of 13-72% of prostate cancer patients prior to surgery and 20-57% of patients with no evidence of disease greater than 5 years after surgery (Morgan et al., "Disseminated Tumor Cells in Prostate Cancer Patients after Radical Prostatectomy and without Evidence of Disease Predicts Biochemical Recurrence," Clin. Cancer Res. 15:677-683 (2009) and Weckermann et al., "Perioperative Activation of Disseminated Tumor Cells in Bone Marrow of Patients with Prostate Cancer," J. Clin. Oncol. 27(10):1549-56 (2009)). The detection of DTCs is prognostic of relapse in patients with clinical dormancy.

As used herein, the phrase "clinical dormancy" refers to the prolonged clinical disease-free time (e.g., greater than 5 years) between removal of a primary tumor and disease recurrence. Clinical dormancy is common in prostate cancer, breast cancer, esophageal cancer, renal cancer, thyroid cancer, B-cell lymphoma, and melanoma (Lam et al., "The Role of the Microenvironment - Dormant Prostate Disseminated Tumor Cells in the Bone Marrow," Drug Discov. Today Technol. 11:41-47 (2014); Gelao et al., "Tumour Dormancy and Clinical Implications in Breast Cancer," Ecancermedicalscience 7:320 (2013); Ellis et al., "Detection and Isolation of Prostate Cancer Cells from Peripheral Blood and Bone Marrow," Urology 61:277-281 (2003); Morgan et al., "Disseminated Tumor Cells in Prostate Cancer Patients after Radical Prostatectomy and without Evidence of Disease Predicts Biochemical Recurrence," Clin. Cancer Res. 15:677-683 (2009); and Pfitzenmaier et al., "Telomerase Activity in Disseminated Prostate Cancer Cells," BJU Int. 97:1309-1313 (2006)), and to reside in distant organs including bone, lymph nodes, liver, and lung where they can remain dormant for a prolonged period of time (*e.g*., greater than 10 years) until, in some patients, clinical metastases may develop.

In some embodiments of carrying out methods described herein, the subject has been diagnosed with CTCs.

In some embodiments of carrying out methods described herein, the subject has been diagnosed with DTCs and/or a non-metastatic cancer.

As used herein, a "subject" is, *e.g*., a patient, such as a cancer patient, and encompasses any animal, but preferably a mammal. In one embodiment, the subject is a human subject. Suitable human subjects include, without limitation, children, adults, and elderly subjects who have been diagnosed with disseminated cancer cells and/or a non-metastatic cancer.

In other embodiments, the subject may be bovine, ovine, porcine, feline, equine, murine, canine, lapine, etc.

In carrying out methods described herein, DCCs in a subject are contacted to induce or maintain dormancy of DCCs. This means the establishment of a sustained non-proliferative state in a DCC or the continuation of a non-proliferative state in a DCC.

In one embodiment, minimal residual cancer is treated in a subject that has been diagnosed with cancer. For example, and without limitation, the subject has been diagnosed with one or more of breast cancer, multiple myeloma, lung cancer, non-small cell lung cancer, brain cancer, cervical cancer, mantel cell lymphoma, leukemia, hepatocellular carcinoma, prostate cancer, ureal and cutaneous melanoma, skin cancers, head and neck cancers, thyroid cancer, glioblastoma, neuroblastoma, and colorectal cancer.

Other cancers may also be amenable to treatment with the methods described herein.

In one embodiment, minimal residual cancer related to or associated with breast cancer is treated in a subject. The breast cancer may be selected from one or more of invasive breast cancer, ductal carcinoma *in situ* (DCIS), lobular carcinoma *in situ* (LCIS), and inflammatory breast cancer.

A variety of molecular factors may be used to molecularly categorize breast cancers, including hormone receptors and Human Epidermal Growth Factor Receptor 2 (HER2) status. The HER2 and basal-like groups are the major molecular subtypes identified among hormone receptor-negative breast cancers (Schnitt, "Classification and Prognosis of Invasive Breast Cancer: From Morphology to Molecular Taxonomy," Modern Pathology 23: S60-S64 (2010)). In one embodiment, the breast cancer is HER2⁺ breast cancer.

In some embodiments of the methods described herein, the subject has undergone surgical resection to remove a tumor. For example, the subject may have undergone one or more of a mastectomy, prostatectomy, skin lesion removal, small bowel resection, gastrectomy, thoracotomy, adrenalectomy, appendectomy, colectomy, oophorectomy, thyroidectomy, hysterectomy, glossectomy, colon polypectomy, and colorectal resection.

Otherwise disclosed herein are methods in which disseminated cancer cells (DCCs) in a subject are contacted with a bone morphogenic protein 7 (BMP7) derivative protein. BMP7 is a member of the TGFβ superfamily, which is secreted from bone marrow stromal osteoblasts and may influence the DCC/DTC microenvironment. BMP7 plays a key role in the transformation of mesenchymal cells into bone and cartilage and has been shown to reversibly induce senescence in prostate cancer stem-like cells (Kobayashi et al., "Bone Morphogenetic Protein 7 in Dormancy and Metastasis of Prostate Cancer Stem-Like Cells in Bone," J. Exp. Med. 208(13):2641-55 (2011)). Pro-BMP7 is an intermediary between pre-BMP7 and mature BMP7 generated via proteolytic processing of pre-protein, which generates subunits of the mature homodimer.

Human BMP7 protein is a secreted signaling molecule of the TGF-beta superfamily and was originally identified for its ability to induce bone formation but later became recognized as a multifunctional cytokine which mediates growth and differentiation of many different cell types. Human BMP7 protein is expressed in cells as a 292 amino acid precursor protein and the mature, biologically active BMP7 is generated by proteolytic removal of the signal peptide and pro-peptide. The wild type human BMP7 protein amino acid sequence containing the signal peptide (the first 29 amino acids), pro-domain, and mature peptide (**in bold**) is indicated as SEQ ID NO:1, as follows: 
MHVRSLRAAA PHSFVALWAP LFLLRSALAD FSLDNEVHSS FIHRRLRSQE RREMQREILS
ILGLPHRPRP HLQGKHNSAP MFMLDLYNAM AVEEGGGPGG QGFSYPYKAV FSTQGPPLAS
LQDSHFLTDA DMVMSFVNLV EHDKEFFHPR YHHREFRFDL SKIPEGEAVT AAEFRIYKDY
IRERFDNETF RISVYQVLQE HLGRESDLFL LDSRTLWASE EGWLVFDITA TSNHWVVNPR
HNLGLQLSVE TLDGQSINPK LAGLIGRHGP QNKQPFMVAF FKATEVHFRS IRSTGSKQRS
QNRSKTPKNQ EALRMANVAE NSSSDQRQAC KKHELYVSFR DLGWQDWIIA PEGYAAYYCE
GECAFPLNSY MNATNHAIVQ TLVHFINPET VPKPCCAPTQ LNAISVLYFD DSSNVILKKY
RNMVVRACGC H
 It would be understood by a person of ordinary skill in the art that the signal peptide may be removed by proteolytic cleavage resulting in an intact pro-domain/mature peptide that is designated as pro-BMP7.

Wild type human mature BMP7 is a dimer of two glycosylated, 139 amino acid disulfide-linked, homodimeric proteins of about 35 kDa. Each homodimeric protein has the amino acid sequence as shown in SEQ ID NO:2:

STGSKQRSQN RSKTPKNQEA LRMANVAENS SSDQRQACKK HELYVSFRDL GWQDWIIAPE
GYAAYYCEGE CAFPLNSYMN ATNHAIVQTL VHFINPETVP KPCCAPTQLN AISVLYFDDS
SNVILKKYRN MVVRACGCH

Variants of human BMP7 protein include variants of human mature BMP7 of SEQ ID NO:2, with specific amino acid changes indicated in the consensus sequence as shown in SEQ ID NO:3: 
STGSKQRSQN RSKTPKNQEA LRMANVAENS SSXQRQXCKK HELYVSFRDL GWQDWIIAPX
GYAAXYCEGE CAFPLNSYMN ATNHAXXQXL XHXXNPETVP KPCCAPTQLX AISXLYFDDX
SNVILKKXRN MXVXACGCH
 Particular variants of human mature BMP7 protein of the present disclosure have increased specific activity, improved solubility characteristics, improved bioavailability, decreased binding to endogenous circulating inhibitors, and/or reduced EBF activity compared to the wild type mature human BMP7 protein.

Suitable variants of human BMP7 protein are selected from the group consisting of F93V/N110G; Y65G/I86L/T89A/N110G; Y65G/I86L/N110G/Y128F; Y65G/I86L/N110G/Y128W; Y65G/I86L/F93V/N110G/Y128W (BMP7-F9); Y65G/T89A/N110G/Y128F; Y65G/I86L/N110G; and Y65G/V114M (*see* Table 2 below).

**Table 2. Exemplary Variants of Human BMP7**

| **BMP7 Variant** | **Amino Acid Sequence** | **SEQ ID NO:** |
|---|---|---|
| F93V/N110G | STGSKQRSQN RSKTPKNQEA LRMANVAENS SSDQRQACKK HELYVSFRDL GWQDWIIAPE GYAAYYCEGE CAFPLNSYMN ATNHAIVQTL VHVINPETVP KPCCAPTQLG AISVLYFDDS SNVILKKYRN MVVRACGCH | 4 |
| Y65G/I86L/ T89A/N110G | STGSKQRSQN RSKTPKNQEA LRMANVAENS SSDQRQACKK HELYVSFRDL GWQDWIIAPE GYAAGYCEGE CAFPLNSYMN ATNHALVQAL VHFINPETVP KPCCAPTQLG AISVLYFDDS SNVILKKYRN MVVRACGCH | 5 |
| Y65G/I86L/ N110G/Y128F | STGSKQRSQN RSKTPKNQEA LRMANVAENS SSDQRQACKK HELYVSFRDL GWQDWIIAPE GYAAGYCEGE CAFPLNSYMN ATNHALVQTL VHFINPETVP KPCCAPTQLG AISVLYFDDS SNVILKKFRN MVVRACGCH | 6 |
| Y65G/I86L/ N110G/Y128W | STGSKQRSQN RSKTPKNQEA LRMANVAENS SSDQRQACKK HELYVSFRDL GWQDWIIAPE GYAAGYCEGE CAFPLNSYMN ATNHALVQTL VHFINPETVP KPCCAPTQLG AISVLYFDDS SNVILKKWRN MVVRACGCH | 7 |
| Y65G/I86L/ F93V/N110G/ | STGSKQRSQN RSKTPKNQEA LRMANVAENS SSDQRQACKK HELYVSFRDL GWQDWIIAPE GYAAGYCEGE CAFPLNSYMN | 8 |
| Y128W (BMP7-F9) | ATNHALVQTL VHVINPETVP KPCCAPTQLG AISVLYFDDS SNVILKKWRN MVVRACGCH | |
| Y65G/T89A/ N110G/Y128F | STGSKQRSQN RSKTPKNQEA LRMANVAENS SSDQRQACKK HELYVSFRDL GWQDWIIAPE GYAAGYCEGE CAFPLNSYMN ATNHAIVQAL VHFINPETVP KPCCAPTQLG AISVLYFDDS SNVILKKFRN MVVRACGCH | 9 |
| Y65G/I86L/ N110G | STGSKQRSQN RSKTPKNQEA LRMANVAENS SSDQRQACKK HELYVSFRDL GWQDWIIAPE GYAAGYCEGE CAFPLNSYMN ATNHALVQTL VHFINPETVP KPCCAPTQLG AISVLYFDDS SNVILKKYRN MVVRACGCH | 10 |
| Y65 G/V 114M | STGSKQRSQN RSKTPKNQEA LRMANVAENS SSDQRQACKK HELYVSFRDL GWQDWIIAPE GYAAGYCEGE CAFPLNSYMN ATNHAIVQTL VHFINPETVP KPCCAPTQLN AISMLYFDDS SNVILKKYRN MVVRACGCH | 11 |

In one instance, variants of BMP7 are selected from the group consisting of Y65G/I86L/N110G/Y128W and Y65G/I86L/F93V/N110G/Y128W.

In one instance, the BMP7 derivative is an engineered BMP7 variant of pro-BMP7. The engineered variant of pro-BMP7 may comprise amino acid substitutions in amino acid positions corresponding to the BMP7 mature protein domain. The engineered variant of pro-BMP7 may be processed to a mature BMP7 derivative protein. Suitable variants of pro-BMP7 that contain the pro-domain fused to the N-terminus of the human mature BMP7 protein variant are selected from the group consisting of SEQ ID NO: 12, SEQ ID NO: 13, SEQ ID NO:14, SEQ ID NO: 15, and SEQ ID NO:16, as illustrated in Table 3.

**Table 3. Exemplary Variants of Human Pro-BMP7**

| **Pro-BMP7 Variant** | **Amino Acid Sequence** |
|---|---|
| SEQ ID NO: 12 | DFSLDNEVHS SFIHRRLRSQ ERREMQREIL SILGLPHRPR PHLQGKHNSA PMFMLDLYNA MAVEEGGGPG GQGFSYPYKA VFSTQGPPLA SLQDSHFLTD ADMVMSFVNL VEHDKEFFHP RYHHREFRFD LSKIPEGEAV TAAEFRIYKD |
| | YIRERFDNET FRISVYQVLQ EHLGRESDLF LLDSRTLWAS EEGWLVFDIT ATSNHWVVNP RHNLGLQLSV ETLDGQSINP KLAGLIGRHG PQNKQPFMVA FFKATEVHFR SIRSTGSKQR SQNRSKTPKN QEALRMANVA ENSSSDQRQA CKKHELYVSF RDLGWQDWII APEGYAAYYC EGECAFPLNS YMNATNHAIV QTLVHVINPE TVPKPCCAPT QLGAISVLYF DDSSNVILKK YRNMVVRACG CH |
| SEQ ID NO: 13 | DFSLDNEVHS SFIHRRLRSQ ERREMQREIL SILGLPHRPR PHLQGKHNSA PMFMLDLYNA MAVEEGGGPG GQGFSYPYKA VFSTQGPPLA SLQDSHFLTD ADMVMSFVNL VEHDKEFFHP RYHHREFRFD LSKIPEGEAV TAAEFRIYKD YIRERFDNET FRISVYQVLQ EHLGRESDLF LLDSRTLWAS EEGWLVFDIT ATSNHWVVNP RHNLGLQLSV ETLDGQSINP KLAGLIGRHG PQNKQPFMVA FFKATEVHFR SIRSTGSKQR SQNRSKTPKN QEALRMANVA ENSSSDQRQA CKKHELYVSF RDLGWQDWII APEGYAAGYC EGECAFPLNS YMNATNHALV QALVHFINPE TVPKPCCAPT QLGAISVLYF DDSSNVILKK YRNMVVRACG CH |
| SEQ ID NO: 14 | DFSLDNEVHS SFIHRRLRSQ ERREMQREIL SILGLPHRPR PHLQGKHNSA PMFMLDLYNA MAVEEGGGPG GQGFSYPYKA VFSTQGPPLA SLQDSHFLTD ADMVMSFVNL VEHDKEFFHP RYHHREFRFD LSKIPEGEAV TAAEFRIYKD YIRERFDNET FRISVYQVLQ EHLGRESDLF LLDSRTLWAS EEGWLVFDIT ATSNHWVVNP RHNLGLQLSV ETLDGQSINP KLAGLIGRHG PQNKQPFMVA FFKATEVHFR SIRSTGSKQR SQNRSKTPKN QEALRMANVA ENSSSDQRQA CKKHELYVSF RDLGWQDWII APEGYAAGYC EGECAFPLNS YMNATNHALV QTLVHFINPE TVPKPCCAPT QLGAISVLYF DDSSNVILKK FRNMVVRACG CH |
| SEQ ID NO: 15 | DFSLDNEVHS SFIHRRLRSQ ERREMQREIL SILGLPHRPR PHLQGKHNSA PMFMLDLYNA MAVEEGGGPG GQGFSYPYKA VFSTQGPPLA SLQDSHFLTD ADMVMSFVNL VEHDKEFFHP RYHHREFRFD LSKIPEGEAV TAAEFRIYKD YIRERFDNET FRISVYQVLQ EHLGRESDLF LLDSRTLWAS EEGWLVFDIT ATSNHWVVNP RHNLGLQLSV ETLDGQSINP KLAGLIGRHG PQNKQPFMVA FFKATEVHFR SIRSTGSKQR SQNRSKTPKN QEALRMANVA ENSSSDQRQA CKKHELYVSF RDLGWQDWII APEGYAAGYC EGECAFPLNS YMNATNHALV QTLVHFINPE TVPKPCCAPT QLGAISVLYF DDSSNVILKK WRNMVVRACG CH |
| SEQ ID NO:16 | DFSLDNEVHS SFIHRRLRSQ ERREMQREIL SILGLPHRPR PHLQGKHNSA PMFMLDLYNA MAVEEGGGPG GQGFSYPYKA VFSTQGPPLA SLQDSHFLTD ADMVMSFVNL VEHDKEFFHP RYHHREFRFD LSKIPEGEAV TAAEFRIYKD YIRERFDNET FRISVYQVLQ EHLGRESDLF LLDSRTLWAS EEGWLVFDIT ATSNHWVVNP RHNLGLQLSV ETLDGQSINP KLAGLIGRHG PQNKQPFMVA FFKATEVHFR SIRSTGSKQR SQNRSKTPKN QEALRMANVA ENSSSDQRQA CKKHELYVSF RDLGWQDWII APEGYAAGYC EGECAFPLNS YMNATNHALV QTLVHVINPE TVPKPCCAPT QLGAISVLYF DDSSNVILKK WRNMVVRACG CH |

Suitable BMP7 derivative proteins for use with the methods described herein include variants of human pre-BMP7 (*i.e*., SEQ ID NO:1).

In one instance, the BMP7 derivative protein is a mature BMP7 protein having enhanced bioactivity (*e.g*., up to greater than 50 times or more bioactive) and biophysical properties (*e.g*., enhanced solubility and stability) when compared to a mature wild type BMP7 protein.

In another instance, the BMP7 derivative is BMP7-F9 (SEQ ID NO:8).

Reference herein to a wildtype BMP7 protein or variant thereof, including by reference to a SEQ ID NO., refers to a homodimer where each monomeric subunit has the identified sequence. For example, reference to BMP7-F9 (SEQ ID NO:8) refers to a homodimer where each monomeric subunit has the sequence shown in SEQ ID NO:8 and the subunits are linked via disulfide bond(s).

For the functional assays described herein, treatment with or administration of a particular pro-BMP7 protein or variant thereof, refers to treatment with or administration of homodimers of the particular mature BMP7, *i.e*., either wild type or a variant thereof, which are generally in a non-covalent complex with wild type human pro-domain.

In accordance with the methods described herein, contacting may be carried out by administering the BMP7 derivative protein to the subject.

The effect of BMP7 on a subject may depend on the BMP Receptor 2 (BMPR2), expression of which has been shown to inversely correlate with recurrence and bone metastasis in prostate cancer patients (Kobayashi et al., "Bone Morphogenetic Protein 7 in Dormancy and Metastasis of Prostate Cancer Stem-Like Cells in Bone," J. Exp. Med. 208(13):2641-55 (2011)). Thus, in one instance, the DCCs contacted in a subject are bone morphogenic protein receptor positive (BMPR⁺).

The methods described herein may further involve administering to the subject a chemotherapeutic agent, an immunotherapeutic agent, an epigenetic agent, or ionizing radiation.

As used herein, the term "chemotherapeutic agent" refers to a synthetic, biological, or semi-synthetic compound that is not an enzyme and that kills cancer cells or inhibits the growth of cancer cells while having less effect on non-cancerous cells. Any suitable chemotherapeutic agent can be used.

Suitable chemotherapeutic agents include, without limitation, an anthracycline, a taxane, a kinase inhibitor, an antibody, a fluoropyrimidine, and a platinum drug. Exemplary anthracyclines include, but are not limited to, doxorubicin, daunorubicin, epirubicin, mitoxantrone, and idarubicin. Exemplary taxanes include, but are not limited to, docetaxel and paclitaxel. Exemplary kinase inhibitors include, but are not limited to, lapatinib, imatinib mesylate, and genefitinib. Exemplary antibodies include, but are not limited to, alemtuzumab, gemtuzumab ozogamicin, rituximab, trastuzumab, and ibritumomab tiuxetan. Exemplary fluoropyrimidines include, but are not limited to, 5-fluoruracil, capecitabine, tegafur, tegafur-uracil, floxuridine, 5-fluorodeoxyuridine and S-1. Exemplary platinum drugs include, but are not limited to, cisplatin, carboplatin, oxaliplatin, and nedaplatin.

Additional suitable chemotherapeutic agents include, without limitation, alkylating agents (e.g., mechlorethamine, cyclophosphamide, ifosfamide, melphalan, chlorambucil, thiotepa, hexamethylmelamine, busulfan, carmustine, lomustine, semustine, streptozocin, decarbazine, estramustine, streptozocin, and temozolomide), vinca alkaloids (*e.g*., vinblastine, vincristine, and vinorelbine), podophyllotoxin (*e.g*., etoposide and teniposide), antibiotics (*e.g*., bleomycin, dactinomycin, mitomycin, and valrubicrin), and camptothecin analogs (*e.g*., irinotecan or topotecan).

In some embodiments, the chemotherapeutic agent is an anti-HER2 chemotherapeutic agent selected from trastuzumab (Herceptin^{®}) and lapatinib (Tykerb^{®}). Trastuzumab is a monoclonal antibody that targets the HER2/neu receptor on cancer cells. Lapatinib is a tyrosine kinase inhibitor that targets Epidermal Growth Factor Receptor (EGFR) and HER2.

As used herein, the term "immunotherapeutic agent" refers to an agent that is capable of inducing or enhancing an immune response in a subject. In the context of cancer, immunotherapeutic agents stimulate the immune system to more effectively target cancerous cells. Suitable immunotherapeutic agents may be selected from an immune checkpoint inhibitor, an interferon, and a tumor vaccine.

Immune checkpoint inhibitors are compounds that inhibit immune checkpoints engagement. Exemplary immune checkpoint modulating agents include PD-1 inhibitors (*e.g*., pembrolizumab and nivolumab), PD-L1 inhibitors (*e.g*., atezolizumab, avelumab, and durvalumab), and CTLA-4 inhibitors (*e.g*., ipilimumab).

The interferons ("IFNs") are a family of cytokines that protect against disease by direct effects on target cells and by activating immune responses. IFNs can be produced by, and act on, both tumor cells and immune cells. Type I IFNs comprise IFNα proteins, IFNβ, IFNε, IFNκ, and IFNω. Type I IFNs are known to mediate antineoplastic effects against several malignancies (Moschos et al., Interferons in the Treatment of Solid Tumors," Cancer Treat. Res. 126:207-241 (2005)).

As used herein, the term "tumor vaccine" refers to a composition that stimulates an immune response in a subject against a tumor or cancerous cell. Tumor vaccines are typically composed of a source of cancer-associated material or cells (antigen) that may be autologous (from self) or allogenic (from others) to the subject, along with other components (*e.g*., adjuvants) to further stimulate and boost the immune response against the antigen. Tumor vaccines can result in stimulating the immune system of the subject to produce antibodies to one or several specific antigens, and/or to produce killer T cells to attack cancer cells that have those antigens.

As used herein, the term "epigenetic agent" refers to an agent that alters the epigenetic state (*e.g*., methylation state) of the DNA of a cell upon or after contact with or administration of such agent.

Suitable epigenetic agents may be selected from, *e.g*., a histone deacetylase ("HDAC") inhibitor, 5-azacytidine, retinoic acid, arsenic trioxide, Enhancer Of Zeste 2 Polycomb Repressive Complex 2 Subunit ("EZH2") inhibitor, bromodomain ("BRD") inhibitor, and derivatives thereof.

Exemplary HDAC inhibitors include, but are not limited to, trichostatin A, trapoxin B, benzamides, phenylbutyrate, valproic acid, vorinostat, belinostat, LAQ824, panobinostat, entinostat, CI994, and mocetinostat.

Exemplary EZH2 inhibitors include, but are not limited to, 3-deazaneplanocin A (DZNep), EPZ005687, GSK126, EI1, UNC1999, and EPZ-6438 (Kim et al., "Targeting EZH2 in Cancer," Nat. Med. 22(2): 128-134 (2016)).

Exemplary bromodomain inhibitors include, without limitation, JQ1, I-BET151/762, PF-1, and RVX-208 (Wadhwa et al., "Bromodomain Inhibitor Review: Bromodomain and Extra-terminal Family Protein Inhibitors as a Potential New Therapy in Central Nervous System Tumors," Cureus 8(5):e620 (2016)).

Additional exemplary epigenetic agents include DNA methyl transferase (DNMT) inhibitors including, but not limited to, azacytidine and decitabine.

The DCC/DTC microenvironment plays a critical role in the enhancement of dormancy. Nuclear Receptor Subfamily 2 Group F Member 1 (NR2F1) is a nuclear hormone receptor and transcriptional regulator that is a key node in a transcription factor network that constitutes a tumor cell dormancy signature. When applied to gene expression profiles of estrogen receptor-positive (ER⁺) breast cancer patients, this signature has been shown to predict longer metastasis-free periods (Kim et al., "Dormancy Signatures and Metastasis in Estrogen Receptor Positive and Negative Breast Cancer," PloS One 7:e35569 (2012)). This dormancy signature has also been found in dormant DTCs in prostate cancer patients who had been asymptomatic for 7-18 years (Sosa et al., "NR2F1 Controls Tumour Cell Dormancy via SOX9- and RARbeta-Driven Quiescence Programmes," Nat. Commun. 6:6170 (2015) and Chery et al., "Characterization of Single Disseminated Prostate Cancer Cells Reveals Tumor Cell Heterogeneity and Identifies Dormancy Associated Pathways," Oncotarget 5:9939-51 (2014)), highlighting its relevance to human disease.

NR2F1 has been shown to upregulate and induce dormancy of local and distant residual tumor cells after tumor surgery in a head and neck squamous carcinoma cell (HNSCC) patient-derived xenograft (PDX) model (Sosa, "Dormancy Programs as Emerging Antimetastasis Therapeutic Alternatives," Mol. Cell. Oncol. 3(1):e1029062 (2016)). The plasticity of NR2F1 expression suggests that changes in the epigenome of residual tumor cells may be controlled by external and internal signals and dictate the fate of DCCs. NR2F1 has been shown to limit induced pluripotent stem cell (iPS) reprogramming, probably by modulating chromatin reprogramming (Onder et al., "Chromatin Modifying Enzymes as Modulators of Reprogramming," Nature 483(7391):598-602 (2012)). NR2F1 is also key in maintaining a globally repressive chromatin in dormant tumor cells while simultaneously allowing for an active chromatin state in the promoters of specific dormancy genes, including its own promoter (Sosa et al., "NR2F1 Controls Tumour Cell Dormancy via SOX9- and RARbeta-Driven Quiescence Programmes," Nat. Commun. 6:6170 (2015)), which emphasizes the existence of an orchestrated epigenetic program modulated by NR2F1 and microenvironmental cues leading to tumor cell dormancy. In one embodiment, the DTCs are NR2F1⁺.

DCCs have been shown to express high levels of PERK pathway activation (Bragado et al., "Microenvironments Dictating Tumor Cell Dormancy," Recent Results Cancer Res. 195:25-39 (2012); Sosa et al., "Regulation of Tumor Cell Dormancy by Tissue Microenvironments and Autophagy," Adv. Exp. Med. Biol. 734:73-89 (2013); Goswami et al., "The Phosphoinositide 3-Kinase/Akt1/Par-4 Axis: A Cancer-Selective Therapeutic Target," Cancer Res. 66(6):2889-92 (2006); and Schewe et al., "ATF6alpha-Rheb-mTOR Signaling Promotes Survival of Dormant Tumor Cells in vivo," PNAS 105(30): 10519-24 (2008)), which is a mediator of the ISR. ISR signaling through PERK and EIF2α phosphorylation results in a decrease in general translation, as well as increases in gene specific translation, oxidative stress and ROS production, protein degradation, RNA degradation, autophagy, and lipid biosynthesis, which may aid in tumor cell survival.

The claimed aspect relates to a method of treating minimal residual cancer in a subject, which method involves contacting disseminated cancer cells (DCCs) in a subject with a protein kinase RNA-like endoplasmic reticulum kinase (PERK) inhibitor selected from LY2, LY3, and LY4, where said contacting eradicates DCCs in the subject to treat minimal residual cancer in the subject.

In one embodiment, the DCCs are phospho-PERK active. Accordingly, the method may further involve contacting DCCs in the subject with a PERK inhibitor, a MEK inhibitor, a CDK4/6 inhibitor, or any combination thereof.

Contacting may be carried out by administering a PERK inhibitor to the subject.

Otherwise disclosed is wherein the PERK inhibitor is a compound of formula (I) 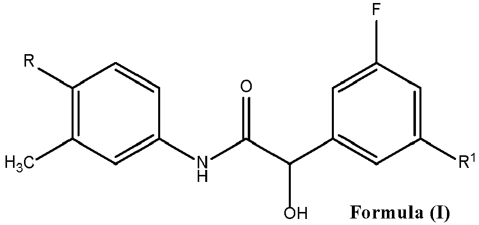 where R is selected from the group consisting of 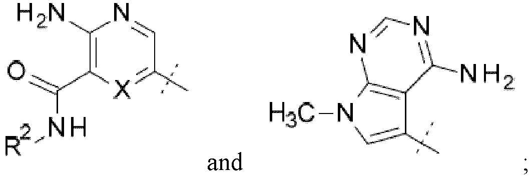
X is CH or N;
R¹ is hydrogen or halogen (*e.g*., fluoro); and
R² is C₁ to C₃ alkyl;
or a pharmaceutically acceptable salt thereof.

Otherwise disclosed is wherein the PERK inhibitor is a compound of formula (Ia) 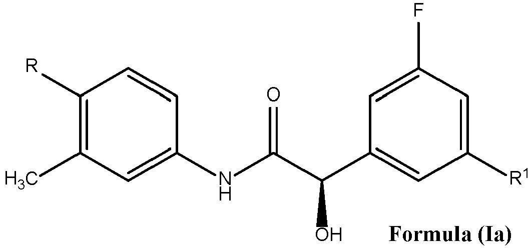 where R is selected from the group consisting of 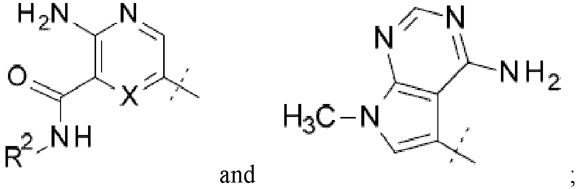
X is CH or N;
R¹ is hydrogen or halogen (*e.g*., fluoro); and
R² is C₁ to C₃ alkyl;
or a pharmaceutically acceptable salt thereof.

When the inhibitor is a compound of formula (I) or formula (Ia), R may be

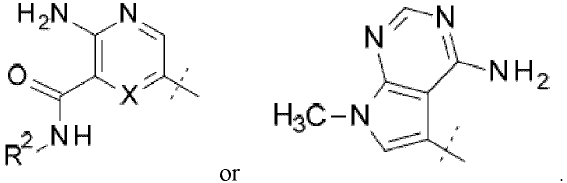

As used herein, the term "alkyl" means an aliphatic hydrocarbon group which may be straight or branched having about 1 to about 6 carbon atoms or 1 to about 3 carbon atoms in the chain (or the number of carbons designated by "Cₙ₋Cₙ", where *n* is the numerical range of carbon atoms). Branched means that one or more lower alkyl groups such as methyl, ethyl, or propyl are attached to a linear alkyl chain. Exemplary alkyl groups include methyl, ethyl, n-propyl, and i-propyl.

The term "halogen" means fluoro, chloro, bromo, or iodo. In one instance, halogen is fluoro.

The term "compound(s)" and equivalent expressions means compounds herein described, which expression includes the prodrugs, the pharmaceutically acceptable salts, the oxides, and the solvates, *e.g.* hydrates, where the context so permits.

Compounds described herein may contain one or more asymmetric centers and may thus give rise to enantiomers, diastereomers, and other stereoisomeric forms. Each chiral center may be defined in terms of absolute stereochemistry, as (R)- or (S)-. The present invention is meant to include all such possible isomers, as well as mixtures thereof, including racemic and optically pure forms. Optically active (R)- and (S)-, (-)- and (+)-, or (D)- and (L)-isomers may be prepared using chiral synthons or chiral reagents, or resolved using conventional techniques. All tautomeric forms are also intended to be included.

The recitation of "a compound" is intended to include salts, solvates, oxides, and inclusion complexes of that compound as well as any stereoisomeric form, or a mixture, of any such forms of that compound in any ratio. Thus, in accordance with some embodiments, a compound as described herein, including in the contexts of pharmaceutical compositions, methods of treatment, and compounds *per se,* is provided as the salt form.

The term "solvate" refers to a compound in the solid state, where molecules of a suitable solvent are incorporated in the crystal lattice. A suitable solvent for therapeutic administration is physiologically tolerable at the dosage administered. Examples of suitable solvents for therapeutic administration are ethanol and water. When water is the solvent, the solvate is referred to as a hydrate. In general, solvates are formed by dissolving the compound in the appropriate solvent and isolating the solvate by cooling or using an antisolvent. The solvate is typically dried or azeotroped under ambient conditions.

Inclusion complexes are described in Remington, The Science and Practice of Pharmacy, 19th Ed. 1:176-177 (1995). The most commonly employed inclusion complexes are those with cyclodextrins, and all cyclodextrin complexes, natural and synthetic, are specifically encompassed by the present invention.

The term "pharmaceutically acceptable salt" refers to salts prepared from pharmaceutically acceptable non-toxic acids or bases including inorganic acids and bases and organic acids and bases.

The term "pharmaceutically acceptable" means it is, within the scope of sound medical judgment, suitable for use in contact with the cells of humans and lower animals without undue toxicity, irritation, allergic response and the like, and are commensurate with a reasonable benefit/risk ratio.

Suitable PERK inhibitors are selected from LY2, LY3, LY4, and combinations thereof (*see* Table 4 below). The PERK inhibitor may be a pharmaceutically acceptable salt of LY2, LY3, and/or LY4.

**Table 4. Exemplary PERK Inhibitors**

| **Compound** | **Chemical Name** | **Structure** |
|---|---|---|
| LY2 | 3-amino-6-[4-[[(2R)-2-(3,5-difluorophenyl)-2-hydroxy-acetyl]amino]-2-methylphenyl]-N-methyl-pyrazine-2-carboxamide | 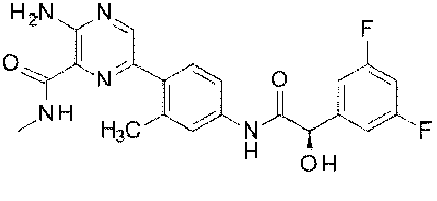 |
| LY3 | (2R)-N-[4-(4-amino-7-methyl-pyrrolo[2,3-d]pyrimidin-5-yl)-3-methyl-phenyl]-2-(3-fluorophenyl)-2-hydroxy-acetamide | 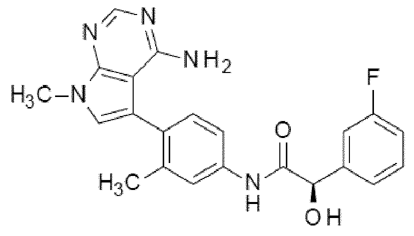 |
| LY4 | 2-amino-5-[4-[[(2R)-2-(3,5-difluorophenyl)-2-hydroxy-acetyl]amino]-2-methylphenyl]-N-isopropyl-pyridine-3-carboxamide | 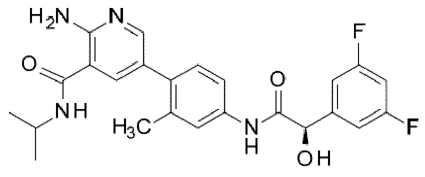 |

In some embodiments, contacting is carried out with a PERK inhibitor that does not inhibit EIF2AK1, EIF2AK2, or EIF2AK4.

In one embodiment, the PERK inhibitor does not inhibit AXL. In accordance with this embodiment, the PERK inhibitor is selected from LY3 and LY4.

In another embodiment, the PERK inhibitor does not inhibit Flt3, MNK2, or NTRK. In accordance with this embodiment, the PERK inhibitor is LY4.

Otherwise disclosed is wherein contacting is carried out by administering a MEK inhibitor to the subject. Exemplary MEK inhibitors are well known in the art and include, for example, PD184352, PD318088, PD98059, PD334581, RDEA119/BAY 869766 (*see, e.g*., Iverson et al., "RDEA119BAY 869766: A Potent, Selective, Allosteric Inhibitor of MEK1/2 for the Treatment of Cancer," Cancer Res. 69(17):6839-47 (2009)).

Otherwise disclosed is wherein contacting is carried out by administering a CDK4/6 inhibitor to the subject. Exemplary CDK4/6 inhibitors are well known in the art and include, for example Abemaciclib (LY2835219), palbociclib (PD0332991), and ribociclib (LEE011).

In one embodiment, the method may further involve selecting a subject with no evidence of disease prior to said contacting. For example, the subject may be in cancer remission prior to said contacting.

In carrying out the methods described herein, minimal residual cancer is treated in a subject. Such treatment may include, without limitation, administering to a subject in need of treatment for minimum residual cancer one or more compounds effective to treat the subject for the condition (*i.e*., cancer, or minimal residual cancer).

In one embodiment, treatment methods of the present disclosure are carried out under conditions effective to induce dormancy in disseminated tumor cells ("DTCs") and/or to induce dormant DTC death.

In carrying out the treatment methods of the present disclosure, administering of compounds to a subject may involve administering pharmaceutical compositions containing the compound(s) (*i.e*., a PERK inhibitor as claimed, or a BMP7 derivative protein as otherwise disclosed) in therapeutically effective amounts, which means an amount of compound effective in treating the stated conditions and/or disorders in the subject. Such amounts generally vary according to a number of factors well within the purview of persons of ordinary skill in the art. These include, without limitation, the particular subject, as well as the subject's age, weight, height, general physical condition, and medical history, the particular compound used, as well as the carrier in which it is formulated and the route of administration selected for it; the length or duration of treatment; and the nature and severity of the condition being treated.

Administering typically involves administering pharmaceutically acceptable dosage forms, which means dosage forms of compounds described herein and includes, for example, tablets, dragees, powders, elixirs, syrups, liquid preparations, including suspensions, sprays, inhalants tablets, lozenges, emulsions, solutions, granules, capsules, and suppositories, as well as liquid preparations for injections, including liposome preparations. Techniques and formulations generally may be found in Remington's Pharmaceutical Sciences, Mack Publishing Co., Easton, Pa., latest edition.

In carrying out treatment methods of the present disclosure, the drug (*i.e*., a PERK inhibitor as claimed, or a BMP7 derivative protein as otherwise disclosed) may be contained, in any appropriate amount, in any suitable carrier substance. The drug may be present in an amount of up to 99% by weight of the total weight of the composition. The composition may be provided in a dosage form that is suitable for the oral, parenteral (*e.g*., intravenously, intramuscularly), rectal, cutaneous, nasal, vaginal, inhalant, skin (patch), or ocular administration route. Thus, the composition may be in the form of, *e.g.,* tablets, capsules, pills, powders, granulates, suspensions, emulsions, solutions, gels including hydrogels, pastes, ointments, creams, plasters, drenches, osmotic delivery devices, suppositories, enemas, injectables, implants, sprays, or aerosols.

Pharmaceutical compositions according to the present disclosure may be formulated to release the active drug substantially immediately upon administration or at any predetermined time or time period after administration.

Controlled release formulations include (i) formulations that create a substantially constant concentration of the drug(s) within the body over an extended period of time; (ii) formulations that after a predetermined lag time create a substantially constant concentration of the drug(s) within the body over an extended period of time; (iii) formulations that sustain drug(s) action during a predetermined time period by maintaining a relatively constant, effective drug level in the body with concomitant minimization of undesirable side effects associated with fluctuations in the plasma level of the active drug substance; (iv) formulations that localize drug(s) action by, *e.g*., spatial placement of a controlled release composition adjacent to or in the diseased cell(s), tissue(s), or organ(s); and (v) formulations that target drug(s) action by using carriers or chemical derivatives to deliver the drug to a particular target cell type.

Administration of drugs in the form of a controlled release formulation may be preferred in cases in which the drug has (i) a narrow therapeutic index (*i.e*., the difference between the plasma concentration leading to harmful side effects or toxic reactions and the plasma concentration leading to a therapeutic effect is small; in general, the therapeutic index (TI) is defined as the ratio of median lethal dose (LD₅₀) to median effective dose (ED₅₀)); (ii) a narrow absorption window in the gastro-intestinal tract; or (iii) a very short biological half-life so that frequent dosing during a day is required in order to sustain the plasma level at a therapeutic level.

Any of a number of strategies can be pursued to obtain controlled release in which the rate of release outweighs the rate of metabolism of the drug in question. Controlled release may be obtained by appropriate selection of various formulation parameters and ingredients, including, *e.g*., various types of controlled release compositions and coatings. Thus, the drug is formulated with appropriate excipients into a pharmaceutical composition that, upon administration, releases the drug in a controlled manner (single or multiple unit tablet or capsule compositions, oil solutions, suspensions, emulsions, microcapsules, microspheres, nanoparticles, patches, and liposomes).

Thus, administering according to the methods of the present disclosure may be carried out orally, topically, transdermally, parenterally, subcutaneously, intravenously, intramuscularly, intraperitoneally, by intranasal instillation, by intracavitary or intravesical instillation, intraocularly, intraarterially, intralesionally, or by application to mucous membranes. Compounds may be administered alone or with suitable pharmaceutical carriers, and can be in solid or liquid form, such as tablets, capsules, powders, solutions, suspensions, or emulsions.

The drug (*i.e*., a PERK inhibitor as claimed, or a BMP7 derivative protein as otherwise disclosed) may be orally administered, for example, with an inert diluent, or with an assimilable edible carrier, or may be enclosed in hard or soft shell capsules, or may be compressed into tablets, or may be incorporated directly with the food of the diet. For oral therapeutic administration, the drug may be incorporated with excipients and used in the form of tablets, capsules, elixirs, suspensions, syrups, and the like. Such compositions and preparations should contain at least 0.001% of active compound. The percentage of the compound in these compositions may, of course, be varied and may conveniently be between about 0.01% to about 10% of the weight of the unit. The amount of active compound in such therapeutically useful compositions is such that a suitable dosage will be obtained. In one embodiment, compositions are prepared so that an oral dosage unit contains between about 1 µg and 1 g of active compound.

The tablets, capsules, and the like may also contain a binder such as gum tragacanth, acacia, corn starch, or gelatin; excipients such as dicalcium phosphate; a disintegrating agent such as corn starch, potato starch, alginic acid; a lubricant such as magnesium stearate; and a sweetening agent such as sucrose, lactose, or saccharin. When the dosage unit form is a capsule, it may contain, in addition to materials of the above type, a liquid carrier, such as a fatty oil.

Various other materials may be present as coatings or to modify the physical form of the dosage unit. For instance, tablets may be coated with shellac, sugar, or both. A syrup may contain, in addition to active ingredient, sucrose as a sweetening agent, methyl and propylparabens as preservatives, a dye, and flavoring such as cherry or orange flavor.

The therapeutic agent may also be administered parenterally. Solutions or suspensions can be prepared in water suitably mixed with a surfactant, such as hydroxypropylcellulose. Dispersions can also be prepared in glycerol, liquid polyethylene glycols, and mixtures thereof in oils. Illustrative oils are those of petroleum, animal, vegetable, or synthetic origin, for example, peanut oil, soybean oil, or mineral oil. In general, water, saline, aqueous dextrose and related sugar solution, and glycols such as, propylene glycol, hyaluronan and its derivatives, carboxymethyl cellulose and other soluble polysaccharide derivatives, or polyethylene glycol, are preferred liquid carriers, particularly for injectable solutions. Under ordinary conditions of storage and use, these preparations contain a preservative to prevent the growth of microorganisms if they are not produced aseptically.

The pharmaceutical forms suitable for injectable use include sterile aqueous solutions or dispersions and sterile powders for the extemporaneous preparation of sterile injectable solutions or dispersions. The form must be sterile and must be fluid to the extent that easy syringability exists. It must be stable under the conditions of manufacture and storage and must be protected against the contaminating action of microorganisms, such as bacteria and fungi. The carrier can be a solvent or dispersion medium containing, for example, water, ethanol, polyol (*e.g*., glycerol, propylene glycol, and liquid polyethylene glycol), suitable mixtures thereof, and vegetable oils.

The therapeutic agent may also be administered directly to the airways in the form of an aerosol. For use as aerosols, the therapeutic agent in solution or suspension may be packaged in a pressurized aerosol container together with suitable propellants, for example, hydrocarbon propellants like propane, butane, or isobutane with conventional adjuvants. The therapeutic agent also may be administered in a non-pressurized form such as in a nebulizer or atomizer.

In one embodiment, administering may increase the amount of detectable dormant DCCs in a subject by at least about 1%, 2%, 3%, 4%, 5%, 6%, 7%, 8%, 9%, 10%, 20%, 30%, 40%, 50%, 60%, 70%, 80%, 90%, 95%, 99%, or more.

In another embodiment, administering may decrease the amount of detectable DCCs in a subject by at least about 1%, 2%, 3%, 4%, 5%, 6%, 7%, 8%, 9%, 10%, 20%, 30%, 40%, 50%, 60%, 70%, 80%, 90%, 95%, 99%, or more.

Within the context of the present disclosure, "treating" is meant the maintenance of no evidence of symptomatic disease (*e.g*., cancer) in a subject.

In one embodiment, the term "treating" or "treatment" designates in particular the elimination of minimal residual cancer in a subject. The term treatment includes the induction of dormancy in DCCs. The term treatment also includes the elimination of dormant DCCs in the subject. The term treatment also includes a decrease in the amount or number of detectable dormant DCCs in a subject.

The claimed aspect relates to a method of treating minimal residual cancer in a subject. This method involves contacting disseminated cancer cells (DCCs) in a subject with a protein kinase RNA-like endoplasmic reticulum kinase (PERK) inhibitor selected from LY2, LY3, and LY4, where said contacting eradicates DCCs in the subject to treat minimal residual cancer in the subject.

As described above, the methods of the present disclosure are suitable for treating minimal residual cancer in a subject that has been diagnosed with any one or more of breast cancer, multiple myeloma, lung cancer, non-small cell lung cancer, brain cancer, cervical cancer, mantel cell lymphoma, leukemia, hepatocellular carcinoma, prostate cancer, melanoma, skin cancers, head and neck cancers, thyroid cancer, glioblastoma, neuroblastoma, colorectal cancer, and other cancers.

The cancer may be a breast cancer selected from invasive breast cancer, ductal carcinoma in situ (DCIS), lobular carcinoma in situ (LCIS), and inflammatory breast cancer.

In one embodiment, the breast cancer is a HER2⁺ breast cancer.

In another embodiment, the subject has been diagnosed with disseminated tumor cells and/or a non-metastatic cancer.

As described above, the methods of the present disclosure may further involve administering to the subject a chemotherapeutic agent, an immunotherapeutic agent, an epigenetic agent, or ionizing radiation.

When a chemotherapeutic agent is administered to the subject, the chemotherapeutic agent may be an anti-HER2 chemotherapeutic agent selected from trastuzumab (Herceptin^{®}) and lapatinib (Tykerb^{®}). In another embodiment, the chemotherapeutic agent may be selected from an anthracycline, a taxane, a kinase inhibitor, an antibody, a fluoropyrimidine, and a platinum drug.

When an immunotherapeutic agent is administered to the subject, the immunotherapeutic agent is selected from an immune checkpoint inhibitor, an interferon, or a tumor vaccine.

When an epigenetic agent is administered to the subject, the epigenetic agent may be selected from a histone deacetylase (HDAC) inhibitor, 5-azacytidine, retinoic acid, arsenic trioxide, Enhancer Of Zeste 2 Polycomb Repressive Complex 2 Subunit ("EZH2") inhibitor, bromodomain (BRD) inhibitor, and derivatives thereof.

Contacting may be carried out by administering the PERK inhibitor to the subject. PERK inhibitors for use according to the invention are LY2, LY3 and LY4. Otherwise disclosed are suitable PERK inhibitors described in detail above.

In one embodiment, the method further involves detecting the presence of DTCs in the subject prior to said contacting. As described in more detail above, the DTCs may be NR2F1⁺, phospho-PERK active, and/or BMPR⁺.

The method may further involve contacting DCCs/DTCs in the subject with a BMP7 derivative protein. In one embodiment, contacting DCCs/DTCs in the subject with a BMP7 derivative protein is carried out by administering the BMP7 derivative protein to the subject. Suitable BMP7 derivative proteins are described above. In one embodiment, the BMP7 derivative protein is BMP7-F9.

In one embodiment, the PERK inhibitor does not inhibit EIF2AK1, EIF2AK2, or EIF2AK4.

As described above, the subject may be a mammal, preferably a human.

In one embodiment, the method may further involve selecting a subject with no evidence of disease prior to said contacting. For example, the subject may be in cancer remission prior to said contacting.

Another aspect otherwise disclosed herein relates to a method of treating late stage cancer in a subject. This method involves contacting disseminated cancer cells (DCCs) in a subject with a protein kinase RNA-like endoplasmic reticulum kinase (PERK) inhibitor selected from LY2, LY3, and LY4, where said contacting eradicates DTCs in the subject to treat minimal residual cancer in the subject.

As used herein, the term "late stage cancer" refers to stage II cancer, stage III cancer, and/or stage IV cancer, or to any cancer that has metastasized. It will be appreciated that the "late stage" nature of the cancer disease states can be determined by a physician.

As described in detail above, the subject may have been diagnosed with breast cancer, multiple myeloma, lung cancer, non-small cell lung cancer, brain cancer, cervical cancer, mantel cell lymphoma, leukemia, hepatocellular carcinoma, prostate cancer, melanoma, skin cancers, head and neck cancers, thyroid cancer, glioblastoma, neuroblastoma, or colorectal cancer.

In one instance, the cancer is breast cancer selected from invasive breast cancer, ductal carcinoma in situ (DCIS), lobular carcinoma in situ (LCIS), and inflammatory breast cancer. The breast cancer may be is a HER2⁺ breast cancer.

The method may further involve administering to the subject a chemotherapeutic agent, an immunotherapeutic agent, an epigenetic agent, or ionizing radiation. In one instance, the chemotherapeutic agent is an anti-HER2 chemotherapeutic agent selected from trastuzumab (Herceptin^{®}) and lapatinib (Tykerb^{®}). In another instance, the chemotherapeutic agent is selected from an anthracycline, a taxane, a kinase inhibitor, an antibody, a fluoropyrimidine, and a platinum drug. The immunotherapeutic agent may be selected from an immune checkpoint inhibitor, an interferon, or a tumor vaccine. The epigenetic agent may be selected from a histone deacetylase (HDAC) inhibitor, 5-azacytidine, retinoic acid, arsenic trioxide, Enhancer Of Zeste 2 Polycomb Repressive Complex 2 Subunit (EZH2) inhibitor, bromodomain (BRD) inhibitor, and derivatives thereof.

The contacting may be carried out by administering the PERK inhibitor to the subj ect.

The method may further involve detecting the presence of DCCs/DTCs in the subject prior to said contacting.

As described above, the DTCs may be NR2F1⁺ or phospho-PERK active.

### EXAMPLES

### Example 1 - Materials and Methods for Examples 2-6

*Reagents, cell culture, and treatments:* EGF was obtained from PeproTech (Rocky Hill, NJ) and used at 100 ng/ml. Thapsigargin was from Sigma (St. Louis, MO) and used at 2 nM. The ZR75.1-H2B-Dendra2 cell line was generated by stable transfection of the H2B-Dendra2 plasmid (Gurskaya et al., "Engineering of a Monomeric Green-to-Red Photoactivatable Fluorescent Protein Induced by Blue Light," Nat. Biotechnol. 24:461-465 (2006)). For 3D cultures, MCF10A-HER2, SKBR3, and ZR75.1-H2B-Dendra2 cells were plated in growth factor-reduced Matrigel (Corning, Corning, NY) and grown as described previously (Avivar-Valderas et al., "Regulation of Autophagy during ECM Detachment is Linked to a Selective Inhibition of mTORC1 by PERK," Oncogene 32(41):4932-40 (2013)). When referring to "low density," 3,500 cells/8-well were seeded, and for "high density" 20,000 cells/8-well. Treatments with vehicle (DMSO) or LY4 (2 µM) were replaced every 24 hours for 2D and every 48 hours for 3D cultures.

*Mice, tumor growth, and tissue processing:* The FVB/N-Tg (MMTVneu) mouse strain was obtained from Jackson Laboratories (Sacramento, CA). These mice express the unactivated neu (HER2) form under the transcriptional control of the mouse mammary tumor virus promoter/enhancer. Before being used in any experiment, females underwent one round of pregnancy and at least two weeks of no lactation after weaning. Females between 24-32 weeks of age were injected intraperitoneally with vehicle (90% corn oil, 10% ethanol) or LY4 (50 mpk) daily, for two weeks. For the combination treatment, females 24-32 weeks of age were treated daily by oral gavage with Abemaciclib (50 mpk) for 4 weeks before starting the treatment described earlier with LY4. Tumor volumes were measured using the formula (Dxd²)/2, where D is the longest and d is the shortest diameter. For CTC count, animals were anesthetized and whole blood was extracted by cardiac puncture. Mammary glands, lungs, and tumors were collected and fixed in 10% buffered formalin overnight before paraffin embedding. The bone marrow from the two lower limbs was flushed with a 26 G needle and further processed by Ficoll density gradient centrifugation. For CTC as well as for DTC detection in bone marrow, tissues were depleted of mature hematopoietic cells by anti-mouse antibody-labeled magnetic bead separation (Miltenyi Biotec, San Diego, CA) before fixation in formalin for 20 minutes at 4°C.

*Mammary gland whole mount staining:* Mammary glands fixed in 10% buffered formalin were incubated in Carmine Alum stain (Carmine 0.2%, Aluminum potassium sulfate 0.5%) (Sigma, St. Louis, MO) for 2 days. Then, they were dehydrated and transferred to methyl salicylate solution before imaging using a stereomicroscope.

*IHC and IF:* IHC and IF from paraffin-embedded sections was performed as previously described (Avivar-Valderas et al., "Regulation of Autophagy during ECM Detachment is Linked to a Selective Inhibition of mTORC1 by PERK," Oncogene 32(41):4932-40 (2013). Briefly, slides were dewaxed and serially rehydrated. Heat-induced antigen retrieval was performed in either citrate buffer (10 mM, pH 6), EDTA buffer (1 mM, pH 8), or Tris/EDTA (pH9). Slides were further permeabilized in 0.1% Triton^{™}-X100, blocked and incubated with primary antibody overnight at 4°C at 1:50-1:200 dilution. For IHC, an additional step of endogenous peroxidase and avidin/biotin quenching was performed before primary antibody incubation. Primary antibodies used were anti-cytokeratin 8/18 (Progen, Heidelberg, Germany), smooth muscle actin-Cy3 (Sigma, St. Louis, MO), P-PERK(T980) (Tenkerian et al., "mTORC2 Balances AKT Activation and eIF2alpha Serine 51 Phosphorylation to Promote Survival Under Stress," Mol. Cancer Res. 13:1377-1388 (2015)), P-EIF2A, Cleaved Caspase 3, P-H3(S10), P-HER2(Y1221/1222) (Cell signaling, Danvers, MA), P-Rb(S249/T252) (Santa Cruz, Dallas, TX), HER2 (Abcam, Cambridge, MA), HER2 (Millipore, Darmstadt, Germany), Ki67 (eBioscience and Abcam), cytokeratin cocktail (C11 and ck7, Abcam; AE1 and AE3, Millipore), and GADD34 (Santa Cruz). Next, slides were incubated in secondary antibodies (Life Technologies, Norwalk, CT) and mounted. For IHC, sections were processed using VectaStain ABC Elite kit (Vector Laboratories, Bulingame, CA) and DAB Substrate kit for peroxidase labelling (Vector Laboratories) and mounted in VectaMount medium (Vector laboratories). For IF, sections were mounted in ProLong Gold Antifade aqueous medium (Thermo Fisher, Waltham, MA).

In the case of immunocytofluorescence, cytospins of fixed cells (100,000 - 200,000 cells/cytospin were prepared by cyto-centrifugation at 500 rpm for 3 minutes on poly-prep slides, and the staining protocol was performed as explained below from the permeabilization onward. For the staining of 3D cultures, acini were fixed in 4% PFA for 20 minutes at 4°C, permeabilized with 0.5% Triton^{™}-X100 in PBS for 20 minutes at room temperature, washed in PBS-glycine, and then blocked with 10% normal goat serum for 1 hour at 37°C, before performing immunofluorescence staining.

The scoring for P-HER2 levels is explained in FIG. 10A. For the scoring of CK8/18 and SMA in mammary gland ducts, 20 low magnification fields were evaluated per animal for the expression of CK8/18 as negative (0), low (1), or high (2) and the same for SMA and the sum of the two scores was considered as the final score (from 0 to 4).

*Microscopy:* Images were captured by using a Nikon Eclipse TS100 microscope, a Leica DM5500 or confocal Leica SP5 multiphoton microscope.

*TUNEL in situ cell death detection:* Apoptosis levels were evaluated using the *In situ* Cell Death Detection kit, AP (Roche, Basel, Switzerland). Paraffin sections from tumors were dewaxed, rehydrated, and permeabilized in phosphate buffered saline (PBS) 0.2% TRITON^{™}-X100 for 8 minutes. Then, slides were washed and blocked in 20% normal goat serum for 1hour at 37°C. The TUNEL reaction mixture was then added and let go for 1hour at 37°C. The reaction was stopped by incubating with Buffer I (0.3 M Sodium chloride, 30 mM Sodium citrate). Next, the slides were incubated with anti-fluorescein-AP antibody for 30 minutes at 37°C. After three washes in Tris buffered saline (TBS), slides were incubated in alkaline phosphatase substrate in 0.1% TWEEN^{™}-20 for 20 min at room temperature. Finally, the slides were mounted using aqueous mounting medium. The percentage of TUNEL positive cells was calculated using Image J software (NIH).

*Immunoblot analysis:* Cells were lysed in RIPA buffer and protein analyzed by immunoblotting as described previously (Ranganathan et al., "Functional Coupling of p38-Induced Up-Regulation of BiP and Activation of RNA-Dependent Protein Kinase-Like Endoplasmic Reticulum Kinase to Drug Resistance of Dormant Carcinoma Cells," Cancer Res. 66:1702-1711 (2006)). Membranes were blotted using the additional following antibodies: P-PERK (T982) (Tenkerian et al., "mTORC2 Balances AKT Activation and eIF2alpha Serine 51 Phosphorylation to Promote Survival Under Stress," Mol. Cancer Res. 13:1377-1388 (2015)), PERK (Santa Cruz, Dallas, TX), P-EGFR(Y1148), EGFR, P-AKT(S473), P-S6 (S235/236) (Cell signaling, Danvers, MA), GAPDH (Millipore, Darmstadt, Germany), and β-Tubulin (Abcam, Cambridge, MA). For induction of ER stress, MCF10A-HER2 cells were plated in low adhesion plates for 24 hours before collection.

*Cell surface biotinylation and endocytosis assay:* For cell surface biotinylation, Pierce cell surface protein isolation kit was used following manufacturer's instructions with minor changes. Briefly, MCF10A-HER2 cells were serum- and EGF-starved and treated +/-LY4 for 24 hours before being stimulated with +/- EGF (100 ng/ml) for 20'. Then, cells were washed with ice-cold PBS and surface proteins biotinylated for 30 minutes at 4°C. After quenching, cells were harvested and lysed using RIPA buffer. Protein lysates were incubated with NeutrAvidin agarose beads and the bound proteins were released by incubation with SDS-PAGE sample buffer containing DTT (50 mM). For endocytosis assays (Cihil et al., "The Cell-Based L-Glutathione Protection Assays to Study Endocytosis and Recycling of Plasma Membrane Proteins," J. Vis. Exp. e50867 (2013)), cells were treated similarly but before treatment with EGF cell surface proteins were biotinylated. After 20 minute incubation +/- EGF (100 ng/ml) at 37°C (to induce endocytosis), cells were washed with ice-cold PBS and incubated with stripping buffer (to remove cell surface biotinylation: 75 mM NaCl, 1mM MgCl2, 0.1mM CaCl2, 50 mM glutathione and 80 mM NaOH, pH 8.6) for 30'. To control for stripping efficiency, cells were stripped without 37°C incubation (t=0). Cell lysates were prepared and processed for biotinylated protein isolation as described before.

*Single cell targeted gene expression analysis:* Primary tumors from MMTV-neu 28-30-week old females were digested with collagenase into a single cell suspension. Lungs from MMTV-neu 15-30-week old females were digested into a single cell suspension with collagenase and resuspended in FACS buffer. Cells were then stained with anti-HER2-PE, anti-CD45-APC and DAPI and the HER2+/CD45- population of cells sorted using a BDFACSAria sorter. Sorted cells were resuspended at a 312,500 cells/ml concentration in media and 80 µl were mixed with 20 µl suspension reagent (C1 Fluidigm). A C1 Single-cell Preamp IFC 10-17 µm was used for the single cell separation. Pre-amplification was run using Ambion Single Cell-to-CT qRT-PCR kit and 20x TaqMan Gene expression FAM-MGB assays. Resulting cDNA was further diluted in C1 DNA dilution reagent 1/3 and used for gene expression analysis using 96.96 IFCs (Fluidigm), Juno System controller and Biomark HD for high-throughput qPCR. TaqMan Fast Advanced Master Mix was used for the qPCR reactions. Analysis was performed using Fluidigm Real-Time PCR Analysis Software and Clustergrammer web-based tool (Fernandez et al., "Clustergrammer, A Web-Based Heatmap Visualization and Analysis Tool for High-Dimensional Biological Data," Sci. Data 4:1-12 (2017)) for hierarchical clustering heatmaps.

*Biochemical assays:* Recombinant human EIF2AK3 (PERK) catalytic domain (amino acids 536-1116; Cat# PV5107), GFP-eIF2α (Cat# PV4809) substrate, and Terbium-labelled phospho-eIF2α antibody (Cat# PR8956B) were purchased from Invitrogen (Carlsbad, CA). HIS-SUMO-GCN2 catalytic domain (amino acids 584-1019) was expressed and purified from *E. coli.* TR-FRET kinase assays were performed in the absence or presence of inhibitors in a reaction buffer consisting of 50 mM HEPES, pH 7.5, 10 mM MgCl₂, 1.0 mM EGTA, and 0.01% Brij-35, and 100-200 nM GFP-eIF2α substrate. PERK assays contained 62.5 ng/ml enzyme and 1.5 µM ATP (K_{m, app} ~1.5 µM) and GCN2 assays contained 3 nM enzyme and 90 µM ATP (K_{m, app} ~200 µM). Following the addition of test compound, the reaction was initiated by addition of enzyme and incubated at room temperature for 45 minutes. The reaction was stopped by addition of EDTA to a final concentration of 10 mM and Terbium-labelled phospho-eIF2α antibody was added at a final concentration of 2 nM and incubated for 90 minutes. The resulting fluorescence was monitored in an EnVison^{®} Multilabel reader (PerkinElmer, Waltham, MA). TR-FRET ratios and the resulting IC₅₀ values were determined from the fitted inhibition curves. Biochemical specificity profiling was performed at Cerep (Redmond, WA) and DiscoverX (San Diego, CA).

*Cell-based TR-FRETassay:* Briefly, GripTite^{™} 293 cells (Invitrogen) expressing GFP-eIF2α were seeded at 10,000 cells per well in 384-well plates and allowed to attach overnight. Cells were pre-treated with test compounds for 1 hour. Tunicamycin (1 µM) was added to induce PERK activity and the plates were incubated at 37°C for 2 hours. The culture media was removed and the cells were lysed in buffer consisting of 20 mM Tris-HCl, pH 7.5, 150 mM NaCl, 5 mM EDTA, 1% NP-40, 5 mM NaF, Protease inhibitors (Sigma Cat# P8340), Phosphatase inhibitors (Sigma Cat# P2850), and 2 nM Terbium-labelled anti-phospho-eIF2 antibody (Invitrogen Cat# PM4312I). Cell lysates were incubated for 2 hours in the dark at room temperature and fluorescence was monitored in an EnVison^{®} Multilabel reader (PerkinElmer, Waltham, MA). TR-FRET ratios and the resulting IC50 values were determined from the fitted inhibition curves using un-induced (100% inhibition) and induced (0% inhibition) wells as controls.

*ATF4-luc assay:* 293 cells were transduced with an ATF4-luc expressing lentivirus (SABiosciences, Frederick, MD) and selected in growth medium containing 1 µg/ml puromycin. To determine the effect of compounds on ER stress-induced ATF4 activity, 293-ATF4-luc cells were seeded at 15,000 cells per well in poly-D-Lysine coated 96-well plates and allowed to attach overnight. The cells were then pre-treated with test compounds for 30 minutes. Tunicamycin (2 µM) was added to induce ER stress and the plates were incubated at 37°C for 6 hours. The culture media was then aspirated and the cells were lysed in passive lysis buffer (Promega Cat# E194A) on a plate shaker for 5 minutes. Luciferase activity was monitored using Luciferase Assay Reagent (Promega Cat# E1501) in a Wallac 1420 Victor2^{™} Multilabel Counter (PerkinElmer, Waltham, MA) and IC₅₀ values were determined from the resulting fitted inhibition curves using un-induced (100% inhibition) and induced (0% inhibition) wells as controls.

*Cell viability assays:* Hela, HT-1080, and Bx-PC-3 cells were monitored for growth in 96-well plates in the absence or presence of PERK inhibitors for 48, 72, or 96 hours, respectively. Cell viability was determined using CellTiter-Glo^{®} reagent (Promega, Madison, WI) and IC₅₀ values were determined from the resulting fitted inhibition curves using untreated (0% inhibition) and wells treated with 20 µM staurosporine (100% inhibition) as controls.

*Statistical analysis:* All points represent independent biological samples with error bars representing standard deviations and statistical significance was determined using a Mann-Whitney test using the Graph Pad Prism Software.

### Example 2 - Quiescent HER2⁺ DTCs Display an ER Stress Response

PERK pathway activation has been shown to serve as a crucial effector of UPR-induced growth arrest and survival linked to a dormant phenotype (Brewer et al., "PERK Mediates Cell-Cycle Exit During the Mammalian Unfolded Protein Response," Proc. Natl. Acad. Sci. U.S.A. 97:12625-30 (2000); Ranganathan et al., "Dual Function of Pancreatic Endoplasmic Reticulum Kinase in Tumor Cell Growth Arrest and Survival," Cancer Res. 68:3260-3268 (2008); and Ranganathan et al., "Functional Coupling of p38-Induced Up-Regulation of BiP and Activation of RNA-Dependent Protein Kinase-Like Endoplasmic Reticulum Kinase to Drug Resistance of Dormant Carcinoma Cells," Cancer Res. 66:1702-1711 (2006)). In MMTV-HER2 animals a high percentage of mice develop metastases to the lungs, which can be initiated by early DCCs or late DCCs (Guy et al., "Expression of the Neu Protooncogene in the Mammary Epithelium of Transgenic Mice Induces Metastatic Disease," Proc. Nat'l. Acad. Sci. U.S.A. 89:10578-10582 (1992); Husemann et al., "Systemic Spread Is an Early Step in Breast Cancer," Cancer Cell 13:58-68 (2008); Harper et al., "Mechanism of Early Dissemination and Metastasis in Her2+ Mammary Cancer," Nature 540:588-592 (2016); Hosseini et al., "Early Dissemination Seeds Metastasis in Breast Cancer," Nature 540:552-558 (2016)). Dormant DCCs display loss of E-cadherin and expression of Twist1 (Harper et al., "Mechanism of Early Dissemination and Metastasis in Her2+ Mammary Cancer," Nature 540:588-592 (2016)) and E-cadherin-negative DCCs in pancreatic cancer models were also shown to be quiescent and displayed upregulation of CHOP, a PERK-induced gene (Pommier et al., "Unresolved Endoplasmic Reticulum Stress Engenders Immune-Resistant, Latent Pancreatic Cancer Metastases," Science 360(6394):eaao4908 (2018)). To evaluate whether this same correlation between the levels of PERK pathway activation and cell cycle arrest is present in the MMTV-HER2 spontaneous metastasis model, two different approaches were used - high resolution imaging using immunofluorescence (IF) and single cell resolution gene expression analysis of DCCs and metastasis. IF of MMTV-HER2 lung tissue sections of animals bearing large tumors and thus bearing dormant and proliferative DCCs was performed (Harper et al., "Mechanism of Early Dissemination and Metastasis in Her2+ Mammary Cancer," Nature 540:588-592 (2016)). Next, the tissues were co-stained to detect DCCs positive for HER2, Ki67 (as a marker of proliferation), and GADD34 (or PPP1r15A). GADD34 is a PERK-inducible stress gene responsible for the programmed shift from translational repression (due to eIF2α phosphorylation) to stress-induced gene expression (Novoa et al., "Stress-Induced Gene Expression Requires Programmed Recovery from Translational Repression," EMBO J. 22:1180-7 (2003)). Image analysis showed that HER2⁺ metastatic lesions or DCCs with a low proliferative index (ki67^{low}) presented high levels of ER stress as shown by high levels of GADD34 expression (FIG. 1A, upper panels and graph). On the other hand, highly proliferative DCCs or lesions showed very low levels of GADD34 staining (FIG. 1A, lower panels and graph). The two markers, Ki67 and GADD34, were anti-correlated in 100% of the cells, supporting that UPR^{high}, quiescent DCCs, and metastatic lesions can be biomarked by GADD34 detection.

Next, whether these correlations would also hold true in human breast metastatic lesions was evaluated by testing 17 breast cancer metastases from different subtypes and sources (lymph node, lung, liver) (Table 5). The breast cancer metastasis were stained for cytokeratins to identify the metastatic lesions, Ki67, and GADD34. Advanced human metastatic lesions displayed a more heterogeneous pattern of staining for both markers between different patients and in-between different areas of the same lesion than in the mouse model. However, an opposite correlation between levels of proliferation (Ki67) and ER stress activation (GADD34), independently of metastasis type was observed (FIG. 1B). This analysis validates the findings in the mouse models and that GADD34 may help identify UPR^{high}/quiescent tumor cells in metastatic sites.

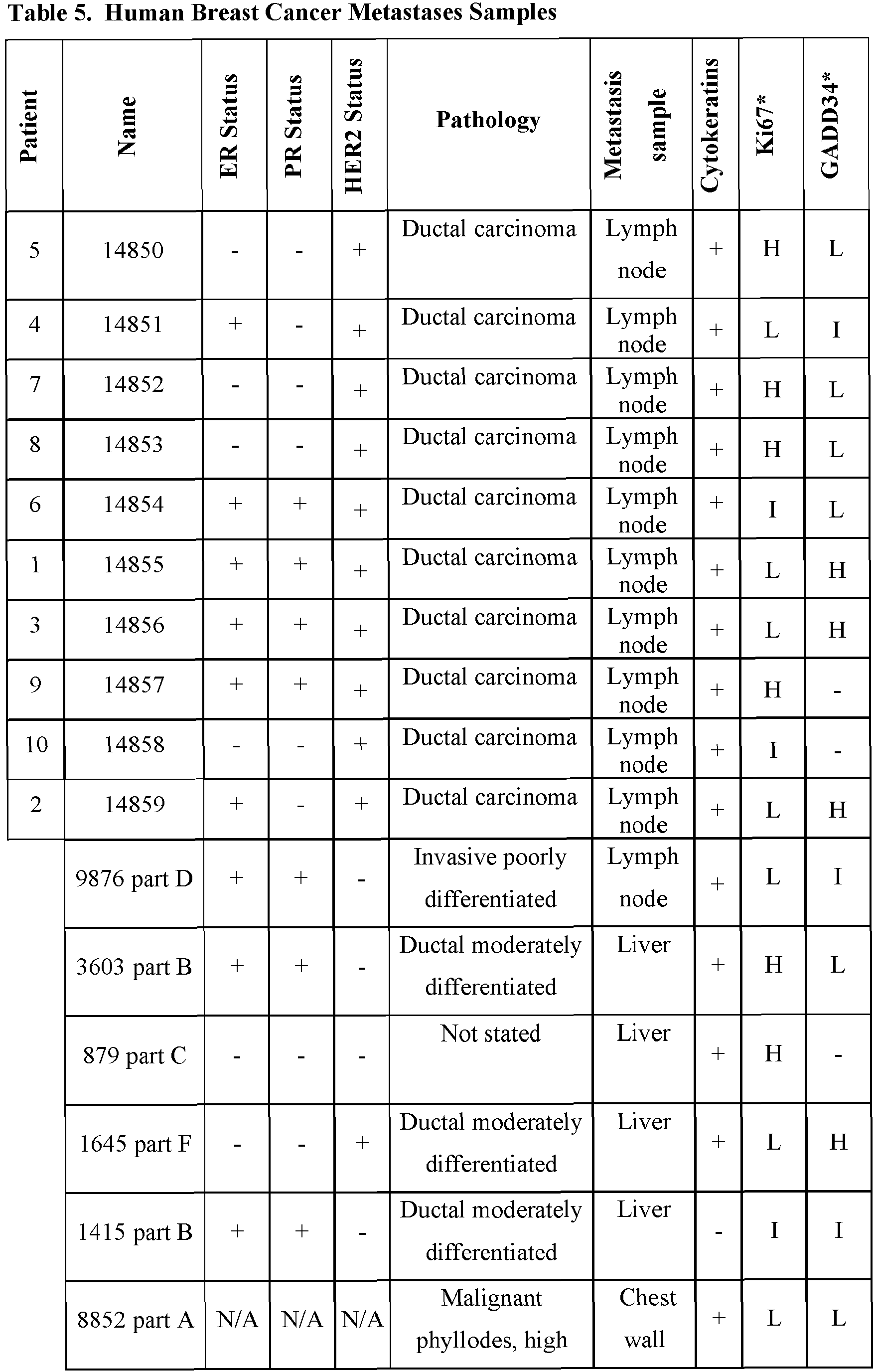

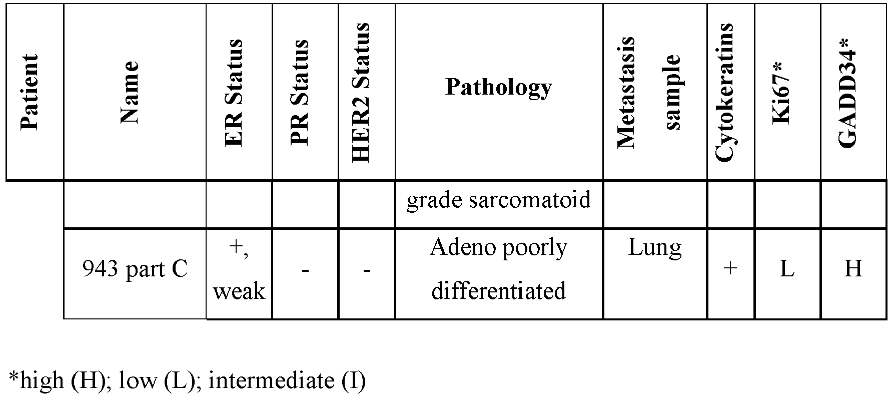

Markers of proliferation, quiescence, dormancy, and ER stress present in metastatic cells were evaluated by performing single cell targeted gene expression analysis of DCCs, micro-metastasis, and macro-metastases lodged in lungs of MMTV-HER2 mice. Lungs from MMTV-HER2 females were processed into single cell suspensions and HER2⁺/CD45⁻ cells were sorted (FIG. 2A). The sorted cells were then processed for single cell separation, lysis, RT, and pre-amplification using the C1 (Fluidigm) technology as shown in FIG. 2A. This pipeline allowed for the isolation and processing, with high degree of confidence (IF and molecular confirmation of HER2⁺ single cell) and quality, of 255 single DCCs and 90 primary tumor cells and their corresponding pools. Next, high-throughput qPCR was used to analyze the expression of ER stress genes, cell cycle genes (both activators and inhibitors), and dormancy genes (Kim et al., "Dormancy Signatures and Metastasis in Estrogen Receptor Positive and Negative Breast Cancer," PloS One 7:e35569 (2012); B'chir et al., "The eIF2α/ATF4 Pathway is Essential for Stress-Induced Autophagy Gene Expression," Nucleic Acids Res. 41:7683-99 (2013); Harper et al., "Mechanism of Early Dissemination and Metastasis in Her2+ Mammary Cancer," Nature 540:588-592 (2016)) (FIG. 2B). The single cell resolution gene expression of DCCs revealed the existence of a population of cells (FIG. 1C, group 1, approximately 19% of the DTCs) that show concomitant and strong upregulation of all the ER stress genes tested (including PERK itself) (box encompassing Fam 123b-Ddit3) with negative regulators of cell proliferation such as Rb1 and TP53 and CDK inhibitors p21, p27, p16 and p15 (box encompassing Cdkn2a-Rb1) (FIG. 1C). Enrichment in the expression of dormancy genes such as NR2F1, DEC2 (*Bhlhe41*), TWIST1, CDH5, STAT3, and COL4a5 was also observed in these cells (Kim et al., "Dormancy Signatures and Metastasis in Estrogen Receptor Positive and Negative Breast Cancer," PIoS One 7:e35569 (2012) and Harper et al., "Mechanism of Early Dissemination and Metastasis in Her2+ Mammary Cancer," Nature 540:588-592 (2016)) (box encompassing Nr2f1-Ccnd1). Another group of DCCs, group 2 (22%) also showed high levels of ER stress gene expression along with p21. Group 3 (6%) showed fewer of the ER stress, cell cycle inhibitors, and dormancy genes, suggesting that these might represent cells transiting out of dormancy or in slow cycling mode. In total, around 40% of the DCCs showed a high to intermediate level of ER stress gene expression, simultaneous with cell cycle inhibitors or dormancy genes. This is in range with the percentage of dormant DCCs detected in advanced progression MMTV-HER2 animals using phosho-Histone H3 and phospho-Rb detection (Harper et al., "Mechanism of Early Dissemination and Metastasis in Her2+ Mammary Cancer," Nature 540:588-592 (2016)). Altogether, this data shows that, even in animals with detectable metastasis, -40% of DCCs display high expression of cell cycle inhibitors. Importantly, in this model, this dormant DCC subpopulation displays an unresolved UPR with prominent activation expression of PERK pathway genes.

### Example 3 - PERK Inhibition Eradicates Quiescent DCCs in Bone Marrow and Lungs and in Turn Suppresses Lung Metastasis

The results of the above examples prompted an evaluation of the effects of selective PERK inhibitors on dormant DCC fate and metastasis formation. LY2, LY3, and LY4 (LY series inhibitors) have been identified as potent and selective PERK inhibitors with appropriate drug-like properties to support *in vivo* studies (Pytel et al., "PERK Is a Haploinsufficient Tumor Suppressor: Gene Dose Determines Tumor-Suppressive Versus Tumor Promoting Properties of PERK in Melanoma," PLoS Genet. 12:1-22 (2016)). The LY series inhibitors were tested in *in vitro* kinase assays (using eIF2α as substrate) and cell-based assays looking at eIF2α phosphorylation and its downstream output ATF4 (Table 6). All three inhibitors showed similar or superior potency compared to GSK2656157 (Axten et al., "Discovery of GSK2656157: An Optimized PERK Inhibitor Selected for Preclinical Development," ACSMed. Chem. Lett. 4:964-968 (2013)); effectively decreased P-PERK (P-T980) levels and its downstream target ATF4 in MCF10A cells expressing HER2 (FIG. 2C and FIG. 3A); and rendered these same cells sensitive to low dose thapsigargin treatment, thereby showing how these PERK inhibitors selectively affect adaptation to ER stress (FIG. 2D).

Using biochemical enzymatic assays (FIG. 2E), LY4 showed the highest specificity presenting no secondary kinase targets below 15 µM concentration, while LY2, LY3, and GSK2656157 presented several secondary targets below 5 µM, and even at 1 µM concentration. Using DicoveRₓ scanMAX^{™} kinase profiling (Table 6), it was corroborated that LY4 displayed greater selectivity compared to the other inhibitors, even at a very high concentration (20 µM), at which LY4 inhibited only 20 kinases by >50% out of a total of 456 compared to 80 by GSK2656157, or 8 compared to 58 by >60% inhibition. None of these secondary targets happened to be any of the other known eIF2α kinases, EIF2AK1 (also known as HRI), EIF2AK2 (also known as PKR) and EIF2AK4 (also known as GCN2) (Table 7), indicating that the measured activity on eIF2α was highly specific to PERK inhibition.

**Table 6. Enzymatic and Cell-Based IC₅₀ Values and Kinase Selectivity of the Different PERK Inhibitors**

| **Compound** | **PERK^{a} Enzyme IC₅₀ (µM)** | **PERK^{b} Cell-based IC₅₀ (µM)** | **ATF4-luc^{c} Cell-based IC₅₀ (µM)** | **GCN2^{d} Enzyme IC₅₀ (µM)** | **# kinases inhibited > 50% ^{e}** | | |
|---|---|---|---|---|---|---|---|
| | | | | | **0.2 µM** | **2.0 µM** | **20.0 µM** |
| LY2 | 0.002 | 0.117 | 0.056 | 10.8 | 16 | 35 | 138 |
| LY3 | 0.002 | 0.026 | 0.016 | 16.4 | 27 | 85 | 229 |
| LY4 | 0.002 | 0.054 | 0.048 | 18.1 | 19 | 20 | 48 |
| GSK2656157^{f} | 0.008 | 0.036 | 0.021 | >200 | 34 | 80 | 183 |

| | | | | | | | |
|---|---|---|---|---|---|---|---|
| ^{a} PERK biochemical assay using purified eIF2a as substrate. ^{b} Cell-based assay of tunicamycin-induced eIF2a phosphorylation in 293 cells. ^{c} Cell-based assay of tunicamycin-induced ATF4-Luc activity in 293 cells. ^{d} GCN2 biochemical assay using purified eIF2a as substrate. ^{e} DiscoveRₓ scanMAX^{™} kinase profiling based on binding data using displacement of active site probes, 456 kinases tested. ^{f} Atkins et al., "Characterization of a Novel PERK Kinase Inhibitor with Antitumor and Antiangiogenic Activity," Cancer Res. 73(6): 1993-2002 (2013). | | | | | | | |

**Table 7. Comparison of in vitro Inhibition of other eiF2α Kinases**

| **KINOMEscan GENE Symbol^{a}** | **Entrez Gene Symbol** | **LY4** | | | **GSK-2656157^{b}** | | |
|---|---|---|---|---|---|---|---|
| | | **0.2 µM** | **2 µM** | **20 µM** | **0.2 µM** | **2 µM** | **20 µM** |
| EIF2AK1 | EIF2AK1 | 0 | 0 | 35 | **8** | 77 | **95** |
| PRKR | EIF2AK2 | 0 | 0 | 0 | **20** | **45** | **67** |
| GCN2(Kin.Dom.2,S808G) | EIF2AK4 | 0 | 0 | 28 | 7 | **13** | **79** |

| | | | | | | | |
|---|---|---|---|---|---|---|---|
| ^{a} DiscoveRₓ scanMAX^{™} kinase profiling based on binding data using displacement of active site probes, 456 kinases tested. ^{b} Atkins et al., "Characterization of a Novel PERK Kinase Inhibitor with Antitumor and Antiangiogenic Activity," Cancer Res. 73(6): 1993-2002 (2013). | | | | | | | |

24-32 week old uniparous MMTV-HER2 female mice were treated with vehicle or LY4 (50 mpk) i.p. daily, for two weeks. Mammary glands, lungs, pancreas, bone marrow, and tumors were collected for further analyses. LY4 was well tolerated, with no significant changes in body weight, which is in agreement with recent studies showing no effect on blood glucose levels or pancreas function (Pytel et al., "PERK Is a Haploinsufficient Tumor Suppressor: Gene Dose Determines Tumor-Suppressive Versus Tumor Promoting Properties of PERK in Melanoma," PLoS Genet. 12:1-22 (2016)). The inhibitor did not have a significant effect on bone marrow cell homeostasis or on peripheral blood white cells as shown by no effect on total cell counts from MMTV-HER2 females (FIG. 2F).

PERK inhibition caused a significant decrease in P-PERK and P-eIF2α levels in the mammary gland ducts and in pancreatic tissue (although only partial specially in pancreatic islets) (FIG. 3B). It was concluded that systemic LY4 delivery effectively inhibits PERK activation and eIF2α phosphorylation. The inhibition of PERK did not fully deplete PERK activity, which may allow mice to control their pancreatic function and glucose levels (Yu et al., "Type I Interferons Mediate Pancreatic Toxicities of PERK Inhibition," Proc. Natl. Acad. Sci. 112:15420-15425 (2015)).

A high percentage of MMTV-HER2 animals develop metastases to the lungs, which can be initiated early in progression (Guy et al., "Expression of the Neu Protooncogene in the Mammary Epithelium of Transgenic Mice Induces Metastatic Disease," Proc. Nat'l. Acad. Sci. U.S.A. 89:10578-10582 (1992); Husemann et al., "Systemic Spread Is an Early Step in Breast Cancer," Cancer Cell 13:58-68 (2008); Harper et al., "Mechanism of Early Dissemination and Metastasis in Her2+ Mammary Cancer," Nature 540:588-592 (2016), Hosseini et al., "Early Dissemination Seeds Metastasis in Breast Cancer," Nature 540:552-558 (2016); Linde et al., "Macrophages Orchestrate Breast Cancer Early Dissemination and Metastasis," Nat. Commun. 9:21 (2018)).

Thus, the effect of the LY4 PERK inhibitor on metastatic disease was monitored in animals with small and/or palpable large tumors. All the vehicle-treated animals presented metastases detectable in sections stained with H&E. Lesions that displayed >100 cells were categorized as macro-metastases as they are also commonly positive for proliferation markers (FIG. 1A). The quantification of macro-metastases per animal (5 non-consecutive lung sections) revealed that, after just a two-week treatment, LY4 reduced the number and the incidence of macro-metastases (FIG. 3C) without affecting the area of these metastases (FIG. 4A). This suggested that PERK inhibition might be acting on the initial steps of metastasis rather than shrinking established macro-metastases. Thus, whether LY4 treatment might be affecting the intravasation of tumor cells from the primary site or the transition from solitary DCC to micro-metastasis (containing 2-100 cells) was next evaluated. Detection of HER2⁺ circulating tumor cells (CTCs) directly in blood samples showed no significant difference between vehicle and LY4-treated animals (FIG. 4B), indicating that LY4 is not grossly affecting the intravasation of tumor cells. On the other hand, detection of micro-metastasis and single DCCs using HER2 detection via IHC revealed a significant decrease in the number of micro-metastases in LY4-treated females (FIG. 3D). More than 80% of single DCCs in lungs are negative for P-Rb, indicating that they are mostly out of cycle and dormant. This measurement reproduces measurements from previous studies (Harper et al., "Mechanism of Early Dissemination and Metastasis in Her2+ Mammary Cancer," Nature 540:588-592 (2016)). Significantly, LY4 strongly reduced the number of non-proliferating (P-Rb negative) single DCCs that are commonly associated with blood vessels in lung sections, without affecting the number of P-Rb positive solitary DTCs (FIG. 3E) or micrometastases (FIG. 4C). Importantly, LY4 significantly decreased the number of DCCs found in bone marrow (FIG. 3F). In this organ, metastases never develop but DCCs are found at high incidence and are dormant (Bragado et al., "TGF-Beta2 Dictates Disseminated Tumour Cell Fate in Target Organs Through TGF-Beta-RIII and P38Alpha/Beta Signalling," Nat. Cell. Biol. 15:1351-1361 (2013); Husemann et al., "Systemic Spread Is an Early Step in Breast Cancer," Cancer Cell 13:58-68 (2008); and Harper et al., "Mechanism of Early Dissemination and Metastasis in Her2+ Mammary Cancer," Nature 540:588-592 (2016)). These results argue that PERK inhibition is selectively targeting non-proliferative dormant DCCs that display active PERK and UPR signaling.

To further test if LY4 treatment could selectively target the survival of human DCCs out of cycle, this biology was modeled in 3D cultures. Human ZR75.1 HER2⁺ cells stably expressing a photo-switchable fluorescent protein (Dendra2) fused to histone H2B were used to perform long-term label retention assays, due to the slow turnover of H2B-containing nucleosomes in quiescent cells (Wilson et al., "Hematopoietic Stem Cells Reversibly Switch From Dormancy to Self-Renewal During Homeostasis and Repair," Cell 135:1118-1129 (2008)). Upon 405 nm-light exposure for approximately 1 minute, the H2B-DENDRA2 protein switches from green to red fluorescence becoming double positive for green and red; cells that return to green have divided and diluted the H2B-DENDRA2-RED molecules while quiescent cells remain H2B-DENDRA2 GREEN and RED (Gurskaya et al., "Engineering of a Monomeric Green-to-Red Photoactivatable Fluorescent Protein Induced by Blue Light," Nat. Biotechnol. 24:461-465 (2006)). Cells seeded in 3D Matrigel matrix at high density (clusters) to mimic macro-metastases or at low density (single cells) to mimic single DCCs, were photo converted (100%) (FIG. 4D). Eight days later, only 35% of high density cells were H2B-DENDRA2-RED positive. In contrast, 65% of low-density ZR75.1 HER2⁺ cells were H2B-DENDRA2-RED positive (FIG. 4E), mimicking the quiescent state of solitary DCCs (Bragado et al., "TGF-Beta2 Dictates Disseminated Tumour Cell Fate in Target Organs Through TGF-Beta-RIII and P38Alpha/Beta Signalling," Nat. Cell. Biol. 15:1351-1361 (2013)). Treatment with the PERK inhibitor LY4 had no significant effect on the viability of ZR75.1 HER2⁺ seeded at high density (FIG. 4F). However, it eradicated the quiescent single ZR75.1 HER2⁺ cells, paralleling the *in vivo* DCCs results (FIG. 3G). These data indicate that intrinsic and/or ECM dependent signals in the solitary tumor cell context cause a dependence on PERK signaling and that LY4 is indeed selectively targeting slow cycling or non-proliferative DCCs that subsequently reactivate to produce metastasis.

### Example 4 - PERK Inhibition Blocks HER2-Driven Early and Late Mammary Tumor Progression

Having demonstrated that there is a dependency on PERK in quiescent UPR^{high} DCCs, where dormancy is most relevant, tumor lesions were next evaluated. HER2-driven progression was found to be genetically dependent on the PERK kinase in the MMTV-HER2 model (Bobrovnikova-Marjon et al., "PERK-Dependent Regulation of Lipogenesis During Mouse Mammary Gland Development and Adipocyte Differentiation," Proc. Nat 7. Acad. Sci. U.S.A. 105:16314-16319 (2008)) and HER2⁺ tumors have been shown to be sensitive to proteotoxicity and dependent on ERAD (Singh et al., "HER2-mTOR Signaling-Driven Breast Cancer Cells Require ER-Associated Degradation to Survive," Sci. Signal. 8:ra52 (2015)). Further, cBIO database (Cerami et al., "The cBio Cancer Genomics Portal: An Open Platform for Exploring Multidimensional Cancer Genomics Data," Cancer Discovery 2:401-404 (2012)) analysis showed that -14% of HER2 amplified human breast tumors display upregulation of the mRNA for PERK (FIG. 5A). Thus, whether LY4 affected HER2-induced breast tumor progression in primary lesions where the different stages of progression from hyperplastic mammary glands through DCIS and invasive cancer can be dissected was investigated (Lu et al., "Mechanism of Inhibition of MMTV-neu and MMTV-wnt1 Induced Mammary Oncogenesis by RARalpha agonist AM580," Oncogene 29(25):3665-76 (2010); Muller et. al., "Single-Step Induction of Mammary Adenocarcinoma in Transgenic Mice Bearing the Activated c-neu Oncogene," Cell 54(1):105-115 (1988); Harper et al., "Mechanism of Early Dissemination and Metastasis in Her2+ Mammary Cancer," Nature 540:588-592 (2016) ; Hosseini et al., "Early Dissemination Seeds Metastasis in Breast Cancer," Nature 540:552-558 (2016)).

Analysis of 24-week old uniparous female mammary glands showed that vehicle-treated MMTV-HER2 animals exhibited ducts with secondary and tertiary dense branching (FIG. 6A, left panels), and histological analysis showed frequent mammary hyperplastic lesions (FIG. 6A, right panels, black arrows). In contrast, LY4-treated animals showed a "normalized" glandular architecture with less dense branching, resembling the mammary tree of non-transgenic normal FVB mice (FIG. 3B). LY4-treated animals also showed a dramatic increase in the number of hollow lumen mammary gland ducts, constituting more than 60% of the structures compared with around 20% in control females (FIG. 6B and FIG. 5C). The number of occluded hyperplasias and DCIS-like lesions was also reduced to less than half of that in vehicle-treated animals. Hyperplastic lesions in control HER2⁺ animals showed varying degrees of luminal differentiation as assessed by the uneven levels of cytokeratin 8/18 expression (FIG. 6C, upper panel). The myoepithelial cells (detected as smooth muscle actin, SMA, positive), otherwise equally spaced in normal FVB animal ducts, were unevenly distributed in the vehicle-treated hyperplasias in the MMTV-HER2 mice. In contrast, LY4-treated MMTV-HER2 animals presented increased expression of cytokeratin 8/18 in the luminal layer, frequently surrounding an empty lumen, and an external continuous layer of myoepithelial cells (FIG. 6C, lower panel and graph). This data indicates that LY4 treatment leads to a "normalization" of early cancer lesions through a mechanism that seems to restore differentiation programs.

Animals were treated once they displayed tumors, ranging from 30 to 200 mm³ volume (two tumors were >200 mm³) for two weeks with LY4 (FIG. 7A). In vehicle treatment group, tumors grew steadily (FIG. 8A), reaching up to 10 times its original volume in two weeks (FIG. 7B, upper graph). In contrast, LY4-treated tumors showed a reduced growth rate (FIG. 8A), with some tumors remaining in complete cytostasis (defined as doubling tumor volume only once in the 2-week period, 43% in LY4-treated vs 7% in controls) (FIG. 7B, lower graph) and some tumors (25%) showing regression in the 2-week window treatment (FIG. 7C). This led to a significant decrease in median final tumor volume (FIG. 8B). While the levels of proliferation (P-histone H3 IHC) were not different between vehicle- and LY4-treated tumors (FIG. 7D), TUNEL staining of tumor sections showed a significant increase in the levels of DNA fragmentation present in LY4-treated animals (FIG. 8C). Thus, in over primary lesions LY4 treatment induced apoptosis of established HER2⁺ tumors, arguing for context dependent fitness promoting functions of PERK during progression.

Treatment with LY4 of human cancer cells with HER2 overexpression (MCF10A-HER2 or ZR75.1) or HER2-amplified (SKBR3) (FIG. 8D and FIG. 7E) 3D acini cultures in Matrigel showed that a 10 days treatment with vehicle or LY4 (2 µM) significantly increased the levels of apoptosis (cleaved caspase-3) in these organoids, especially in the inner cell mass that is deprived from contact with the ECM (FIG. 8D). As *in vivo,* a significant change in the levels of proliferation as detected by phospho-histone H3 levels was not observed (FIG. 7F). It was concluded that early MMTV-HER2⁺ lesions require PERK for HER2-driven alterations in ductal epithelial organization. In HER2⁺ human cancer cells and mouse tumors HER2 is dependent on PERK for survival.

### Example 5 - PERK Signaling is Required for Optimal HER2 Phosphorylation, Localization and AKT and ERK Activation

Since HER2⁺ tumors are sensitive to proteotoxicity (Singh et al., "HER2-mTOR Signaling-Driven Breast Cancer Cells Require ER-Associated Degradation to Survive," Sci. Signal. 8:ra52 (2015)), whether PERK inhibitors might affect optimal HER2 activity due to increased ER client protein load was evaluated. Detection of HER2 phosphorylation at residues Y1221/1222 in tumors showed that the area positive for P-HER2 reported by others (DiGiovanna et al., "Active Signaling by Neu in Transgenic Mice," Oncogene 17:1877-1884 (1998)) overlapped with the staining for P-PERK and P-eIF2α (FIG. 9A). This finding indicated that the activation of the PERK and HER2 pathways co-localize. Similarly, single cell targeted gene expression profiling of primary tumor cells also showed a population of primary tumor cells (around 25%) with high levels of ER stress genes expression (FIG. 9B), which could correspond to the ones showing P-HER2 activation. Importantly, when P-HER2 levels were scored in the tumors, taking into account both the area and the intensity of the staining (FIG. 10A), it was found that LY4-treated tumors showed significantly lower levels of P-HER2 than control animals (FIG. 9C). HER2 signals as a homodimer or heterodimer with EGFR and HER3 (Moasser MM, "The Oncogene HER2: Its Signaling and Transforming Functions and its Role in Human Cancer Pathogenesis," Oncogene 26(45):6469-87 (2007) and Negro et al., "Essential Roles of Her2/erbB2 in Cardiac Development and Function," Recent Prog. Horm. Res. 59:1-12 (2014)). *In vitro* treatment of MCF10A-HER2 cells that were starved and treated with EGF (100 ng/ml, 15 minutes) in the presence or absence of LY4 (2 µM) revealed that the PERK inhibitor decreased both the basal and EGF-induced levels of P-EGFR and P-HER2, along with downregulation of the survival pathway P-AKT, P-S6 and P-ERK1/2 levels (FIG. 9D and graph and FIG. 10B). No obvious effect was observed under these conditions on total HER2 levels or heterodimerization with EGFR as determined by surface biotinylation and co-immunoprecipitation studies. Since LY4 does not have a direct inhibitory effect on the active site of any of the HER family members, AKT or S6 kinases (Table 8), this effect must be due to an indirect effect of PERK inhibition on HER2 signaling. In contrast to other HER family members, HER2 is known to remain at the plasma membrane after ligand binding and dimerization (Hommelgaard et al., "Association with Membrane Protrusions Makes ErbB2 an Internalization-Resistant Receptor," Mol Biol Cell. 15(4):1557-67 (2004); Bertelsen et al., "The Mysterious Ways of ErbB2/HER2 Trafficking," Membranes (Basel) 4:424-446 (2014)). To test if LY4 might be disturbing the mechanism of activation of HER2 receptors, surface biotinylation assays were performed to measure the presence of the receptor on the cell surface, and reversible surface biotinylation to measure receptor endocytosis (Cihil et al., "The Cell-Based L-Glutathione Protection Assays to Study Endocytosis and Recycling of Plasma Membrane Proteins," J. Vis. Exp. e50867 (2013)). The data showed that LY4 treatment decreased the amount of P-HER2 and total HER2 in the cell surface (FIG. 9E and FIG. 10C), while concomitantly increasing endocytosed phospho-HER2 and total HER2 (FIG. 9F). This data, along with that of Singh et al., "HER2-mTOR Signaling-Driven Breast Cancer Cells Require ER-Associated Degradation to Survive," Sci. Signal. 8:ra52 (2015), suggest that PERK signaling and proper UPR function is required to maintain proper HER2 downstream signaling by affecting optimal receptor localization and activation.

**Table 8. Direct Inhibitory Activity of LY4 on HER Family Signaling Pathway**

| **KINOMEscan GENE Symbol** | **Entrez Gene Symbol** | **LY4** | | | **GSK-2656157** | | |
|---|---|---|---|---|---|---|---|
| | | **0.2 µM** | **2 µM** | **20 µM** | **0.2 µM** | **2 µM** | **20 µM** |
| AKT1 | AKT1 | 0 | 5 | 0 | 14 | 32 | 0 |
| AKT2 | AKT2 | 0 | 0 | 0 | 0 | 4 | 8 |
| AKT3 | AKT3 | 0 | 0 | 13 | 24 | 3 | 0 |
| EGFR | EGFR | 30 | 23 | 16 | 18 | 16 | 50 |
| ERBB2 | ERBB2 | 0 | 0 | 0 | 57 | 64 | 97 |
| ERBB3 | ERBB3 | 0 | 0 | 0 | 40 | 22 | 53 |
| ERBB4 | ERBB4 | 0 | 0 | 4 | 0 | 0 | 0 |
| ERK1 | MAPK3 | 2 | 18 | 0 | 0 | 17 | 0 |
| ERK2 | MAPK1 | 0 | 0 | 1 | 0 | 0 | 0 |
| ERK3 | MAPK6 | 0 | 32 | 3 | 0 | 16 | 0 |
| ERK4 | MAPK4 | 0 | 0 | 0 | 0 | 35 | 0 |
| ERK5 | MAPK7 | 0 | 0 | 4 | 4 | 23 | 12 |
| ERK8 | MAPK15 | 0 | 0 | 0 | 0 | 3 | 6 |
| S6K1 | RPS6KB1 | 31 | 0 | 24 | 0 | 30 | 56 |
| SRC | SRC | 0 | 1 | 11 | 10 | 45 | 69 |
| SYK | SYK | 0 | 0 | 0 | 0 | 12 | 23 |
| CSK | CSK | 31 | 0 | 14 | 0 | 33 | 64 |
| PIK3CA | PIK3CA | 9 | 0 | 8 | 0 | 8 | 0 |
| PIK3CB | PIK3CB | 0 | 0 | 12 | 0 | 0 | 0 |
| PIK3CG | PIK3CG | 24 | 0 | 26 | 36 | 47 | 50 |
| PIK3C2B | PIK3C2B | 40 | 39 | 94 | 0 | 9 | 22 |
| PYK2 | PTK2B | 0 | 0 | 6 | 0 | 0 | 3 |

| | | | | | | | |
|---|---|---|---|---|---|---|---|
| ¹DiscoveRₓ scanMAX^{™} kinase profiling based on binding data using displacement of active site probes, 456 kinases tested. | | | | | | | |

### Example 6 - Sequential Combination of a CDK Inhibitor Followed by PERK Inhibition Enhances the Anti-Metastatic Effect of LY4

The effects of PERK pharmacologic inhibition on primary lesions was established and, most importantly, it was also established that LY4 can inhibit metastasis via eradication of dormant DCCs. With this information, it was evaluated whether clinically available drugs, that would mimic dormancy, could be used to render dormancy-induced cancer cells UPR^{high} and sensitive to LY4. This rationale was supported by the finding that UPR^{high} DCCs expressed higher levels of CDK inhibitors (FIG. 1C). Thus, it was next asked whether increasing the pool of quiescent DCCs above the basal 50% via pre-treatment of the animals with a CDK4/6 inhibitor, Abemaciclib (50 mpk, 4 weeks) (FIG. 11A) would further enhance the anti-metastatic effect of LY4. Indeed, pre-treatment of MMTV-HER2 females with Abemaciclib alone resulted in a striking increase in GADD34⁺ cells in primary tumor sections (FIG. 11B), which otherwise show very low and localized levels of GADD34 staining (control). The measurement in primary tumors served as a surrogate biomarker of quiescence-associated UPR caused by Abemaciclib. As expected, the treatment with LY4 eliminated the expression of GADD34 in treated animals primary tumor (FIG. 11B). The sequential treatment of mice with Abemaciclib (dormancy-like induction phase) followed by LY4 (dormant DCC eradication phase) resulted in the same decrease in macro-metastasis burden observed with the single treatment LY4 (FIG. 11C). However, the combination almost completely eliminated the presence of micro-metastases (FIG. 11D) and, as seen with the single agent, greatly decreased the number of quiescent single disseminated cancer cells (FIG. 11E). Together, these results support the rationale of the sequential combination of a cytostatic agent, such as a CDK inhibitor, followed by LY4 as a promising therapeutic strategy to prevent metastasis by targeting quiescent DCCs that reactivate and seed these lesions.

### Example 7 - Discussion of Examples 2-6

Multiple studies in HER2⁺ breast cancer models have concluded that HER2⁺ breast cancer tumorigenesis depends on PERK signaling for survival and adaptation (Bobrovnikova-Marjon et al., "PERK Promotes Cancer Cell Proliferation and Tumor Growth by Limiting Oxidative DNA Damage," Oncogene 29(27):3881-95 (2010); Singh et al., "HER2-mTOR Signaling-Driven Breast Cancer Cells Require ER-Associated Degradation to Survive," Sci. Signal. 8:ra52 (2015); Avivar-Valderas et al., "PERK Integrates Autophagy and Oxidative Stress Responses to Promote Survival During Extracellular Matrix Detachment," Mol. Cell. Biol. 31:3616-3629 (2011); and Avivar-Valderas et al., "Regulation of Autophagy During ECM Detachment is Linked to a Selective Inhibition of mTORC1 by PERK," Oncogene 32(41):4932-40 (2013)). Intriguingly, it had also been found that quiescent tumor cells, that exist in surgery margins and also as dormant disseminated cancer cells in target organs (Bragado et al., "TGF-Beta2 Dictates Disseminated Tumour Cell Fate in Target Organs Through TGF-Beta-RIII and P38Alpha/Beta Signalling," Nat. Cell. Biol. 15:1351-1361 (2013); Chéry et al., "Characterization of Single Disseminated Prostate Cancer Cells Reveals Tumor Cell Heterogeneity and Identifies Dormancy Associated Pathways," Oncotarget 5(20):9939-51 (2014); Sosa et al., "Mechanisms of Disseminated Cancer Cell Dormancy: An Awakening Field," Nat. Rev. Cancer 14:611-622 (2014); and Sosa et al., "NR2F1 Controls Tumour Cell Dormancy Via SOX9- and RARbeta-Driven Quiescence Programmes," Nat. Commun. 6:6170 (2015)), activated PERK signaling for survival along with other ER stress pathways (Adomako et al., "Identification of Markers that Functionally Define a Quiescent Multiple Myeloma Cell Sub-Population Surviving Bortezomib Treatment," BMC Cancer 15:444 (2015); Ranganathan et al., "Dual Function of Pancreatic Endoplasmic Reticulum Kinase in Tumor Cell Growth Arrest and Survival," Cancer Res. 68:3260-3268 (2008); Ranganathan et al., "Functional Coupling of p38-Induced Up-Regulation of BiP and Activation of RNA-Dependent Protein Kinase-Like Endoplasmic Reticulum Kinase to Drug Resistance of Dormant Carcinoma Cells," Cancer Res. 66:1702-1711 (2006); Schewe et al., "ATF6alpha-Rheb-mTOR Signaling Promotes Survival of Dormant Tumor Cells In Vivo," Proc. Nat'l. Acad. Sci. U.S.A. 105:10519-10524(2008); Schewe et al., "Inhibition of eIF2alpha Dephosphorylation Maximizes Bortezomib Efficiency and Eliminates Quiescent Multiple Myeloma Cells Surviving Proteasome Inhibitor Therapy," Cancer Res. 69:1545-1552 (2009); and Chéry et al., "Characterization of Single Disseminated Prostate Cancer Cells Reveals Tumor Cell Heterogeneity and Identifies Dormancy Associated Pathways," Oncotarget 5(20):9939-51 (2014)). Recently, Pommier et al., "Unresolved Endoplasmic Reticulum Stress Engenders Immune-Resistant, Latent Pancreatic Cancer Metastases," Science 360(6394):eaao4908 (2018) () validated this work by showing that pancreatic DCCs lodged in livers (and other models) activate UPR during quiescence. This level of reproducibility across different cancers and models suggests a high level of biological relevance for this biology.

The Examples herein demonstrate that the PERK inhibitor LY4 can selectively target HER2 dependency in DCCs and in primary lesions. A salient finding to discuss is the inhibitory effect of LY4 on metastasis. In the MMTV-HER2 model, like in patients, metastasis can be asynchronous with the primary tumor and sometimes develop even with occult primary lesions, with some metastasis initiating earlier than overt tumor detection (Husemann et al., "Systemic Spread Is an Early Step in Breast Cancer," Cancer Cell 13:58-68 (2008); Pavlidis et al., "Cancer of Unknown Primary (CUP)," Crit. Rev. Oncol. Hematol. 54:243-250 (2005); Harper et al., "Mechanism of Early Dissemination and Metastasis in Her2+ Mammary Cancer," Nature 540:588-592 (2016); and Hosseini et al., "Early Dissemination Seeds Metastasis in Breast Cancer," Nature 540:552-558 (2016)). LY4 treatment reduced all metastasis, those initiated early (before overt tumors were obvious) or those metastases that were coincident with overt primary tumor growth (FIG. 8). This is important because it argues that the effect on metastasis was not simply due to reduced primary tumor burden caused by LY4. Surprisingly, metastatic burden was reduced by LY4 treatment by eliminating non-proliferative solitary or small clusters of P-Rb-negative DTCs. Imaging and single cell multiplex qPCR revealed that these DCCs showed more frequently upregulation of GADD34 (protein) and a larger set of ER stress genes, including PERK itself, while also showing a quiescent phenotype, revealed by upregulation of several negative regulators of cell proliferation. It should be taken into account that part of the PERK-induced ER stress program entails transcription regulation and another part is preferential translation of upstream ORF-containing genes, such as ATF4 and GADD34 (Young et al., "Upstream Open Reading Frames Differentially Regulate Gene Specific Translation in the Integrated Stress Response," J. Biol. Chem. 291:16927-16935 (2016)). Similarly, UPR-induced G1 arrest has been shown to be caused by inhibiting the translation of cyclin D1 (Brewer et al., "Mammalian Unfolded Protein Response Inhibits Cyclin D1 Translation and Cell-Cycle Progression," Proc. Natl. Acad. Sci. 96:8505-8510 (1999)). 3D organogenesis experiments using human HER2⁺ cancer cell lines confirmed the selective killing by LY4 of quiescent single cancer cells. These data argue that quiescent DCCs are more likely to rely on PERK signaling for survival. Similarly, a sub-population of human metastatic cells from breast cancer patients also showed a negative correlation between GADD34 and Ki67, validating the association found in mice. These data suggest that along with NR2F1 (Borgen et al., "NR2F1 Stratifies Dormant Disseminated Tumor Cells in Breast Cancer Patients," Breast Cancer Research 20:120 (2018)), GADD34 alone or in combination with NR2F1 may serve as a robust biomarker set for dormant/LTPRhigh DCCs and thus guide patient selection for treatment.

Discovering a target and drug that can eradicate dormant DCCs is highly significant because dormant DCCs, are known to evade anti-proliferative therapies via active and passive mechanisms (Aguirre-Ghiso et al., "Metastasis Awakening: Targeting Dormant Cancer," Nat. Med. 19:276-277 (2013); Naumov et al., "Ineffectiveness of Doxorubicin Treatment on Solitary Dormant Mammary Carcinoma Cells or Late-Developing Metastases," Breast Cancer Res. Treat. 82(3):199-206 (2003); Oshimori et al., "TGF-Beta Promotes Heterogeneity and Drug Resistance in Squamous Cell Carcinoma," Cell 160:963-976 (2015); and Fluegen et al., "Phenotypic Heterogeneity of Disseminated Tumour Cells is Preset by Primary Tumour Hypoxic Microenvironments," Nat. Cell Biol. 19(2):120-132 (2017)). The eradication of DCCs in the bone marrow, where these cells are also commonly dormant (Bragado et al., "TGF-Beta2 Dictates Disseminated Tumour Cell Fate in Target Organs Through TGF-Beta-RIII and P38Alpha/Beta Signalling," Nat. Cell. Biol. 15:1351-1361 (2013); Chéry et al., "Characterization of Single Disseminated Prostate Cancer Cells Reveals Tumor Cell Heterogeneity and Identifies Dormancy Associated Pathways," Oncotarget 5(20):9939-51 (2014); Ghajar et al., "The Perivascular Niche Regulates Breast Tumour Dormancy," Nat. Cell Biol. 15:807-817 (2013); and Husemann et al., "Systemic Spread Is an Early Step in Breast Cancer," Cancer Cell 13:58-68 (2008)), further strengthens the notion of PERK inhibition as an anti-dormant DCC therapy (Aguirre-Ghiso et al., "Metastasis Awakening: Targeting Dormant Cancer," Nat. Med. 19:276-277 (2013)) that may be used in the adjuvant setting to eliminate dormant minimal residual disease (Aguirre-Ghiso et al., "Metastasis Awakening: Targeting Dormant Cancer," Nat. Med. 19:276-277 (2013)).

The exact mechanisms by which PERK kinase inhibition blocks tumor growth are unclear. It is possible that reduced adaptation to stress imposed by proteotoxicity (Singh et al., "HER2-mTOR Signaling-Driven Breast Cancer Cells Require ER-Associated Degradation to Survive," Sci. Signal. 8:ra52 (2015)) is a mechanism. The results described herein demonstrate that LY4 reduced phospho-HER2 levels *in vivo* and LY4 decreased the abundance of active receptor in the membrane through enhanced endocytosis. Changes in HER2 protein degradation were not observed. Regarding how exactly PERK controls HER2 membrane localization or endocytosis, it is possible that the internalization of the receptor allows for better or faster dephosphorylation of the receptor or decreases the chances of the receptor to get activated, hence resulting in decreased downstream signaling. It has been shown that receptor endocytosis can reduce the signaling output of many plasma membrane localized receptors by physically reducing the concentration of cell surface receptors (Sorkin and Zastrow, "Endocytosis and Signaling: Intertwining Molecular Networks," Nat. Rev. Mol. Cell Biol. 10:609-22 (2009)).

The results herein further demonstrate that, in early lesions, LY4 induced a differentiation phenotype. However, in established tumors, LY4 pushed tumors into stasis or regression as a single agent. This argues that, early on, PERK signaling deregulation in HER2⁺ early lesions is more linked to loss of differentiation programs, through yet to be determined mechanisms. Then, as the biology of the tumor changes to become highly proliferative, the dependency on PERK is still highly reliant for these HER2⁺ tumors. This may be linked to how HER2 functions shift during progression; at early stages it mainly deregulates a morphogenesis program that leads to anoikis resistance and dissemination, while later it is mainly engaged in proliferative and survival programs.

The results described herein also point to the value of combining standard antiproliferative therapies with LY4 that would eliminate remaining quiescent cells. Such an approach was tested using the combination of the CDK4/6 inhibitor Abemaciclib followed by LY4, which revealed an improved anti-metastatic effect. Encouragingly, the doses of LY4 used did not significantly affect glucose levels, bone marrow, or peripheral blood cell counts, drinking and feeding behavior of non-tumor or cancer bearing mice. This argues that the doses used, while severely blocking tumor growth and metastasis through dormant DCC eradication, did not affect the host's normal organ function. Since dormant/UPR^{high} DCCs were also found to downregulate MHC-I surface expression (Pommier et al., "Unresolved Endoplasmic Reticulum Stress Engenders Immune-Resistant, Latent Pancreatic Cancer Metastases," Science 360(6394):eaao4908 (2018)), LY4 may also help the adaptive immune response target DCCs and perhaps established tumors as well. Current work is addressing such possibility. The results described herein open the door to the use of anti-dormant DCC survival therapies as a new way to target metastatic disease. This would allow targeting the full phenotypic heterogeneity of disseminated disease that may include proliferative, slow-cycling, and dormant DCCs (Aguirre-Ghiso et al., "Metastasis Awakening: Targeting Dormant Cancer," Nat. Med. 19:276-277 (2013)).

### Example 8 - Combination of the CDK4/6 Inhibitor Abemaciclib and the PERK Inhibitor LY4 in a Melanoma Cell Line

Since CDK4/6 inhibitors have been shown to induce cell cycle arrest and LY4 induces cell death in dormant cell cycle arrested DTCs (FIG. 12A), it was next investigated whether the combination of a CDK4/6 inhibitor and LY4 would reduce cell viability in an *in vitro* cell line. The CDK 4/6 inhibitor Abemaciclib has been shown to inhibit the growth of WM35 melanoma cells in both 2D and 3D *in vitro* cell cultures. *In vitro* acute treatment (48 hours) with 2 µM LY4 following 1 week of 50 nM Abemaciclib pre-treatment (2D) decreases the viability of Braf-mutatnt melanoma WM35 cells, as compared to the treatment of cells with 2 µM LY4 alone (FIG. 12B). In *in vitro* 3D cultures, the addition of 2 µM LY4 following a 1 week Abemaciclib pre-treatment had an additive effect on decreasing cell viability (FIG. 12C). The results demonstrated in FIG. 12C suggest that Abemaciclib pre-treatment may induce growth arrest and some cell death in 3D cell culture. The addition of LY4 then seems to enhance that effect on cell death. This is consistent with the notion that Abemaciclib arrested cells may upregulate an ER stress response as shown by GADD34 upregulation (FIG. 12G) and then become sensitive to LY4.

In melanoma cells, an Abemaciclib-resistant phenotype arises after 4-5 weeks of continuous treatment. The co-treatment of cells with LY4 and Abemaciclib decreases the number of viable Abemaciclib resistant cells in 2D cell culture but does not show an enhancement as expected from performing these experiments in 2D cultures. However, in the Abemaciclib resistant cells, LY4 has an additive effect on decreasing viability in 3D cell culture after persistent treatment with Abemaciclib (FIGs. 12D-12E). These data argue that the melanoma CDK4/6 inhibitor resistant cells remain dependent on the ER stress response mediated by PERK to survive. In *in vitro* 3D cultures, the addition of LY4 after 5 week Abemaciclib pre-treatment had an additive effect on decreasing cell viability (FIG. 12F), as measured by apoptosis of cells that take up DAPI. Although resistant cells grow as well as control cells in 2D cultures, Abemaciclib pre-treatment seems to induce cell death in 3D culture (FIG. 12F).

### Example 9 - BMP7-F9 Induces and Maintains Dormancy of DTCs (HNSCC)

BMP7-F9 reduces the ERK/p38 activity ratio and induces various mRNAs in the dormancy signature (FIGs. 13A-13C). FIG. 13A shows that BMP7-F9 treatment at 2 ng/ml, 5 ng/ml, and 10 ng/ml (second, third, and fourth gray bars, respectively; control is first black bar) reduces the ERK/p38 activity ratio over control, as determined by Western blot in HEp3 HNSCC cells. The effect on the ERK/p38 activity ratio is observed after 2-6 and 24 hours (second through fourth group of columns). In the first 30 minutes ERK activity is stimulated by BMP7 (first column set). FIG. 13B shows that BMP7-F9 treatment induces DEC2, p53, and p27 mRNAs (10 ng/ml BMP7-F9, 24 hours), which encode dormancy signature genes. FIG. 13C shows that BMP7-F9 treatment of the same cells induces nuclear accumulation of NR2F1, a potent dormancy inducing transcription factor, as determined by immunofluorescence (10 ng/ml, 24 hours). Differences in FIG. 13A and FIG. 13B, p<0.05 as calculated using Student's t test.

*In vitro* and *in vivo* BMP7-F9 induces growth arrest of T-HEp3 cells (FIGs. 14A-14E). FIG. 14A shows that BMP7-F9 treatment of T-HEp3 cells inhibits their proliferation *in vitro* for 48 hours, as determined by cell titer blue assay (RFU, relative fluorescence units). FIG. 14B is a schematic illustration of the *in vivo* experimental procedure used in FIGs. 14C-14D. T-HEp3 cells were pre-treated for 24 hours with BMP7-F9 *in vitro* and then inoculated on chicken embryo chorioallantoic membranes (CAMs) (FIG. 14C), where they were treated daily *in vivo* with vehicle or BMP7-F9 (50 ng/ml) prior to collection of the tumors and quantification of number of HEp3 HNSCC cells/tumor (FIG. 14D) and levels of P-H3 (FIG. 14E).

NSG mice were treated following the protocol in FIG. 15A for 3 and 6 weeks. At those time points, the percentage of local recurrence and DTC incidence was scored. Tabulated results corresponding to FIG. 15B show that BMP7 limits the incidence of local recurrences (Table 9) and DTC incidence in lungs (Table 10) post-tumor surgery are shown below (Table 11).

**Table 9. Effect of BMP7-F9 in the Adjuvant Setting on Local Recurrences in Surgery Margins**

| **Treatment (adjuvant treatment only)** | | **n** | **+local recurrence** | **Local recurrence incidence (%)** |
|---|---|---|---|---|
| 3 weeks | control | 11 | 4 | 36.4 |
| | BMP7 | 11 | 1 | 9.1 |
| 6 weeks | control | 8 | 2 | 25.0 |
| | BMP7 | 7 | 0 | 0.0 |

**Table 10. Effect of BMP7-F9 on DCC Incidence in Lungs**

| **Treatment (adjuvant treatment only)** | | **n** | **+DCCs in lungs** | **DCC incidence (%)** |
|---|---|---|---|---|
| 3 weeks | control | 11 | 9 | 81.8 |
| | BMP7 | 10 | 5 | 50.0 |
| 6 weeks | control | 8 | 6 | 75.0 |
| | BMP7 | 7 | 3 | 42.9 |

**Table 11. Median Number of GFP⁺/Vimentin⁺ Tumor Cells/Lung in the Same Experiments Depicted in FIGs. 15A-15B and Tables 9 and 10**

| **Treatment (adjuvant treatment only)** | | **n** | **Median number of GFP⁺/Vimentin⁺ tumor cells/lung** | **range** | **p value** |
|---|---|---|---|---|---|
| 3 weeks | control | 11 | 2.2E+04 | 0-1.14E+0.6 | 0.2294 |
| | BMP7 | 10 | 0.62E+04 | 0-333333 | |
| 6 weeks | control | 8 | 25.2E+04 | 0-1.05E+06 | 0.2859 |
| | BMP7 | 7 | 0 | 0-6.8E+06 | |

HEp3-GFP HNSCC tumors were grown until they were approximately 300 mm³ and then treated in the neo-adjuvant setting with 50 µg/kg BMP7-F9 until tumors were approximately 600 mm³. Tumors were then removed via surgery. 1-2 days after surgery, the adjuvant treatment with BMP7-F9 was continued for another 4 weeks. Animals were then euthanized and the DCC burden in lung was scored using fluorescence microscopy. BMP7 was observed to limit the development of local and distant recurrences post-tumor surgery. NSG mice were treated following the protocol in FIG. 15A for 4 weeks. At those time points, the percentage of local recurrence and DCC incidence was scored. The number of GFP positive cells in dissociated lungs was scored following treatment. This is a measure of DCC burden in lungs which is significantly decreased by BMP7-F9 treatment. Note that the median of DCC burden drops one log and that BMP-7 apparently cures from DCCs 3 of 7 animals.

The effect of BMP7-F9 in the neoadjuvant + adjuvant setting on local recurrences in surgery margins (Table 12) and DCC incidence in lungs (Table 13) is shown below. The results are tabulated from the results in FIG. 15C, where mice were treated as in FIG. 15A, except that adjuvant treatment was for 4 weeks. The number of GFP positive cells in dissociated lungs was scored following treatment. The results show that BMP7 limits the incidence of local recurrences (Table 12) and DCC incidence in lungs (Table 13) post-tumor surgery with a neoadjuvant and adjuvant treatment. Table 14 shows a measure of DCC burden in lungs, which is significantly decreased by BMP7-F9 treatment. Note that the median of DCC burden drops one log and that BMP-7 apparently cures from DCCs 3 of 7 animals.

**Table 12. Effect of BMP7-F9 in the Neoadjuvant + Adjuvant Setting on Local Recurrences in Surgery Margins**

| **Treatment (neo-adjuvant and adjuvant treatment)** | | **n** | **+local recurrence** | **%** |
|---|---|---|---|---|
| 4 weeks | control | 8 | 5 | 62.5 |
| | BMP7 | 7 | 3 | 42.9 |

**Table 13. DCC Incidence in Lungs**

| **Treatment (neo-adjuvant and adjuvant treatment)** | | **n** | **+DCCs in lungs** | **%** |
|---|---|---|---|---|
| 4 weeks | control | 8 | 8 | 100 |
| | BMP7 | 7 | 4 | 57.1 |

**Table 14. Median Number of GFP⁺/Vimentin⁺ Tumor Cells/Lung of Mice Reported in Tables 12 and 13.**

| **Treatment (neo-adjuvant and adjuvant treatment)** | | **n** | **Median number of GFP⁺/Vimentin⁺ tumor cells/lung** | **range** | **p value** |
|---|---|---|---|---|---|
| 4 weeks | control | 8 | 5.4E+04 | 0.02-3.2E+05 | 0.046 |
| | BMP7 | 7 | 0.02E+04 | 0-2.4E+04 | |

Although preferred embodiments have been depicted and described in detail herein, it will be apparent to those skilled in the relevant art that various modifications, additions, substitutions, and the like can be made.

## Claims

1. A protein kinase RNA-like endoplasmic reticulum kinase (PERK) inhibitor for use in a method of treating minimal residual cancer in a subject, said method comprising:
contacting disseminated cancer cells (DCCs) in a subject with the protein kinase RNA-like endoplasmic reticulum kinase (PERK) inhibitor, wherein said contacting eradicates DCCs in the subject to treat minimal residual cancer in the subject;
wherein said protein kinase RNA-like endoplasmic reticulum kinase (PERK) inhibitor is selected from
3-amino-6-[4-[[(2R)-2-(3,5-difluorophenyl)-2-hydroxy-acetyl]amino]-2-methyl-phenyl]-N-methyl-pyrazine-2-carboxamide (LY2), having a chemical structure 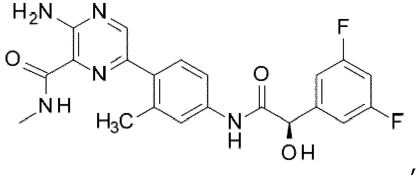
(2R)-N-[4-(4-amino-7-methyl-pyrrolo[2,3-d]pyrimidin-5-yl)-3-methylphenyl]-2-(3-fluorophenyl)-2-hydroxy-acetamide (LY3), having a chemical structure 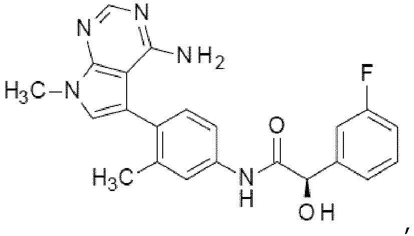 and
2-amino-5-[4-[[(2R)-2-(3,5-difluorophenyl)-2-hydroxy-acetyl]amino]-2-methyl-phenyl]-N-isopropyl-pyridine-3-carboxamide (LY4), having a chemical structure

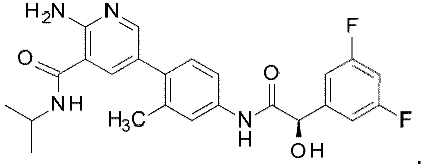

2. The protein kinase RNA-like endoplasmic reticulum kinase (PERK) inhibitor for use according to claim 1, wherein:
(a) the subject has been diagnosed with breast cancer, multiple myeloma, lung cancer, non-small cell lung cancer, brain cancer, cervical cancer, mantel cell lymphoma, leukemia, hepatocellular carcinoma, prostate cancer, melanoma, skin cancers, head and neck cancers, thyroid cancer, glioblastoma, neuroblastoma, or colorectal cancer; optionally
wherein the cancer is breast cancer selected from invasive breast cancer, ductal carcinoma in situ (DCIS), lobular carcinoma in situ (LCIS), and inflammatory breast cancer; optionally
wherein the breast cancer is a HER2⁺ breast cancer; or
(b) the subject has been diagnosed with disseminated tumor cells and/or a non-metastatic cancer.

3. The protein kinase RNA-like endoplasmic reticulum kinase (PERK) inhibitor for use according to either of claims 1 or 2 further comprising administering to the subject a chemotherapeutic agent, an immunotherapeutic agent, an epigenetic agent, or ionizing radiation; optionally
wherein a chemotherapeutic agent, an immunotherapeutic agent, or an epigenetic agent is administered to the subject, and wherein:
(a) the chemotherapeutic agent is an anti-HER2 chemotherapeutic agent selected from trastuzumab (Herceptin^{®}) and lapatinib (Tykerb^{®});
(b) the chemotherapeutic agent is selected from an anthracycline, a taxane, a kinase inhibitor, an antibody, a fluoropyrimidine, and a platinum drug;
(c) the immunotherapeutic agent is selected from an immune checkpoint inhibitor, an interferon, or a tumor vaccine; or
(d) the epigenetic agent is selected from a histone deacetylase (HDAC) inhibitor, 5-azacytidine, retinoic acid, arsenic trioxide, Enhancer Of Zeste 2 Polycomb Repressive Complex 2 Subunit (EZH2) inhibitor, bromodomain ("BRD") inhibitor, and derivatives thereof.

4. The protein kinase RNA-like endoplasmic reticulum kinase (PERK) inhibitor for use according to any one of claims 1-3 further comprising:
detecting the presence of DCCs in the subject prior to said contacting; optionally wherein the DCCs are:
(a) NR2F1⁺; and/or
(b) phospho-PERK active.

5. The protein kinase RNA-like endoplasmic reticulum kinase (PERK) inhibitor for use according to claim 4, wherein the DCCs are bone morphogenic protein receptor positive ("BMPR⁺").

6. The protein kinase RNA-like endoplasmic reticulum kinase (PERK) inhibitor for use according to claim 5, wherein the method further comprises contacting DCCs in the subject with a bone morphogenic protein 7 (BMP7) derivative protein by administering the BMP7 derivative protein to the subject, wherein the BMP7 derivative protein is BMP7-F9 having the amino acid sequence of SEQ ID NO: 8.

7. The protein kinase RNA-like endoplasmic reticulum kinase (PERK) inhibitor for use according to any one of claims 1-6, wherein the PERK inhibitor does not inhibit EIF2AK1, EIF2AK2, or EIF2AK4.

8. The protein kinase RNA-like endoplasmic reticulum kinase (PERK) inhibitor for use according to any one of claims 1-7, wherein the subject is:
(a) a human; and/or
(b) in cancer remission prior to said contacting.

## Patentansprüche

1. Inhibitor von Proteinkinase-RNA-ähnlicher endoplasmatischer Retikulum-Kinase (PERK) zur Verwendung in einem Verfahren zur Behandlung von minimalem Restkrebs bei einem Subjekt, wobei das Verfahren umfasst:
In-Kontakt-Bringen disseminierter Krebszellen (DCCs) in einem Subjekt mit dem Inhibitor von Proteinkinase-RNA-ähnlicher endoplasmatischer Retikulum-Kinase (PERK), wobei das In-Kontakt-Bringen DCCs im Subjekt ausrottet, um minimalen Restkrebs im Subjekt zu behandeln;
wobei der Inhibitor von Proteinkinase-RNA-ähnlicher endoplasmatischer Retikulum-Kinase (PERK) ausgewählt ist aus
3-Amino-6-[4-[[(2R)-2-(3,5-difluorphenyl)-2-hydro-xyacetyl]amino]-2-methylphenyl]-N-methylpyrazin-2-carboxamid (LY2), aufweisend eine chemische Struktur 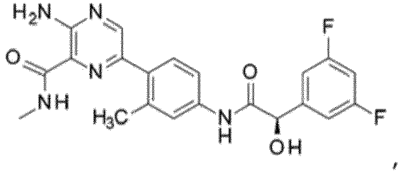
(2R)-N-[4-(4-Amino-7-methylpyrrolo[2,3-d]pyrimidin-5-yl)-3-methylphenyl]-2-(3-fluorphenyl)-2- hydroxyacetamid (LY3), aufweisend eine chemische Struktur 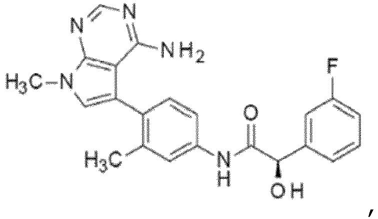 und
2-Amino-5-[4-[[(2R)-2-(3,5-difluorphenyl)-2-hydro-xyacetyl]amino]-2-methylphenyl]-N-isopropylpyridin-3-carboxamid (LY4), aufweisend eine chemische Struktur

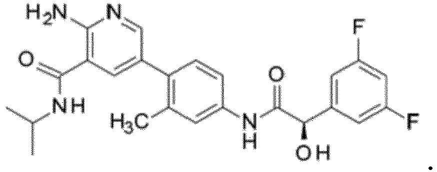

2. Inhibitor von Proteinkinase-RNA-ähnlicher endoplasmatischer Retikulum-Kinase (PERK) zur Verwendung nach Anspruch 1, wobei:
(a) bei dem Subjekt Brustkrebs, multiples Myelom, Lungenkrebs, nichtkleinzelliger Lungenkrebs, Gehirnkrebs, Gebärmutterhalskrebs, Mantelzelllymphom, Leukämie, hepatozelluläres Karzinom, Prostatakrebs, ein Melanom, Hautkrebs, Kopf- und Halskrebs, Schilddrüsenkrebs, ein Glioblastom, Neuroblastom oder Kolorektalkrebs diagnostiziert wurde; optional
wobei der Krebs Brustkrebs ist, ausgewählt aus invasivem Brustkrebs, duktalem Karzinom in situ (DCIS), lobulärem Karzinom in situ (LCIS) und inflammatorischem Brustkrebs; optional
wobei der Brustkrebs ein HER2⁺-Brustkrebs ist; oder
(b) bei der Person disseminierte Tumorzellen und/oder ein nicht metastasierter Krebs diagnostiziert wurden.

3. Inhibitor von Proteinkinase-RNA-ähnlicher endoplasmatischer Retikulum-Kinase (PERK) zur Verwendung nach einem der Ansprüche 1 oder 2, weiter umfassend Verabreichen eines chemotherapeutischen Mittels, eines immuntherapeutischen Mittels, eines epigenetischen Mittels oder ionisierender Strahlung an das Subjekt; optional
wobei dem Subjekt ein chemotherapeutisches Mittel, ein immuntherapeutisches Mittel oder ein epigenetisches Mittel verabreicht wird, und wobei:
(a) das chemotherapeutische Mittel ein Anti-HER2-chemotherapeutisches Mittel ist, ausgewählt aus Trastuzumab (Herceptin^{®}) und Lapatinib (Tykerb^{®});
(b) das chemotherapeutische Mittel ausgewählt ist aus einem Anthracyclin, einem Taxan, einem Kinase-Inhibitor, einem Antikörper, einem Fluoropyrimidin und einem Platin-Arzneimittel;
(c) das immuntherapeutische Mittel ausgewählt ist aus einem Immun-Checkpoint-Inhibitor, einem Interferon oder einem Tumorimpfstoff; oder
(d) das epigenetische Mittel ausgewählt ist aus einem Histondeacetylase (HDAC)-Inhibitor, 5-Azacytidin, Retinsäure, Arsentrioxid, einem Enhancer Of Zeste 2 Polycomb Repressive Complex 2 Subunit (EZH2)-Inhibitor, einem Bromodomänen("BRD")-Inhibitor, und Derivaten davon.

4. Inhibitor von Proteinkinase-RNA-ähnlicher endoplasmatischer Retikulum-Kinase (PERK) zur Verwendung nach einem der Ansprüche 1-3, weiter umfassend:
Erkennen des Vorhandenseins von DCCs im Subjekt vor dem In-Kontakt-Bringen; optional
wobei die DCCs:
(a) NR2F1⁺; und/oder
(b) Phospho-PERK-aktiv sind.

5. Inhibitor von Proteinkinase-RNA-ähnlicher endoplasmatischer Retikulum-Kinase (PERK) zur Verwendung nach Anspruch 4, wobei die DCCs positiv für knochenmorphogene Proteinrezeptoren ("BMPR⁺") sind.

6. Inhibitor von Proteinkinase-RNA-ähnlicher endoplasmatischer Retikulum-Kinase (PERK) zur Verwendung nach Anspruch 5, wobei das Verfahren weiter In-Kontakt-Bringen von DCCs im Subjekt mit einem von knochenmorphogenem Protein 7 (BMP7) abgeleiteten Protein durch Verabreichen des von BMP7 abgeleiteten Proteins an das Subjekt umfasst, wobei das von BMP7 abgeleitete Protein BMP7-F9 ist, das die Aminosäuresequenz von SEQ ID NO: 8 aufweist.

7. Inhibitor von Proteinkinase-RNA-ähnlicher endoplasmatischer Retikulum-Kinase (PERK) zur Verwendung nach einem der Ansprüche 1-6, wobei der PERK-Inhibitor EIF2AK1, EIF2AK2 oder EIF2AK4 nicht hemmt.

8. Inhibitor von Proteinkinase-RNA-ähnlicher endoplasmatischer Retikulum-Kinase (PERK) zur Verwendung nach einem der Ansprüche 1-7, wobei das Subjekt:
(a) ein Mensch; und/oder
(b) in Krebsremission vor dem In-Kontakt-Bringen ist.

## Revendications

1. Inhibiteur de la protéine kinase du réticulum endoplasmique (PERK) semblable à la protéine kinase RNA destiné à être utilisé dans un procédé de traitement d'un cancer résiduel minime chez un sujet, ledit procédé comprenant :
la mise en contact de cellules cancéreuses disséminées (DCC) chez un sujet avec un inhibiteur de la protéine kinase du réticulum endoplasmique (PERK) semblable à la protéine kinase RNA, dans lequel ladite mise en contact éradique les DCC chez le sujet pour traiter un cancer résiduel minimal chez le sujet ;
dans lequel ledit inhibiteur de la protéine kinase du réticulum endoplasmique (PERK) semblable à la protéine kinase RNA est choisi parmi
le 3-amino-6-[4-[[(2R)-2-(3,5-difluorophényl)-2-hydroxy-acétyl]amino]-2-méthyl-phényl]-N-méthyl-pyrazine-2-carboxamide (LY2), ayant une structure chimique 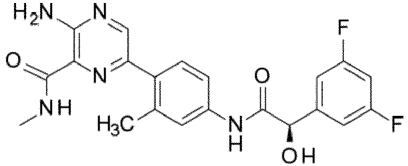
le (2R)-N-[4-(4-amino-7-méthyl-pyrrolo[2,3-d]pyrimidin-5-yl)-3-méthyl-phényl]-2-(3-fluorophényl)-2-hydroxy-acétamide (LY3), ayant une structure chimique 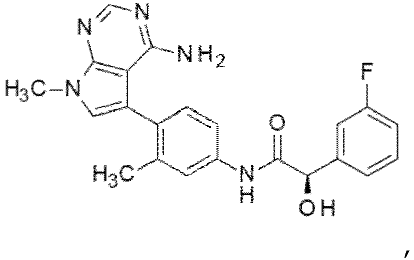 et
le 2-amino-5-[4-[[(2R)-2-(3,5-difluorophényl)-2-hydroxy-acétyl]amino]-2-méthyl-phényl]-N-isopropyl-pyridine-3-carboxamide (LY4), ayant une structure chimique

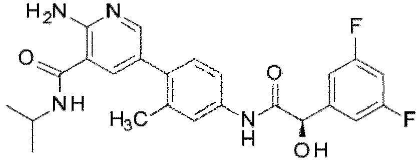

2. Inhibiteur de la protéine kinase du réticulum endoplasmique (PERK) semblable à la protéine kinase RNA destiné à être utilisé selon la revendication 1, dans lequel :
(a) le sujet a reçu un diagnostic de cancer du sein, de myélome multiple, de cancer du poumon, de cancer du poumon non à petites cellules, de cancer du cerveau, de cancer du col de l'utérus, de lymphome à cellules du manteau, de leucémie, de carcinome hépatocellulaire, de cancer de la prostate, de mélanome, de cancers de la peau, de cancers de la tête et du cou, de cancer de la thyroïde, de glioblastome, de neuroblastome ou de cancer colorectal ; éventuellement
dans lequel le cancer est un cancer du sein choisi parmi le cancer du sein invasif, le carcinome canalaire in situ (DCIS), le carcinome lobulaire in situ (LCIS) et le cancer du sein inflammatoire ; éventuellement
dans lequel le cancer du sein est un cancer du sein HER2+ ; ou
(b) le sujet a reçu un diagnostic de cellules tumorales disséminées et/ou d'un cancer non métastatique.

3. Inhibiteur de la protéine kinase du réticulum endoplasmique (PERK) semblable à la protéine kinase RNA destiné à être utilisé selon l'une des revendications 1 ou 2, comprenant en outre l'administration au sujet d'un agent chimiothérapeutique, d'un agent immunothérapeutique, d'un agent épigénétique ou d'un rayonnement ionisant ; éventuellement
dans lequel un agent chimiothérapeutique, un agent immunothérapeutique ou un agent épigénétique est administré au sujet, et dans lequel :
(a) l'agent chimiothérapeutique est un agent chimiothérapeutique anti-HER2 choisi parmi le trastuzumab (Herceptin^{®}) et le lapatinib (Tykerb^{®}) ;
(b) l'agent chimiothérapeutique est choisi parmi une anthracycline, un taxane, un inhibiteur de kinase, un anticorps, une fluoropyrimidine et un médicament à base de platine ;
(c) l'agent immunothérapeutique est choisi parmi un inhibiteur de point de contrôle immunitaire, un interféron ou un vaccin antitumoral ; ou
(d) l'agent épigénétique est choisi parmi un inhibiteur d'histone désacétylase (HDAC), la 5-azacytidine, l'acide rétinoïque, le trioxyde d'arsenic, l'inhibiteur de Enhancer Of Zeste 2 Polycomb Repressive Complex 2 Subunit (EZH2), l'inhibiteur de bromodomaine (« BRD ») et des dérivés de ceux-ci.

4. Inhibiteur de la protéine kinase du réticulum endoplasmique (PERK) semblable à la protéine kinase RNA destiné à être utilisé selon l'une quelconque des revendications 1 à 3, comprenant en outre :
la détection de la présence des DCC chez le sujet avant ladite mise en contact ; éventuellement
dans lequel les DCC sont :
(a) NR2F1+ ; et/ou
(b) actives pour la phospho-PERK.

5. Inhibiteur de la protéine kinase du réticulum endoplasmique (PERK) semblable à la protéine kinase RNA destiné à être utilisé selon la revendication 4, dans lequel les DCC sont positives au récepteur de la protéine morphogénique osseuse (« BMPR+ »).

6. Inhibiteur de la protéine kinase du réticulum endoplasmique (PERK) semblable à la protéine kinase RNA destiné à être utilisé selon la revendication 5, dans lequel le procédé comprend en outre la mise en contact des DCC chez le sujet avec une protéine dérivée de la protéine morphogénique osseuse 7 (BMP7) en administrant la protéine dérivée de BMP7 au sujet, dans lequel la protéine dérivée de BMP7 est BMP7-F9 ayant la séquence d'acides aminés de SEQ ID NO : 8.

7. Inhibiteur de la protéine kinase du réticulum endoplasmique (PERK) semblable à la protéine kinase RNA destiné à être utilisé selon l'une quelconque des revendications 1 à 6, dans lequel l'inhibiteur de PERK n'inhibe pas EIF2AK1, EIF2AK2 ou EIF2AK4.

8. Inhibiteur de la protéine kinase du réticulum endoplasmique (PERK) semblable à la protéine kinase RNA destiné à être utilisé selon l'une quelconque des revendications 1 à 7, dans lequel le sujet est :
(a) un être humain ; et/ou
(b) en rémission du cancer avant ladite mise en contact.
